# EUROPEAN PATENT SPECIFICATION

(11) **EP 1 409 646 B1**
(45) Date of publication and mention of the grant of the patent: **13.06.2012**
(21) Application number: 01994325.7
(22) Date of filing: 19.12.2001
(51) Int. Cl.: C12N 5/02

(54) **TRANSGENIC ANIMALS COMPRISING A HUMANIZED IMMUNE SYSTEM**
TRANSGENE TIERE MIT HUMANISIERTEM IMMUNSYSTEM
ANIMAUX TRANSGENIQUES A SYSTEME IMMUNITAIRE HUMANISE

(30) Priority: 19.12.2000 US 256591 P
(43) Date of publication of application: 21.04.2004
(73) Proprietor: Altor BioScience Corporation, Miramar, Florida 33025 (US)
(72) Inventor: BELMONT, Heather, J., North Miami Beach, FL 33180 (US); WONG, Hing, C., Weston, FL 33332 (US); WITTMAN, Vaughan, P., Weston, FL 33332 (US); WEIDANZ, Jon, A., Amarillo, TX 79124 (US)
(74) Representative: Hiebl, Inge Elisabeth
(86) International application number: PCT/US2001/049413
(87) International publication number: WO 2002/059263

(56) References cited:
- WO-A-97/32603
- WO-A2-98/24893
- US-A- 6 150 584
- ROTHE JOACHIM ET AL: "Functional expression of a human TCR-beta gene in transgenic mice" INTERNATIONAL IMMUNOLOGY, vol. 5, no. 1, 1993, pages 11-17, XP009039278 ISSN: 0953-8178
- VINEY J L ET AL: "GENERATION OF MONOCLONAL ANTIBODIES AGAINST A HUMAN T CELL RECEPTORBETA CHAIN EXPRESSED IN TRANSGENIC MICE" HYBRIDOMA, LIEBERT, NEW YORK, NY, US, vol. 11, no. 6, 1 December 1992 (1992-12-01), pages 701-713, XP002062863 ISSN: 0272-457X
- FUKUI YOSHINORI ET AL: "Differential requirement of MHC class II molecules expressed on hematopoietic cells for positive selection of CD4+ thymocytes in TCR-alpha-beta and TCR-beta transgenic mice" INTERNATIONAL IMMUNOLOGY, vol. 9, no. 9, 1997, pages 1385-1391, XP002303910 ISSN: 0953-8178
- CHUNG S ET AL: "FUNCTIONAL THREE-DOMAIN SINGLE-CHAIN T-CELL RECEPTORS" PROCEEDINGS OF THE NATIONAL ACADEMY OF SCIENCES OF USA, NATIONAL ACADEMY OF SCIENCE. WASHINGTON, US, vol. 91, no. 26, 20 December 1994 (1994-12-20), pages 12654-12658, XP002039051 ISSN: 0027-8424
- ISHIMOTO T ET AL: "In vitro and in vivo evidence for high frequency of I-Ab-reactive CD4+ T cells in HLA-DQ or HLA-DRA transgenic mice lacking endogenous MHC class I and/or class II expression." JOURNAL OF IMMUNOLOGY (BALTIMORE, MD. : 1950) 15 OCT 1997, vol. 159, no. 8, 15 October 1997 (1997-10-15), pages 3717-3722, XP002303911 ISSN: 0022-1767
- ALVAREZ J.D. ET AL.: 'V(D)J recombination and allelic exclusion of a TCR beta-chain minilocus occurs in the absence of a functional promoter' J. IMMUNOL. vol. 155, 1995, pages 1191 - 1202, XP002961818

### SEQUENCE LISTING

<110> SUNOL MOLECULAR CORPORATION
<120> TRANSGENIC ANIMALS COMPRISING A HUMANIZED IMMUNE SYSTEM
<130> 13448EP
<140> 01 994 325.7
   <141> 2001-12-19
<150> US 60/256,591
   <151> 2000-12-19
<160> 22
<170> PatentIn Ver. 2.1
<210> 1
   <211> 24
   <212> DNA
   <213> Artificial Sequence
<220>
   <223> Description of Artificial Sequence:Primer
<400> 1
   actgggatcc aaatgagtct tcgg 24
<210> 2
   <211> 32
   <212> DNA
   <213> Artificial Sequence
<220>
   <223> Description of Artificial Sequence:Primer
<400> 2
   actggcggcc gccaaacgac ccaacacccg tg 32
<210> 3
   <211> 44
   <212> DNA
   <213> Artificial Sequence
<220>
   <223> Description of Artificial Sequence:Oligonucleotide probe
<400> 3
   cccacctgga tctcccagat ttgtgaggaa ggttgctgga gagc 44
<210> 4
   <211> 45
   <212> DNA
   <213> Artificial Sequence
<220>
   <223> Description of Artificial Sequence:Oligonucleotide probe
<400> 4
   ggaaagccct gctggctcca agatggctga gggaaaggtc tacgg 45
<210> 5
   <211> 36
   <212> DNA
   <213> Artificial Sequence
<220>
   <223> Description of Artificial Sequence:Primer
<400> 5
   tagtggatcc catgcagaga gaaaccgaag tacgtg 36
<210> 6
   <211> 26
   <212> DNA
   <213> Artificial Sequence
<220>
   <223> Description of Artificial Sequence:Primer
<400> 6
   gctacagagt gaagtcatgg atcctg 26
<210> 7
   <211> 28
   <212> DNA
   <213> Artificial Sequence
<220>
   <223> Description of Artificial Sequence:Primer
<400> 7
   ggtctgtgtt ccatatgacg tcagtacg 28
<210> 8
   <211> 39
   <212> DNA
   <213> Artificial Sequence
<220>
   <223> Description of Artificial Sequence:Primer
<400> 8
   attacatatg ggtcctaact taggtcagaa ctcagatgc 39
<210> 9
   <211> 38
   <212> DNA
   <213> Artificial Sequence
<220>
   <223> Description of Artificial Sequence:Oligonucleotide probe
<400> 9
   cgttccctgt gatgccacgt tgactgagaa aagctttg 38
<210> 10
   <211> 40
   <212> DNA
   <213> Artificial Sequence
<220>
   <223> Description of Artificial Sequence:Oligonucleotide probe
<400> 10
   tgagaaagtc caaaaactcg gggtaccatt ccaccataga 40
<210> 11
   <211> 45
   <212> DNA
   <213> Artificial Sequence
<220>
   <223> Description of Artificial Sequence:Oligonucleotide probe
<400> 11
   ggagttaacc tggttgtgtc tcagcagttt ctttggactc ctgtg 45
<210> 12
   <211> 13
   <212> DNA
   <213> Artificial Sequence
<220>
   <223> Description of Artificial Sequence:Linker
<400> 12
   gatccgttaa cgc 13
<210> 13
   <211> 13
   <212> DNA
   <213> Artificial Sequence
<220>
   <223> Description of Artificial Sequence:Linker
<400> 13
   gcaattgcgc cgg 13
<210> 14
   <211> 28
   <212> DNA
   <213> Artificial Sequence
<220>
   <223> Description of Artificial Sequence:Primer
<400> 14
   ggattcaaag gttaccttat gtggccac 28
<210> 15
   <211> 23
   <212> DNA
   <213> Artificial Sequence
<220>
   <223> Description of Artificial Sequence:Primer
<400> 15
   gccccaaagg cctacccgct tcc 23
<210> 16
   <211> 28
   <212> DNA
   <213> Artificial Sequence
<220>
   <223> Description of Artificial Sequence:Polylinker oligonucleotide
<400> 16
   aattcggccg gccccgcggg gcgcgccg 28
<210> 17
   <211> 28
   <212> DNA
   <213> Artificial Sequence
<220>
   <223> Description of Artificial Sequence:Polylinker oligonucleotide
<400> 17
   aattcggcgc gccccgcggg gccggccg 28
<210> 18
   <211> 46
   <212> DNA
   <213> Artificial Sequence
<220>
   <223> Description of Artificial Sequence:Oligonucleotide probe
<400> 18
   gtctctactt tactaaaaat acaaaaatta gccaggtgtg gtggtg 46
<210> 19
   <211> 40
   <212> DNA
   <213> Artificial Sequence
<220>
   <223> Description of Artificial Sequence:oligonucleotide probe
<400> 19
   gtcacagggc tgagggaagg agacaagagc ctggacagca 40
<210> 20
   <211> 25
   <212> DNA
   <213> Artificial Sequence
<220>
   <223> Description of Artificial Sequence:Primer
<400> 20
   atcctttctc ttgaccatgg ccatc 25
<210> 21
   <211> 25
   <212> DNA
   <213> Artificial Sequence
<220>
   <223> Description of Artificial Sequence:Primer
<400> 21
   gctggaccac agccgcagcg tcatg 25
<210> 22
   <211> 17
   <212> DNA
   <213> Artificial Sequence
<220>
   <223> Description of Artificial Sequence:Oligonucleotide probe
<400> 22
   tgtctccccg tcccaat 17

## Description

### FIELD OF THE INVENTION

The present invention relates to transgenic non-human animals capable of producing functional human T-cell receptors (TCRs), heterologous Major Histocompatibility Complex (MHC) molecules and co-receptor molecules as well as methods and transgenes for producing the transgenic animals. The animals and heterologous proteins produced are useful for a variety of applications including development of novel therapeutics and vaccines.

### BACKGROUND OF THE INVENTION

The biopharmaceutical industry has been built on the success of developing protein agents as therapeutics, e.g. for treating diseases in humans and animals. The development of biopharmaceuticals has been driven by the use of recombinant DNA technology or genetic engineering to clone and express the proteins of interest and engineer their manufacture at commercial scale. The field has evolved to the point where it is recognized and accepted that the proteins produced for use in humans should contain as much human sequence as possible to insure that the protein therapeutic will be less likely to elicit an antibody response in the patient being treated. This has led to a series of developments for producing more fully human proteins.

The human immune response system is a highly complex and efficient defense system against invading organisms. Recently, there has been a surge of interest in using components of the body's own immune system as therapeutic agents to either modulate or induce an immune attack in a disease state or to inhibit an attack in an autoimmune disorder. Therapeutic molecules that mimic native immune system components would integrate into the body's natural defense mechanisms and thus would provide an efficient method of treatment for such diseases. As a result of such efforts, a number of antibody products have recently been developed and approved as therapeutics for human use. Many of these were originally developed as murine monoclonal antibodies, however murine antibodies generally elicit a human-anti-mouse-antibody (HAMA) response in which the patient's immune system produces antibodies against the therapeutic antibody. In response to such effects, methods were developed to create chimeric antibodies or "humanized" antibodies, in which the murine constant regions or the framework regions of the antibody were replaced with human sequences. Another approach has been to create transgenic animals in which the murine antibody genes have been deleted or inactivated and replaced with human antibody genes (Lonberg and Kay, US Pat. 5,877,397, Kucherlapati et al. US 6,150,584 A, Abgenix Inc. WO 98/24893 A2). These transgenic animals are capable of producing human antibodies in response to vaccination.

T-cells are the primary effector cells involved in the cellular response. Just as antibodies have been developed as therapeutics, (TCRs), the receptors on the surface of the T-cells, which give them their specificity, have unique advantages as a platform for developing therapeutics. While antibodies are limited to recognition of pathogens in the blood and extracellular spaces or to protein targets on the cell surface, TCRs recognize antigens displayed by MHC molecules on the surfaces of cells (including antigens derived from intracellular proteins). Depending on the subtype of T-cells that recognize displayed antigen and become activated, TCRs and T-cells harboring TCRs participate in controlling various immune responses. For instance, helper T-cells are involved in regulation of the humoral immune response through induction of differentiation of B cells into antibody secreting cells. In addition, activated helper T-cells initiate cell-mediated immune responses by cytotoxic T-cells. Thus, TCRs specifically recognize targets that are not normally seen by antibodies and also trigger the T-cells that bear them to initiate wide variety of immune responses.

A T-cell recognizes an antigen presented on the surfaces of cells by means of the TCRs expressed on their cell surface. TCRs are disulfide-linked heterodimers, most consisting of α and β chain glycoproteins. T-cells use recombination mechanisms to generate diversity in their receptor molecules similar to those mechanisms for generating antibody diversity operating in B cells (Janeway and Travers, Immunobiology 1997). Similar to the immunoglobulin genes, TCR genes are composed of segments that rearrange during development of T-cells. TCR polypeptides consist of variable, constant, transmembrane and cytoplasmic regions. While the transmembrane region anchors the protein and the intracellular region participates in signaling when the receptor is occupied, the variable region is responsible for specific recognition of an antigen and the constant region supports the variable region-binding surface. The TCR α chain contains variable regions encoded by variable (V) and joining (J) segments only, while the β chain contains additional diversity (D) segments.

The V, D and J segments of the TCR chains are present in multiple copies in germline DNA. Diversity of the T-cell repertoire and the ability to recognize various antigens is generated through a random recombination process that results in joining of one member of each segment family to generate a single molecule encoding a single TCR α or β chain. While this rearrangement process occurs at both alleles in the T-cell, allelic exclusion result in only one TCR expressed per T-cell (Janeway and Travers, Immunobiology, 1997).

A TCR recognizes a peptide antigen presented on the surfaces of antigen presenting cells in the context of self- (MHC) molecules. Two different types of MHC molecules recognized by TCRs are involved in antigen presentation, the class I MHC and class II MHC molecules. Mature T-cell subsets are defined by the co-receptor molecules they express. These co-receptors act in conjunction with TCRs in the recognition of the MHC-antigen complex and activation of the T-cell. Mature helper T-cells recognize antigen in the context of MHC class II molecules and are distinguished by having the co-receptor CD4. Cytotoxic T-cells recognize antigen in the context of MHC class I determinants and are distinguished by having the CD8 co-receptor.

Due to the specificity of TCRs and their ability to recognize various threats and initiate a natural immune response, TCRs are currently being evaluated for use as a platform for developing therapeutics. In one example, human TCRs are chemically conjugated to an anti-cancer drug, so as to use the specificity of the TCR to guide and deliver the drug to cells that the TCR can recognize. In another example, the TCR gene is genetically fused (or chemically conjugated) to a biologically active protein (e.g. cytokine, chemokine or lymphokine), and thus delivers or directs the active agent to the site of action by means of the TCR specificity. In a third example, TCRs are linked to an antibody specific for a cell type so that the antibody can recruit an effector cell and target or guide the effector cell to the vicinity of the target cell, which the TCR recognizes. In a further example a murine T-cell hybridoma cell line lacking endogenous TCR chains, expressing the human α and β variable regions and the human β constant region has been developed (Chung S. et al., Proc. Natl. Acad. Sci., 1994). In still another example transgenic mice containing a single rearranged human TCR β chain gene that is specific to a particular peptide have been developed (Rothe J. et al., International Immunology, 1993; Viney J.L. et al., Hybridoma, 1992).

Complications encountered when using non-human antibodies as therapeutics provide ample justification for the desire to use human TCRs as the basis for TCR therapeutics for human use. Human TCRs should significantly reduce the chances of developing an antibody response against TCR-based therapeutics, and improve functional interactions necessary for initiation of an efficient, desired cell mediated immune response. Thus, a consequence of efforts to develop TCR-based therapeutics is an interest in having the means to elicit the production of appropriate human TCRs for use in developing such therapeutics.

A current method for isolating TCRs which recognize and react with a desired antigen rely on vaccinating a host with an antigenic protein or peptide in order to elicit a T-cell response or finding a naturally immunized source expressing suitable T-cells. Once an appropriate source is created or identified, T-cells specific for the desired antigen can be propagated, immortalized and screened to identify an appropriate TCR.

The present approaches for identification and production of human TCRs pose difficulties for groups requiring highly specific receptors. These approaches are laborious, expensive and time-consuming means for identifying and producing desired TCRs. Additionally, the described approaches do not always result in selection of TCRs that can effectively recognize a specific antigen of interest. Further, there are obvious limitations on the use of experimental vaccinations in order to elicit human T-cell responses. Finally, TCRs recognizing self-antigens (self antigens are often over-expressed in cancerous cells) are not often found in abundance due to tolerance effects.

A further limitation of current approaches is isolation of specific, high affinity TCRs. Generation of co-receptor independent, human TCR molecules may result in high affinity TCRs that would be more effective in recognizing and participating in functional interactions with antigenic peptide displayed in the context of human MHC molecules.

In view of the above, it is apparent that a need exists for a method to obtain human TCR molecules that are functional, recognize specific antigens of interest, and can be produced readily and in significant amounts. It would therefore be desirable to have methods for engineering the efficient production of heterologous TCRs.

### SUMMARY OF THE INVENTION

The present invention relates to transgenic non-human animals and methods for making the same that are capable of producing human TCR with a substantial TCR repertoire, comprising inactivated endogenous T-cell receptor loci which are α and β chain receptor loci; and transgenes contained within its genome composed of human T-cell receptor loci which are α and β chain receptor loci, wherein said human T-cell receptor loci are unrearranged. Such transgenic animals are capable of producing a repertoire of T-cells expressing human TCRs. Immunizing the transgenic animal with a protein or peptide of interest allows for production of T-cells specific for that antigen. Furthermore, the invention provides for production of co-receptor independent TCRs which produce high affinity, efficient, discriminatory molecules capable of effectively participating in functional interactions.

In one aspect of the invention the transgenic non-human animals have inactivated endogenous TCR loci which are α and β chain receptor loci and carry in the genome transgenes encoding heterologous human TCR loci which are α and β chain receptor loci. The inactivated TCR loci are the α and β chains of endogenous TCRs that can be inactivated through a functional disruption which may include deletion of any one of the V, D,J, or C regions. Alternatively, the functional disruption may include mutations or deletions of regulatory regions such as the promoter region of the gene.

Heterologous transgenes of the animal encode unrearranged α and β loci of the human TCR that are capable of undergoing functional rearrangement of the V, D, J, or C genes of the loci such that the transgenic animal is capable of producing functional human TCRs that are necessary for T-cell maturation. The transgenic non-human animals of the invention are also capable of producing a repertoire of human TCRs that bind particular antigens with specificity and high affinity.

In one embodiment, the non-human transgenic animal also carries within the genome at least one transgene that has sequences of human MHC genes (HLA) contained within the transgene. The transgene may contain a portion of HLA genes such as HLA-A2. More preferably the transgene may contain all of the human HLA genes for MHC class I or MHC class II molecules. Still, most preferably, the non-human transgenic animal will carry transgenes containing sequences of all human MHC genes, class I and class II, such that the animal will have the ability to produce a wide variety of MHC molecules to allow for presentation of a variety of antigenic peptides to T-cells. The genes encoding MHC contained within the transgenes may be unrearranged, partially rearranged, or fully rearranged from that of the germline sequence of the locus, as long as expression of the desired molecules is properly obtained in the transgenic animal. The heterologous α and β chain TCRs produced by the animals facilitate recognition and reaction of the T-cell with the heterologous MHC molecule-antigen presenting complex in order to initiate an immune response to the antigen.

Another embodiment of the invention includes at least one gene encoding one of the two types of co-receptor molecules that are included in the genome of the above-described transgenic animals. Preferably, the transgenic animal will harbor and express genes for both co-receptors CD4 and CD8. The presence of expressed co-receptors further facilitates the T-cell response generated by antigen presented by heterologous MHC molecule. Co-receptors incorporated into the heterologous TCR complex differentially recognize MHC molecules (CD4-TCR complexes preferentially recognize MHC class II complexes while CD8-TCR complexes preferentially recognize MHC class I complexes), are highly sensitized to antigen presenting MHC complexes and initiate immune response to antigen more efficiently than TCR complexes alone.

The heterologous molecules produced, such as TCRs or MHCs, are human molecules. However, heterologous molecules derived from other sources may serve analogous purposes. For example, heterologous molecules derived from a particular animal such as dog or horse for instance may be used for development of veterinary therapeutics.

A preferred non-human transgenic animal host for the present invention is a mouse, however, any animal that can be manipulated transgenically and has an immune system capable of carrying out required recombination and expression events of the present invention may serve as a non-human transgenic animal host. Additionally preferred animals include, but are not limited to, rat, chimpanzee, other primates, goat, pig, or zebrafish.

Another aspect of the invention includes methods of producing non-human transgenic animals capable of producing human TCR with a substantial TCR repertoire. Inactivation of endogenous T-cell receptor loci which are α and β chain receptor loci and insertion of transgenes encoding heterologous human T-cell receptor loci, which are α and β chain loci are required for production of the animals. This may be accomplished by a number of steps. An animal may be produced from a non-human embryo or embryonic stem cell that has had endogenous TCR loci, which are α and β chain loci functionally disrupted and carries transgenes containing heterologous human α and β TCR loci.

Disrupted endogenous loci of non-human animals further comprise in preferred embodiments MHC class I, MHC class II, CD4 and/or CD8 loci. The endogenous genes may be disrupted through any one of a number of means. Preferably, disruption may occur through incorporation by homologous recombination of targeting sequences that disrupt specific sequence for the locus. At the TCR α or β locus, this may include targeting a deletion of required sequences such as the V, D, J, or C regions. Alternatively, targeted disruptions may cause a mutation or deletion in the promoter or other regulatory sequence that results in a functional disruption of the locus. Other preferred methods may include use of the *cre-lox* recombination system or anti-sense methods to cause a functional disruption of expression of the locus.

The transgenes carried by the animals further comprise in preferred embodiments transgenes containing a heterologous MHC class I and/or MHC class II loci, and/or CD4 and CD8 genes. The transgenes containing heterologous TCR loci encompass the germline sequences of the V, D, and/or J, and C regions of the α and β chains of the TCR loci. The sequences are unrearranged in order to allow for production of various species of TCRs. The transgenes may also include regulatory sequences of the loci in order to maintain functioning of the transgene. The regulatory sequences may be derived from the same heterologous source as the gene sequences. Alternatively, regulatory sequences may be derived from the endogenous species.

Another preferred method for producing the non-human transgenic animals includes creation of non-human transgenic animals from non-human embryos or non-human embryonic stem cells that have one disrupted locus or inserted transgene. Creation of the non-human transgenic animal then consists of breeding one animal with a disruption with another animal containing the same disruption to create progeny animals that are homozygous for the disruption. Upon creation of homozygotes, these animals are bred with homozygous animals having another desired disruption and progeny selected that have homozygous double disruptions. Similarly, animals are created which carry two transgenes by breeding animals each carrying within their respective genomes a transgene of interest. An animal carrying endogenous disruptions and transgenes can be produced through breeding selected animals carrying homozygous disruptions with animals having the transgenes contained in their genome and their progeny selected so as to have homozygous double mutations and carrying transgenes of interest. The steps of breeding need not be carried out in the above mentioned order. Rather, breeding of animals may be carried out in any order as long as selection for the desired genotype is obtained in progeny animals.

Nucleic acid molecules serve as transgene constructs encoding heterologous molecules.

The transgenes of the invention include heterologous TCR and/or MHC constructs. Additional transgene constructs include co-receptors CD4 and/or CD8.

The transgenes of the invention include a TCR β chain transgene comprising DNA encoding at least one V gene segment, at least one D gene segment, at least one J gene segment and at least one C region gene segment. The invention also includes a TCR α chain transgene comprising DNA encoding at least one V gene segment, at least one J gene segment and at least one C region gene segment. The gene segments encoding the α and β chain gene segments are heterologous to the transgenic non-human animal in that they are derived from, or correspond to, germline DNA sequences of TCR α and β gene segments from a species not consisting of the non-human host animal.

In one embodiment of the invention, heterologous α and β TCR transgenes comprise relatively large fragments of unrearranged heterologous DNA (*i.e*., not rearranged so as to encode a functional TCR α or β chain). Preferably all of the genes of the α and β loci are included in the transgenes. Such fragments typically comprise a substantial portion of the C, J (and in the case of β chain, D) segments from a heterologous TCR locus. In addition, such fragments also comprise a substantial portion of the V gene segments. Such unrearranged transgenes permit recombination of the gene segments (functional rearrangement) and expression of the resultant rearranged TCR α and/or β chains within the transgenic non-human animal when said animal is exposed to antigen, to generate a repertoire of TCRs. Alternatively, the transgenes may comprise partially rearranged or completely rearranged TCR loci in order to produce a subset of TCRs.

Such transgene constructs may additionally comprise regulatory sequences, e.g. promoters, enhancers, recombination signals and the like, corresponding to sequences derived from the heterologous DNA. Alternatively, such regulatory sequences may be incorporated into the transgene from the same or a related species of the non-human animal used in the invention. For example, human TCR gene segments may be combined in a transgene with a rodent TCR enhancer sequence for use in a transgenic mouse.

Another embodiment of the invention includes heterologous MHC loci transgenes. The MHC transgenes comprise DNA sequence encoding at least one heterologous MHC molecule, such as HLA-A2. More preferred are transgenes encoding some or all of a class of MHC class I or MHC class II molecules. Some or all of the transgenes may include germline MHC loci sequences. The MHC transgenes may be rearranged genes, partially rearranged or unrearranged such that the non-human animal carrying the transgene is able to express the encoded molecules.

Yet another embodiment of the invention includes co-receptor transgenes. The co-receptor transgenes comprise DNA sequence encoding co-receptors molecules with an extracellular domain of a co-receptor gene linked to a transmembrane and cytoplasmic domain of a co-receptor gene, where the domains may be from homologous or heterologous sources. These co-receptor transgenes may encode CD4 and/or CD8.

In a preferred embodiment, MHC loci (MHC class I and/or MHC class II) and co-receptors CD4 and/or CD8 are derived from the same heterologous source. Alternatively, MHC loci and co-receptors may be derived from closely related sources. Additionally, co-receptors CD4 and/or CD8 may be chimeric genes, where an extracellular domain derived from one heterologous source is fused to a transmembrane and cytoplasmic domain of either a different heterologous source, or a homologous source.

Nucleic acid molecules to be used in the invention to disrupt the endogenous loci in the non-human animal, utilize homologous segments of DNA, preferably on a vector with positive and negative selection markers, which is constructed such that it targets the functional disruption of a locus. The targeted disruption includes a class of gene segments encoding an α and/or β chain TCR endogenous to the non-human animal used in the invention. Such endogenous gene segments can include D, J and C region gene segments. Additional sequences may be targeted, for example regulatory sequences such as for example, the promoter where a targeted disruption will result in loss of function of the locus.

Additional embodiments include targeted disruption of endogenous MHC loci (MHC class I and/or class II), and/or co-receptor loci, CD4 and/or CD8.

Methods of utilizing the invention are included. The positive-negative selection vector is contacted with at least one non-human embryo or embryonic stem cell of a non-human animal after which cells are selected wherein the positive-negative selection vector has integrated into the genome of the non-human animal by way of homologous recombination. After transplantation, the resultant transgenic non-human animal is substantially incapable of mounting an endogenous TCR-mediated immune response as a result of homologous integration of the vector into chromosomal DNA. Such immune deficient non-human animals may thereafter be used for study of immune deficiencies, study of passive T-cell function, models for study of cancer and cancer therapeutics, or used as the recipient of heterologous transgenes.

T-cells from such transgenic animals that are capable of expressing heterologous TCRs may be immortalized to provide a source of a TCR specific for a particular antigen. T-cells may be selected for specificity so as to react with a particular antigen and/or peptide-MHC complex. Hybridoma cells that are derived from such T-cells can serve as one source of such heterologous TCR.

The T-cells and/or derived hybridoma cells can also serve as a source of mRNA for the preparation of cDNA libraries from which loci encoding alpha and beta chains for the heterologous TCRs can be cloned. Such cloned TCR genes can be expressed in recombinant mammalian cells to produce heterodimeric TCRs. The cloned TCR genes can also be genetically manipulated so as to provide for the expression in recombinant mammalian cells of soluble, single-chain TCRs.

Producing immortalized cell lines may be achieved by fusing a selected T-cell of interest with an immortalizing cell line, e.g. a myeloma cell line.

Heterologous TCRs and TCR complexes that may or may not include chimeric CD4 or CD8 may or may not be purified or partially purified. Additionally heterologous TCRs may be specific for a particular antigen-MHC or peptide-MHC complex.

To induce: an immune response in the aforementioned transgenic non-human animal to induce heterologous TCRs of various specificities, a preferred method includes one where a cell mediated response is initiated in a non-human transgenic animal by administering to the animal an effective amount of an antigen, whether a peptide or protein of interest. With these immunogens it is possible to induce the animal to initiate an antigen-stimulated response and produce T-cells that express human TCRs specific to that antigen. Such T-cells can be identified by conventional methods and can be purified if desired and assayed for capacity to undergo proliferation or any of the uses described above.

### BRIEF DESCRIPTION OF THE DRAWINGS

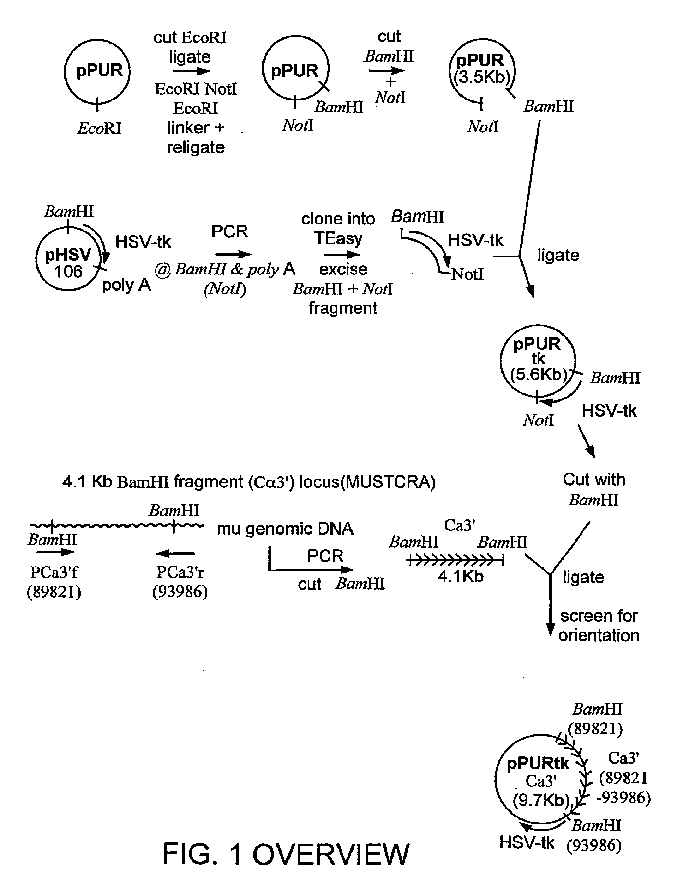

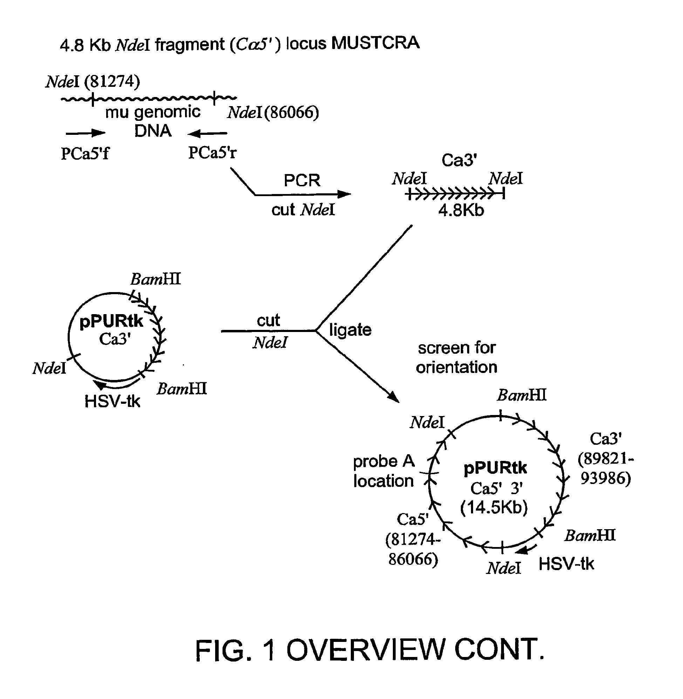

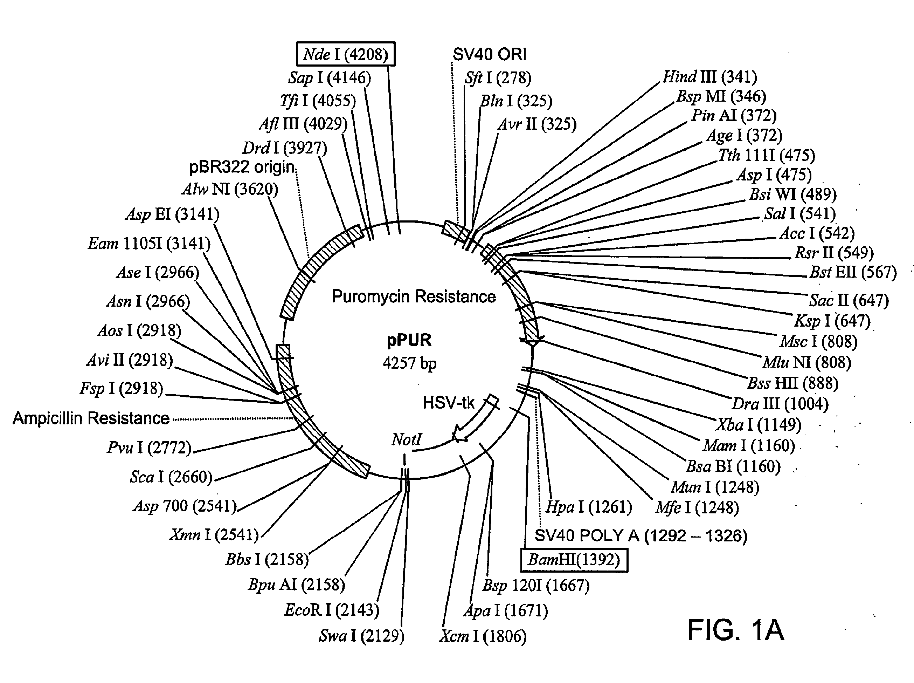

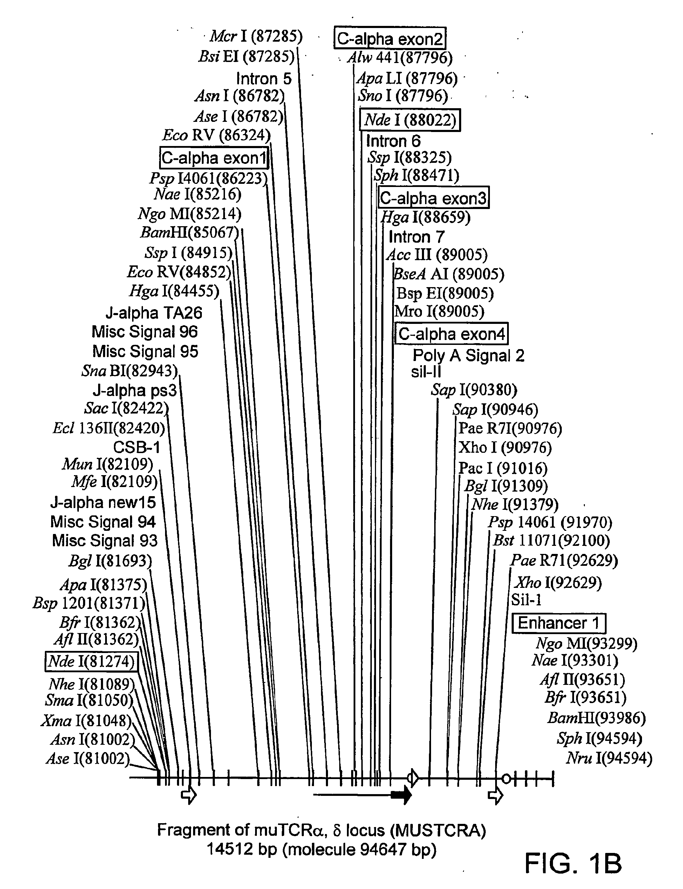

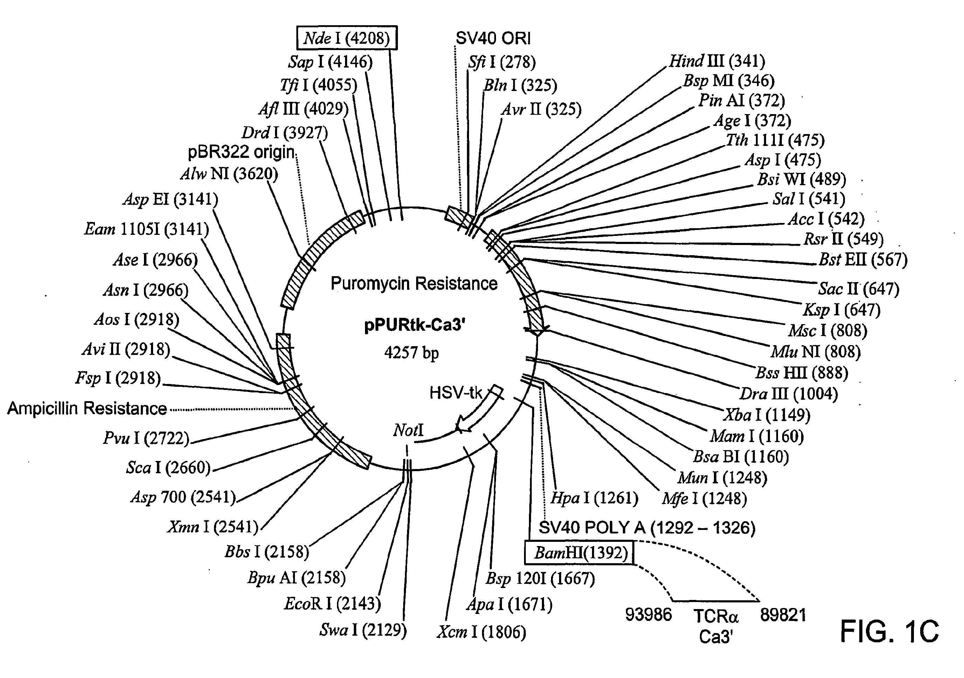

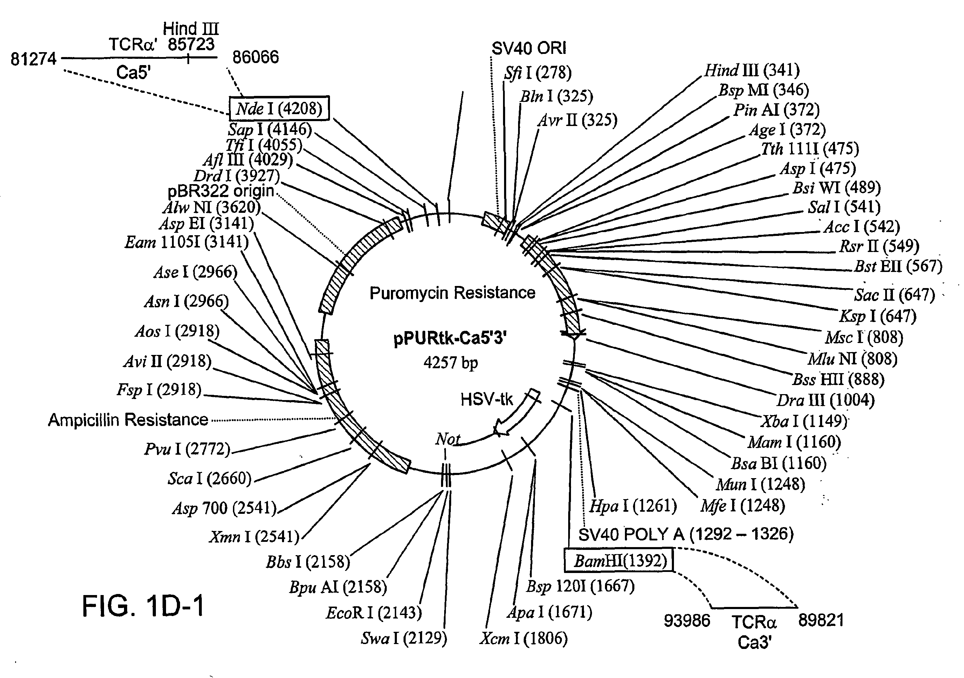

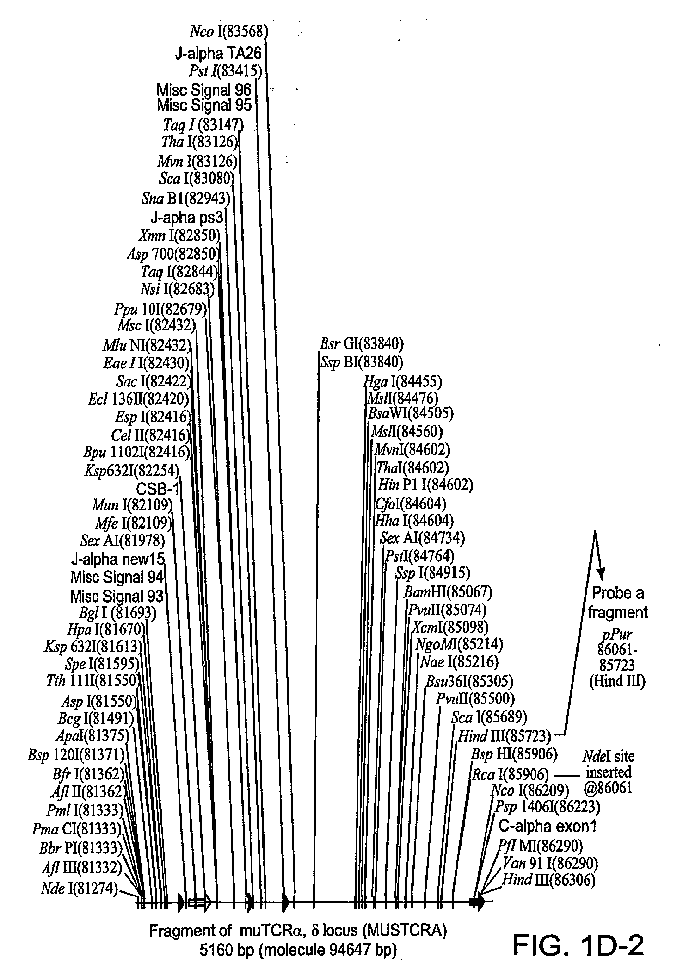

Figure 1 is a diagrammatic illustration showing an overview of the main procedural steps used in the construction of murine T-cell receptor α locus knockout constructs.
Figure 1A is a schematic illustration showing the constructed plasmid pPRtk, comprising unique *Nde*I and *BamH*I sites.
Figure 1B is a schematic illustration showing the general steps for isolation, amplification and insertion of the 4.1 Kb *BamH*I mouse α chain sequences, specific for the 3' end of the Cα locus.
Figure 1 C is a schematic illustration of the plasmid, pPURtk-Ca3', comprised of the inserted 4.1 Kb *BamH*I 3' end of the Cα locus.
Figure 1D is a schematic illustration showing the procedural steps in the construction of plasmid, pPURtk-Cα5'3', which is comprised of a 4.8 Kb *Nd*eI fragment 5' end of the Cα locus inserted into the pPURtk-Cα3' plasmid.
Figure 1 E is a schematic representation of the pPUR plasmid used in the construction of the alpha targeting vector, pPURtk-Cα5'3'.
Figure 1 F is a schematic representation of the TCRα, d locus (MUSTCRA), showing the positions of the Cα exons 1-4, relative to endonuclease restriction sites.
Figure 1 G is a schematic representation of pPURtk-Cα3', showing the cloning site of the Cα3' of TCRα, relative to the restriction sites present in the plasmid.
Figure 1 H is a schematic representation of pPURtk-Cα5'3', showing the cloning sites of the Cα5' and Cα3' of TCRα, relative to the restriction sites present in the plasmid.
Figure 1 I is a schematic representation of the TCRα, d locus (MUSTCRA), showing the endonuclease restriction positions from which probe A was excised.
Figure 2 is a diagrammatic illustration showing an overview of the main procedural steps used in the construction of murine T-cell receptor β locus knockout constructs. The resultant vector is plasmid pNEOtkCβ5'3'. Indicated in the overview are details from other figures which are incorporated to show how each step in the procedure is conducted. The relevant steps which refer to the corresponding figures are indicated in the boxes, e.g. figure 2a and 2b.
Figure 2 A is a schematic illustration showing the TCRβ locus 3' region wherein probes A and B are generated from.

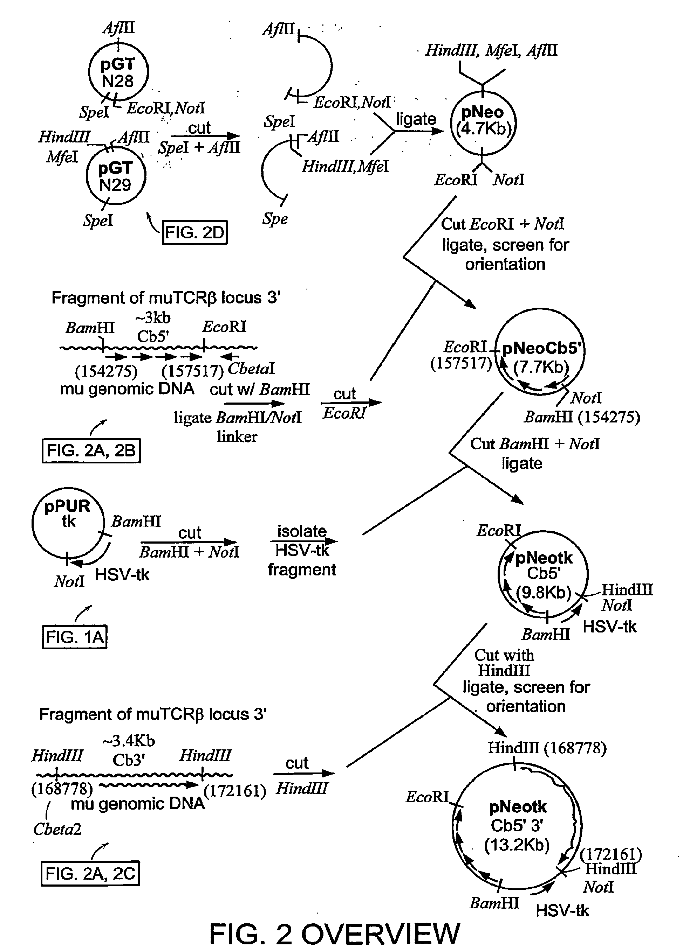

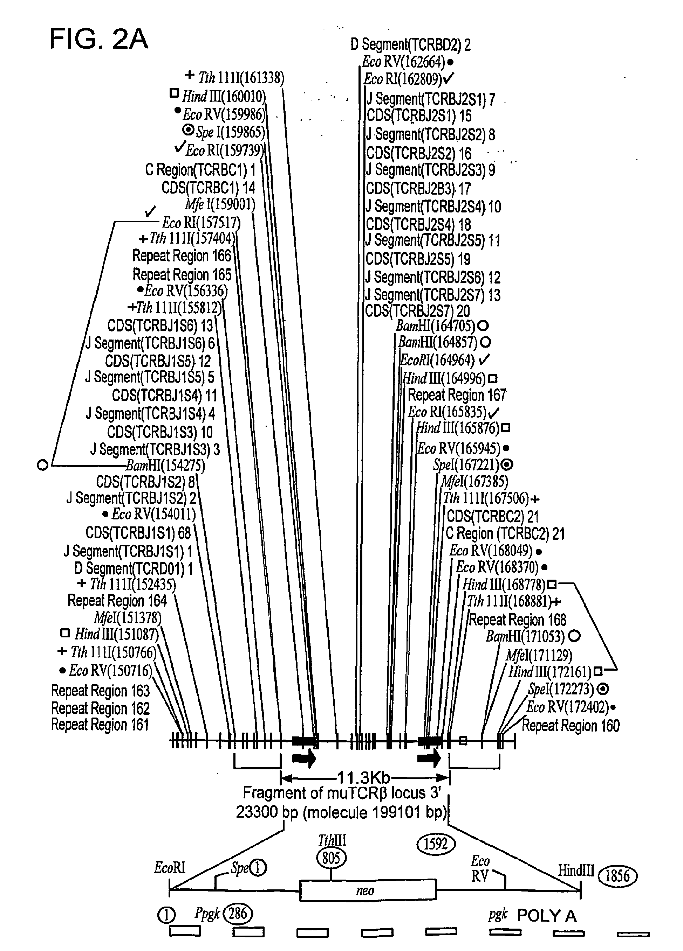

Figure 2 A is a schematic illustration showing the region comprised of the TCR β locus, 5' to Cβ1.

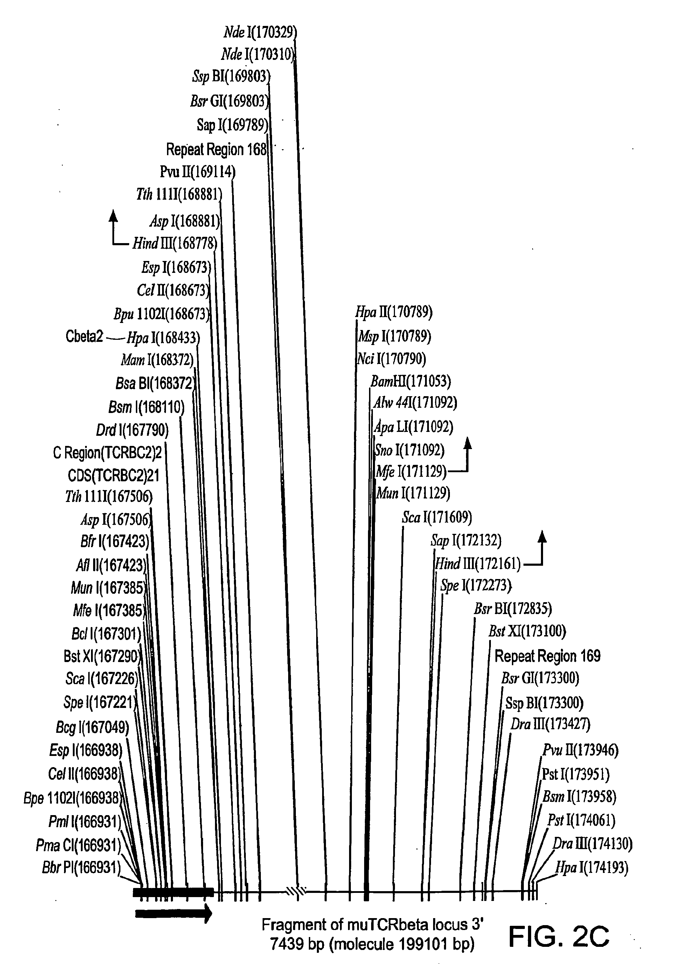

Figure 2 C is a schematic illustration showing the region comprised of the TCR β locus, 3' to Cβ2.

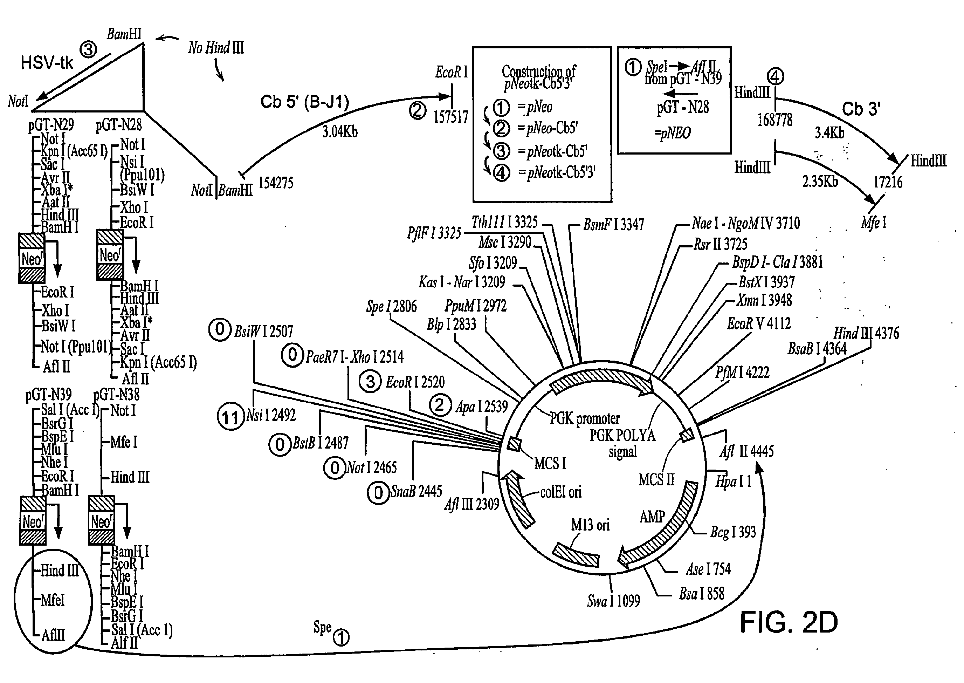

Figure 2 D is a schematic illustration of the vector, showing the restriction sites, used to generate the plasmid pNEOtkCβ5'3'.

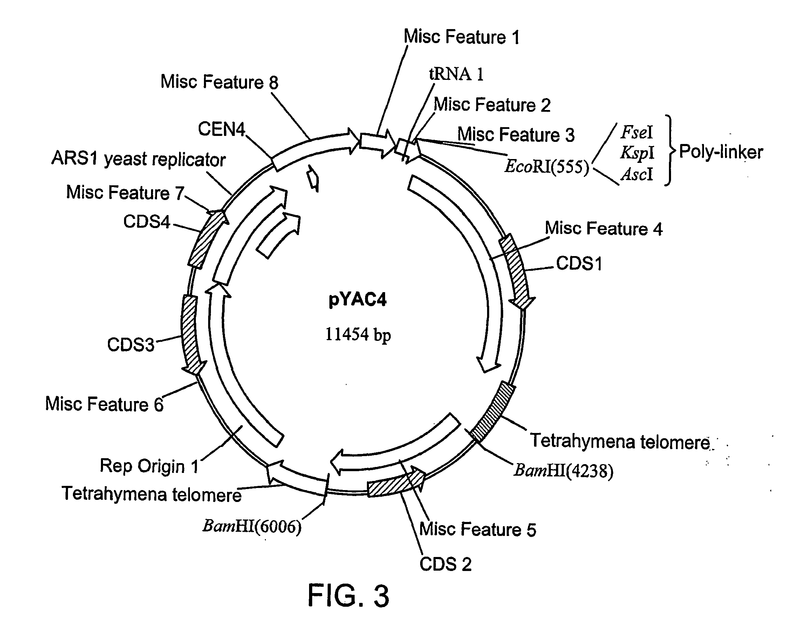

Figure 3 is a schematic illustration which depicts Yeast Artificial Chromosome 4 (pYAC4-Neo).

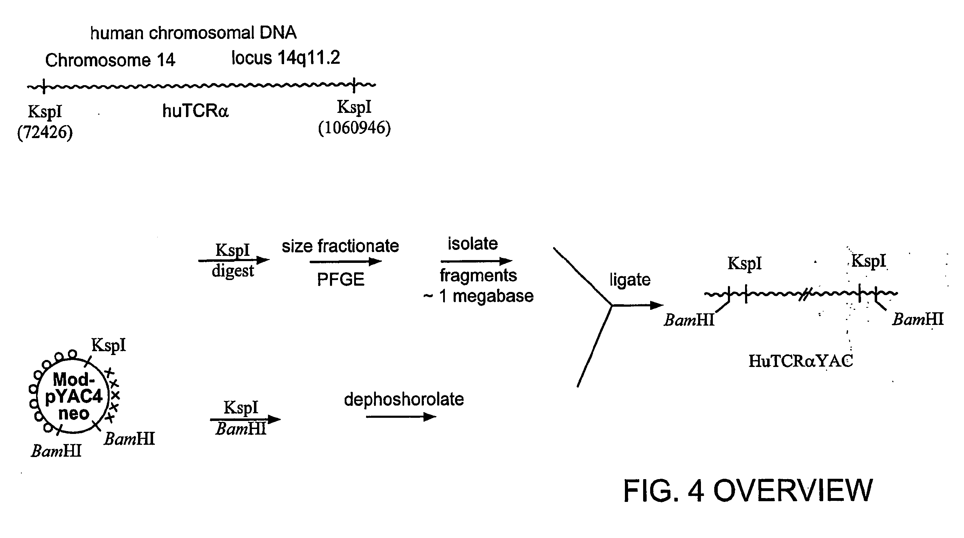

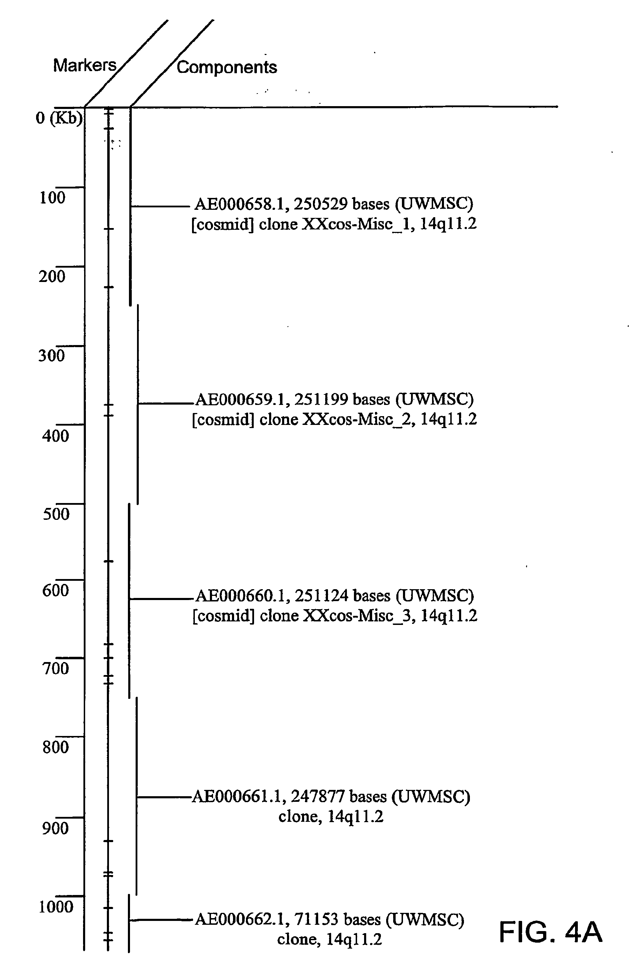

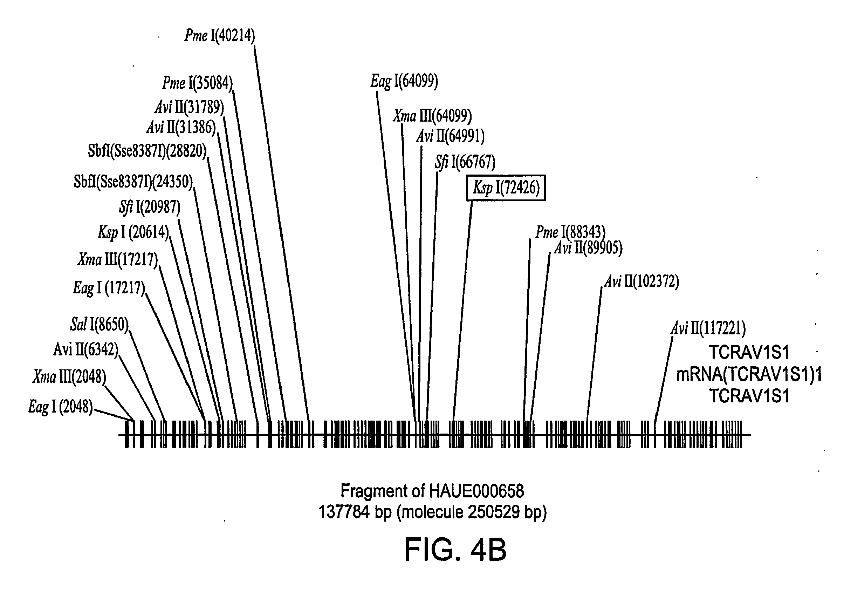

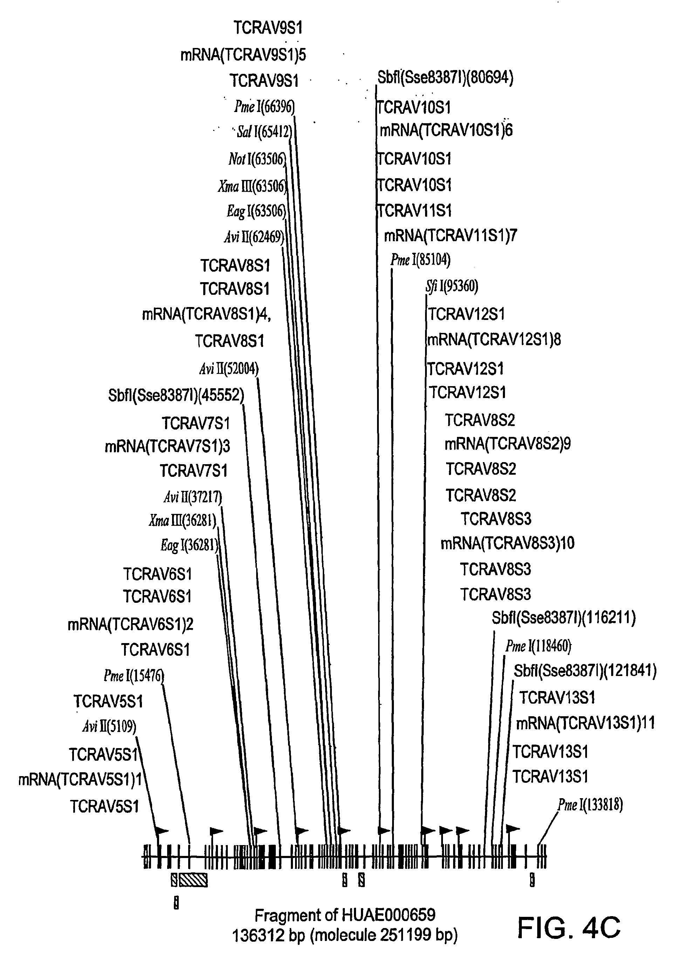

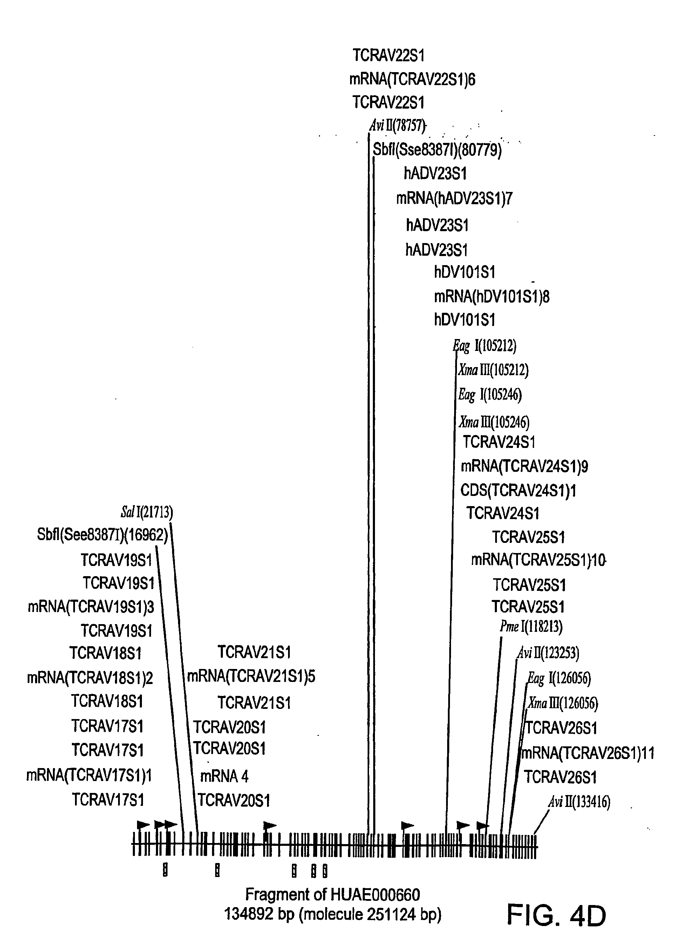

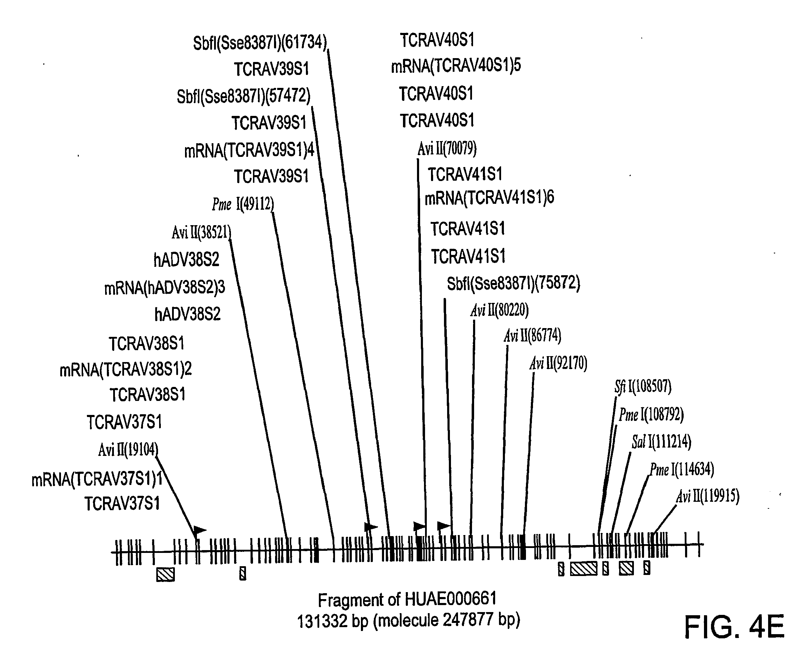

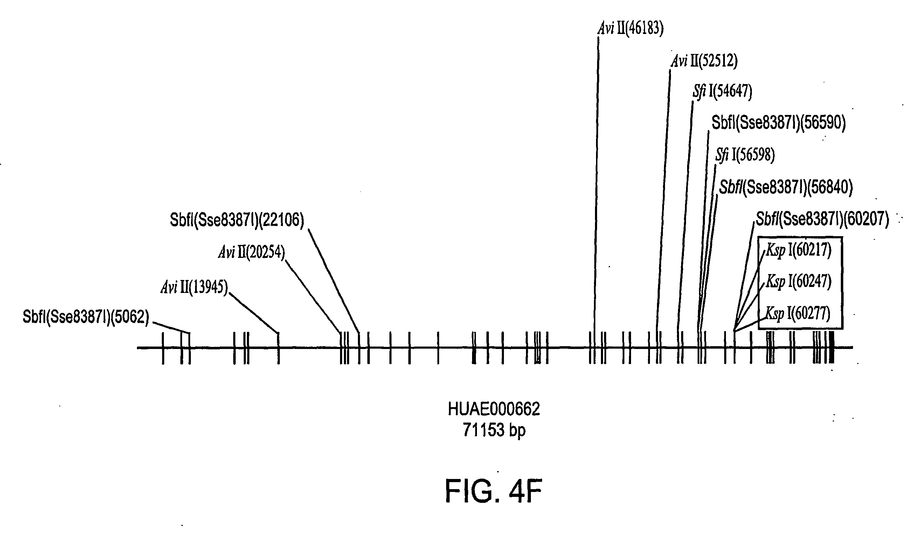

Figure 4 is a schematic illustration showing a general overview of the steps taken for cloning the Human T-cell receptor α locus transgene into the modified pYAC4-neo vector, mod-pYAC4-neo.
Figure 4 A is a schematic illustration showing the chromosomal location of the human TCR alpha locus.
Figure 4 B - F is a schematic illustration showing the restriction map of the human TCR alpha locus.

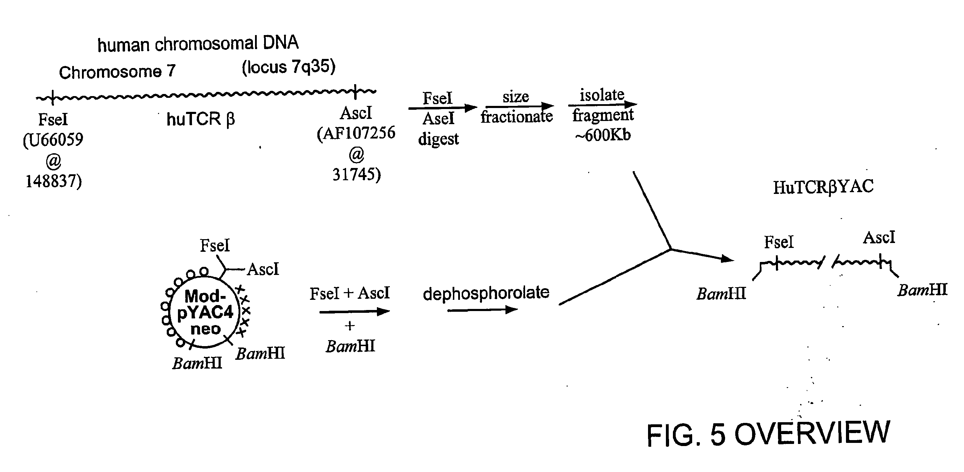

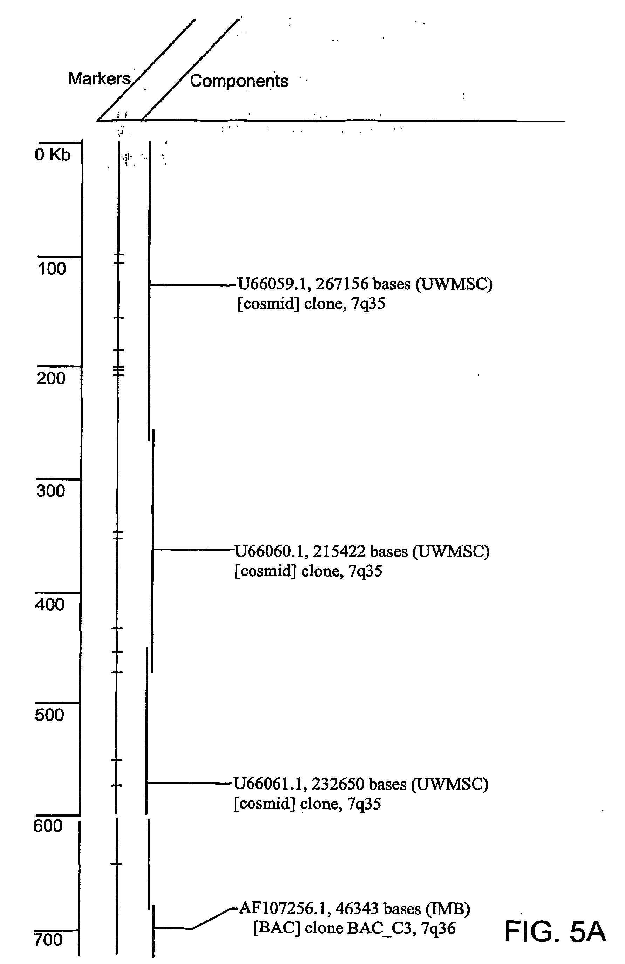

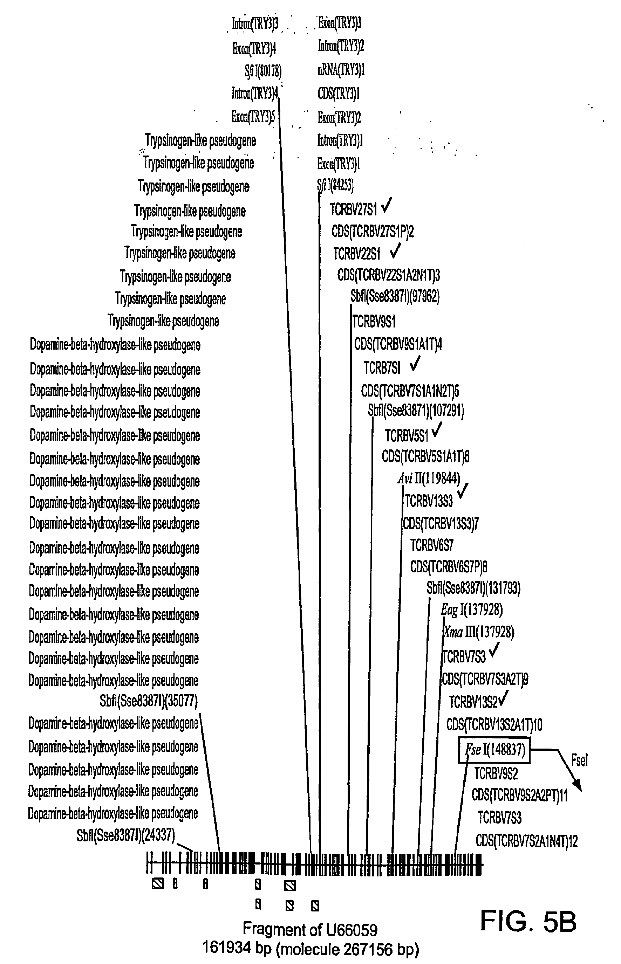

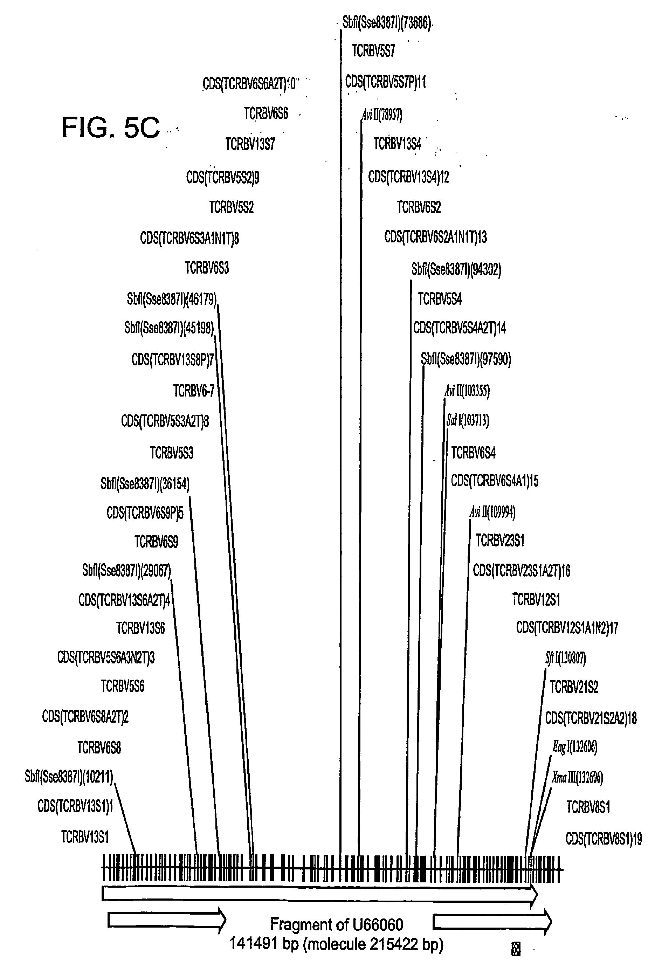

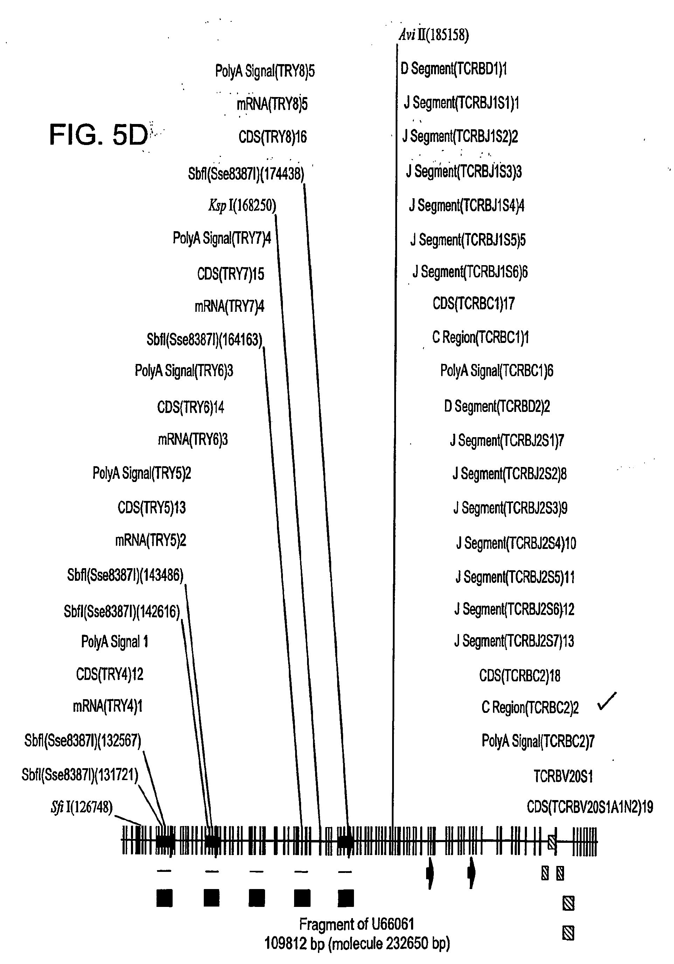

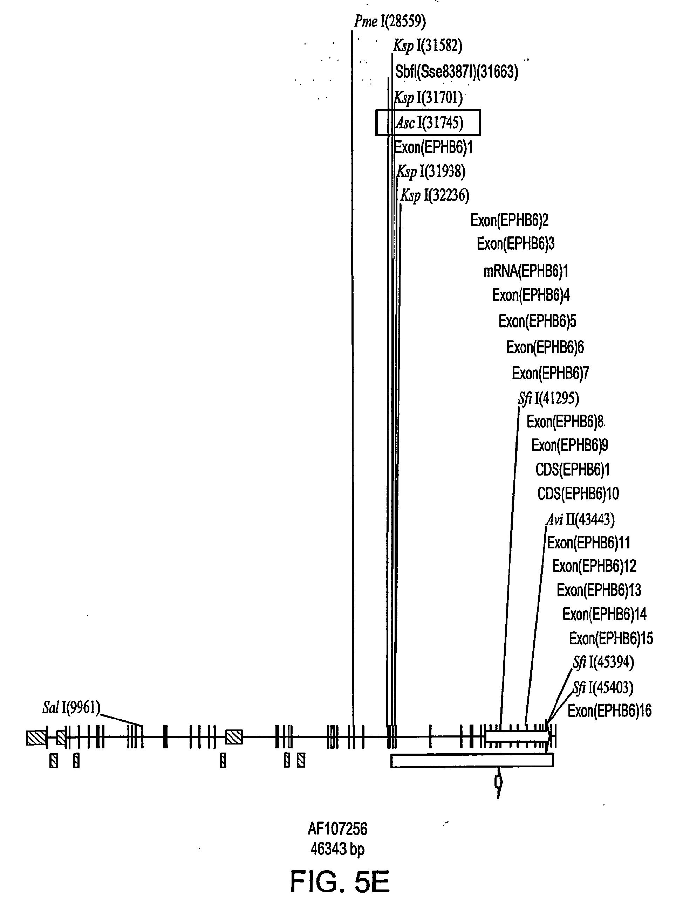

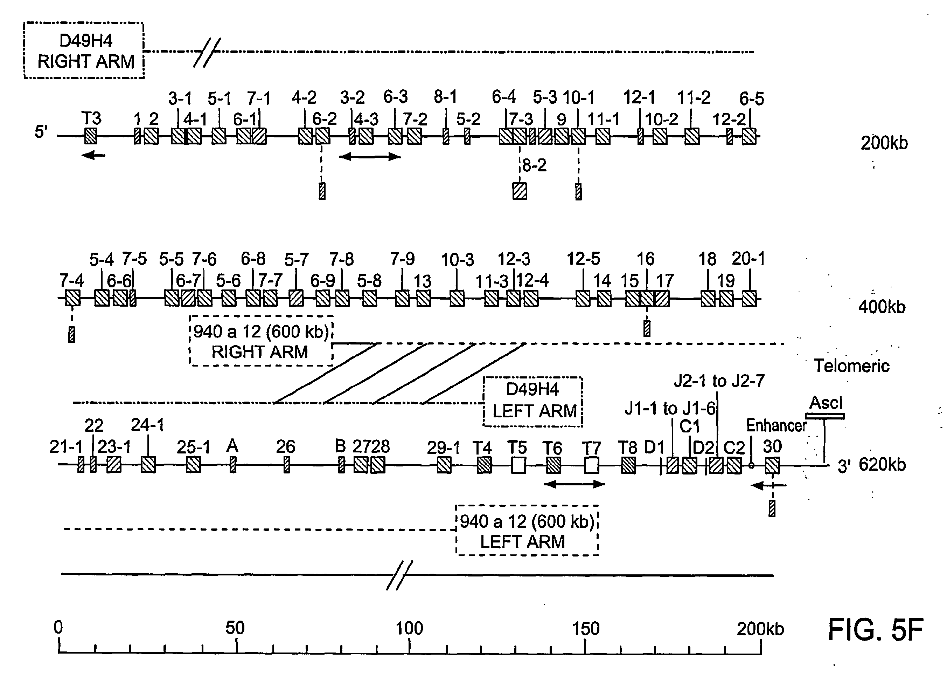

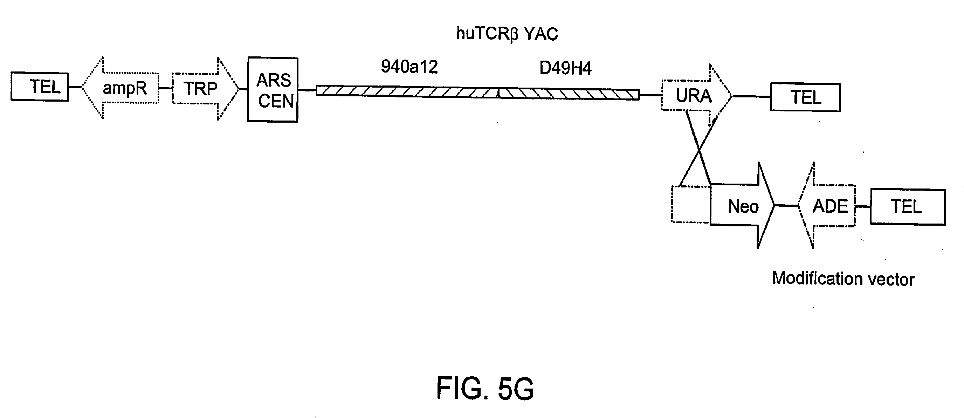

Figure 5 is a schematic illustration which depicts a general overview of the steps used to construct a Human T-cell receptor β locus transgene.
Figure 5 A is a schematic illustration of the chromosomal location of the Human TCR beta locus.
Figure 5 B - E is a schematic representation showing the results obtained from the nucleotide mapping of the Human TCR beta locus.
Figure 5 F is a schematic representation showing the TCRβ chain gene superimposed onto the YAC sequence.
Figure 5 G is a schematic representation of the Human TCR beta YAC vector which illustrates the general regions wherein regulatory sequences and/or mammalian selection cassettes may be inserted.

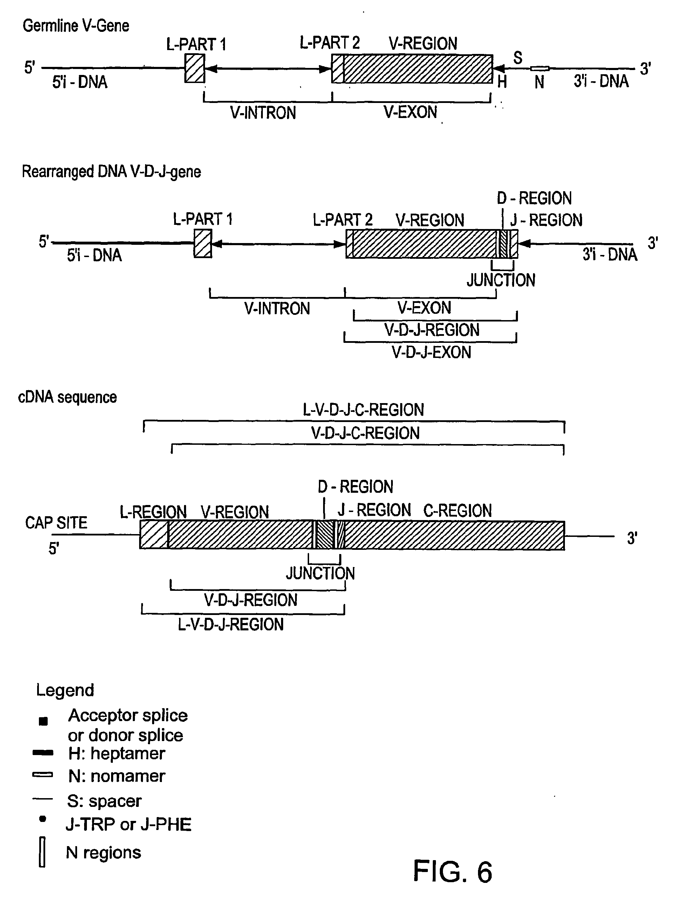

Figure 6 is a schematic representation illustrating the VDJ rearrangement steps of the TCR starting from the unrearranged germline V gene to the rearranged cDNA sequence.

### DETAILED DESCRIPTION OF THE INVENTION

Transgenic non-human animals are provided, as summarized above, which are capable of producing a human TCR with a substantial TCR repertoire. In order for such transgenic non-human animals to produce an immune response, it is necessary for the transgenic pre-T-cells to express surface-bound TCRs so to effect T-cell development, produce mature, functional T-cells, and elicit an effective antigen-stimulated response. Thus, the invention provides transgenic non-human animals harboring heterologous TCR α and β chain transgenes, wherein the transgenic non-human animal is capable of producing human TCR. Such transgenes and transgenic non-human animals produce TCRs that are necessary for T-cell maturation. Transgenic non-human animals of the invention are thus able to produce TCRs that are encoded by heterologous TCR genetic sequences and which also bind specific antigens.

It is often desirable to produce human TCRs that are reactive with specific human antigens which are promising therapeutic and/or diagnostic targets. However, producing human TCRs that bind specifically with human antigens is problematic. The immunized non-human animal that serves as the source of T-cells must mount an effective immune response against the presented antigen. In order for an animal to mount an immune response, the antigen presented must be foreign and the animal must not be tolerant to the antigen. Thus, for example, if it is desired to produce a human TCR that binds to a human peptide in the context of a HLA receptor, self-tolerance will prevent an immunized human from producing an substantial immune response to the human protein, since the only immunogenic epitopes will be those with sequence polymorphisms within the human population. A transgenic non-human animal could be constructed for this application that contains an inactivated murine TCR locus, and active human alpha and beta chain TCR loci and human loci encoding MHC, such as the HLA-A2 receptor for example. Challenge in such an animal with an antigenic peptide or protein would generate human TCRs capable of recognizing the antigen in the context of human HLA.

Furthermore, it is known that class 1 MHC interaction with TCR is enhanced by the presence of a CD8 co-receptor; and for class II MHC, the presence of a CD4 co-receptor. For certain applications, it may be desirable to have human loci for the TCR, MHC and co-receptor so as to have a system that mimics the human immune response. Alternatively, interaction of the human TCR with the human HLA-peptide complex in the absence of a contribution from a human co-receptor might result in a bias in favor of higher affinity TCRs. Such high-affinity TCRs might not arise in the normal endogenous situation, and would be desirable as the basis for therapeutics. Thus; for other applications, it may be desirable to have only the expressed TCR and MHC molecules encoded by human genes, without the human CD co-receptor genes.

The use of such a TCR transgenic non-human animal system is to mimic the generation of heterologous TCRs in response to challenge by antigen, such that the TCRs produced can recognize and interact with the antigen in the context of a heterologous HLA/MHC molecule. Variations of TCR transgenic non-human animals are envisioned. In the examples provided, the first is an animal that can be used to produce high-affinity, fully human TCRs, which recognize antigenic peptide in the context of human HLA molecules. Additional TCR transgenic non-human animals include animals in which some or all of the TCRs, co-receptor molecules and/or HLA molecules are transgenic. In order to create such transgenic animals, the endogenous loci may or may not be inactivated or removed. The heterologous transgene must be introduced into the animal. The advantage conferred by inactivating the endogenous TCR loci is that inactivation eliminate the possibility of a mixed TCR response such that the only response generated is based on heterologous TCRs. In order to create a fully modified TCR transgenic non-human animal, it may also be desirable to inactivate endogenous TCR sources and incorporate MHC / HLA transgenes, as well as CD4 and/or CD8 co-receptors, from the same heterologous source.

As used herein, a "transgene" is a DNA sequence introduced into the germline of a non-human animal by way of human intervention such as by way of the described methods herein.

By the term "endogenous loci" is meant to include the natural genetic loci found in the animal to be transformed into the transgenic host.

"Disruption" or "inactivation" of loci as used herein may include physical disruption of the endogenous locus, or a functional disruption that results in an inability of the locus to perform the required function (*i.e.* expression of a gene or genes correctly).

As used herein, the term "heterologous molecule" is defined in relation to the transgenic non-human organism producing such molecules. It is defined as a molecule having an amino acid sequence or an encoding DNA sequence corresponding to that found in an organism not consisting of the transgenic non-human animal.

In this preferred description, a transgenic mouse is engineered to express α and β chains of the human TCR. The mouse is then capable of producing T-cells bearing TCRs that specifically recognize peptide antigens displayed in the context of an MHC molecule. This transgenic mouse can generate numerous antigen-specific human TCRs that can then be selected for development of novel therapeutics, and/or monitoring agents. It would also provide a basic research tool for studying immune system regulation.

Another envisioned application for such transgenic non-human animals is the development of a human-like host for for evaluating the effectiveness of immunomodulation therapies and/or vaccines. In addition to TCR alteration, this would require the following additional modifications to achieve the fully transgenic host: deletion or inactivation of the native murine MHC I and/or MHC II loci; introduction of partial or whole human HLA loci; deletion or inactivation of the murine CD4 and/or CD8 co-receptors; introduction of the human CD4 and/or CD8 co-receptors or chimeras thereof.

For purposes of this description, the heterologous molecules are of human origin and the non-human animal host is mouse. However, this invention teaches how to produce any heterologous TCR in any non-human animal that can be manipulated transgenically. Additional preferred non-human animals may include for example, rat, primate, chimpanzee, goat, pig, or zebrafish. Heterologous TCRs produced may be any animal for which development of therapeutics, vaccines, or use of TCRs is required. For example, additional sources of heterologous molecules may include any domestic animal for which vaccination development is desired such as dog, cat, horse, *etc*.

The basic approach towards production of the transgenic non-human animals is to inactivate or remove the genetic loci of the mouse TCR and introduce into the mouse DNA the germline sequences of the α and β chain loci of the human TCR. The steps shown in Table 1 can be envisioned as a path toward creating a desired transgenic animal where, for purposes of example, the transgenic host is murine and the heterologous source for the transgenes are human. The order of steps shown in Table 1 is for exemplary purposes only and alternative orders can be considered in order to reach comparable desired endpoints. With the endogenous loci knocked out or inactivated and the heterologous loci introduced (in either order), the transgenic animal thus created can be considered an intermediate in the evolution towards a creation of a mouse capable of producing only human TCR. It should also be pointed out that some of the intermediates might be of value for particular applications as will be discussed below. The transgenic animal with the most extensive human transgene replacements will be useful for evaluating vaccine formulations targeted for human use.

Transgenic non-human animals, having inactivated endogenous loci and harboring transgenes of heterologous TCRs, may be produced through a number of individual steps. Each step consists of matings (or crosses) of animals having individual disruptions and/or transgenes. In this strategy, individual animals are produced from non-human embryos or non-human ES cells which have one endogenous locus of interest disrupted Additionally, in separate steps, animals are produced from non-human embryos or non-human ES cells which harbor a single transgene of interest within their genome. An individual mouse having heterozygous mutations are crossed with mouse having the same heterozygous mutation in order to generate progeny mice that are homozygous for the mutation. This procedure is followed for any desired mutation.

Production of the desired transgenic non-human animals may also be accomplished through additional strategies. For instance, the transgenic animal may be produced from a non-human embryo or non-human embryonic stem (ES) cell having the desired endogenous genetic loci inactivated and having inserted in the genome transgenes which comprise the human TCR α and β loci. Once a non-human embryo or non-human ES cell containing the desired genetic alterations is produced and selected, a non-human transgenic animal having the same genetic alterations is created through the use of the selected non-human embryo or non-human ES cells.

In order to generate mice that are homozygous for two disruptions, parent mice having homozygous mutations of one disruption are crossed with mice homozygous for another desired disruption. In order to generate mice that harbor more than one transgene of interest, parent mice having one transgene contained within their genomes are crossed and progeny selected which contain both transgenes. Finally, once mice have been created which are homozygous for the desired mutations, and mice are created which harbor the desired transgenes, parent mice of each genotype are crossed and progeny selected which are homozygous for all mutations and also contain the desired transgenes. By breeding appropriate intermediate transgenic non-human animals (as shown in steps 1b, 4 and 5 of Table 1), transgenics with more extensive replacements can be obtained. Again, it should be noted that the steps described here need not be carried out in the above mentioned order, but may be shuffled in order to create mice having various intermediate genotypes.

In a preferred embodiment, transgenic non-human animals of the invention will be created by incorporation of the transgenes into the germline of non-human embryos or non-human ES cells. Non-human ES cells can be obtained from pre-implantation non-human embryos cultured *in vitro* (Evans, M. J., et al. (1981) Nature 292:154-156; Bradley, M. O., et al. (1984) Nature 309: 255-258; Gossler, et al. (1986) Proc. Natl. Acad. Sci. 83: 9065-9069; and Robertson, et al. (1986) Nature 322: 445-448). Transgenes may be efficiency introduced into non-human ES cells through a number of means including DNA transfection, microinjection, protoplast fusion, retroviral-mediated transduction, or micelle fusion. Resulting transformed non-human ES cells will then be introduced into a non-human embryo, and result in contribution of transgenic DNA to the animal germ line (for review see Jaenisch, R. (1988) Science 240: 1468-1474).

**TABLE 1**

| |
|---|
| 1a. knock out insert trangene mu TCR → mu TCR- → muTCR- huTCR⁺ |
| 1b. muTCR- huTCR+ X huHLA-A2.1 → huTCR+ huHLA-A2+ |
| 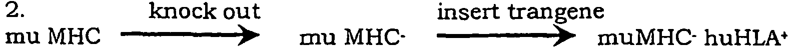 |
| (For this example, the MHC / HLA can be Class I or Class II or both.) |
| 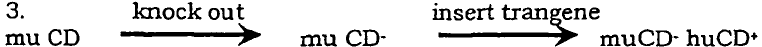 |
| (For this example, the CD coreceptor can be CD4 or CD8 or both.) |
| 4. muTCR-huTCR⁺ X muMHC- huHLA⁺ → muTCR- huTCR⁺ / muMHC⁻ muHLA+ |
| 5. huTCR⁺ / huHLA⁺ X muCD- huCD⁺ → muCD-huCD⁺ / huTCR⁺ / huHLA⁺ |

An alternative method of creation of transgenic non-human animals includes the use of retroviral infection to introduce transgene(s) directly into a non-human animal (Jaenich, R. (1976) Proc. Natl. Acad. Sci. 73: 1260-1264). The developing non-human embryo is cultured to the blastocyst stage, when efficient infection can be obtained through enzymatic treatment. Alternatively, virus or virus-producing cells can be injected into later stage non-human embryos (Hogan, et al. (1986) in Manipulating the Mouse Embryo, Cold Spring Harbor Laboratory Press, Cold Spring Harbor, N.Y.).

Transfer of transgenes to non-human animals can also include microinjection of DNA into zygotes. In most cases, injected DNA will be incorporated into the host genome before development begins to occur. Consequently, the resulting animal will carry the incorporated transgene within the genome of all somatic cells of the animal (Brinster, et al. (1985) Proc. Natl. Acad. Sci. 82: 4438-4442).

In a preferred embodiment, inactivation of the endogenous loci is achieved by targeted disruption of the appropriate loci through homologous recombination in non-human embryonic stem cells. Incorporation of the modified non-human embryonic stem cells containing a genetic disruption into the genome of the resulting organism results in generation of non-human animals that are capable of transmitting the genetic modifications through the germ line, thereby generating transgenic animals having inactivated genetic loci.

To inactivate the host TCR loci by homologous recombination, DNA is introduced into a cell by transformation and recombines at the endogenous loci to inhibit the production of endogenous TCR subunits. The term "transformation" is intended to mean any technique for introducing DNA into a viable cell, such as conjugation, transformation, transfection, transduction, electroporation, microinjection, lipofection, etc. Generally, homologous recombination may be employed to functionally inactivate each of the loci, by introduction of the homologous DNA into non-human embryos or non-human embryonic stem cells. Production of non-human animals having inactivated loci then results from introduction of the modified cells into recipient blastocysts. Subsequent breeding allows for germ line transmission of the inactivated locus. One can next breed resulting heterozygous offspring then select for homozygous progeny from the heterozygous parents. Alternatively, one may use the transformed non-human embryonic stem cell for additional rounds of homologous recombination to generate inactivation of additionally targeted loci, if desired.

Transgenes of the invention include DNA sequences that are capable of disruption of endogenous-alleles and may be referred to herein as "knockout", disruption, or inactivation constructs or transgenes. Further, such transgenes are capable of either physical or functional disruption of endogenous alleles such that incorporation of the disruption transgenes results in lack of expression of the endogenous alleles. Such transgenes comprise DNA sequences homologous to the targeted loci and also incorporate a disruption allele encoding either a disrupted α chain TCR or β chain TCR in a transgenic non-human animal.

For inactivation, any lesion in the target locus resulting in the prevention of expression of a TCR subunit of that locus may be employed. Thus, the lesion may be in a region comprising the enhancer, *e*.*g*., 5' upstream or intron, in the V, J or C regions of the TCR loci, and with the β chain, the opportunity exists in the D region, or combinations thereof. Thus, the important factor is that TCR germ line gene rearrangement is inhibited, or a functional message encoding the TCR subunit cannot be produced, either due to failure of transcription, failure of processing of the message, or the like.

Preferably, in the case of T-cells, the Cβ1 and Cβ2 alleles for the TCRβ chain and most preferably the Cα allele for the TCRα chain are targeted for insertion of a transgene that disrupts expression of the allele. For example, in the case of the Cα allele, once a genotype is identified containing a transgene disrupting Cα expression, cross-breeding can be used to produce transgenic non-human animals homozygous for the Cα-negative genotype.

Structurally, the knockout transgene, in one aspect of the invention, encodes a TCR polypeptide variant comprising a TCR wherein all or part of the constant region is deleted. Preferably, at least part of the C region is deleted. However, the deleted sequences may also include part of the V, D and/or J segment of the TCR polypeptide. Thus, one produces a construct that lacks a functional C region and may lack the sequences adjacent to, upstream and/or downstream from the C region or comprises all or part of the region with an inactivating insertion in the C region. The deletion may be 50 bp or more, where such deletion results in disruption of formation of a functional mRNA. Desirably, the C region in whole or substantial part, usually at least about 75% of the locus, preferably at least about 90% of the locus, is deleted.

For ease of indication of incorporation of the transgene, a marker gene is used to replace the C region. Various markers may be employed, particularly those which allow for positive selection. Of particular interest is the use of G418 resistance, resulting from expression of the gene for neomycin phosphotransferase.

Upstream and/ or downstream from the target gene construct may be a gene which provides for identification of the occurrence of a double crossover event. For this purpose, the *Herpes simplex* virus thymidine kinase (HSV-tk) gene may be employed, since cells expressing the thymidine kinase gene may be killed by the use of nucleoside analogs such as acyclovir or gancyclovir, by their cytotoxic effects on cells that contain a functional HSV-tk gene. The absence of sensitivity to these nucleoside analogs indicates the absence of the HSV-tk gene and, therefore, where homologous recombination has occurred, that a double crossover has also occurred.

After transformation or transfection of the target cells, target cells may be selected by means of positive and/or negative markers, as previously indicated, G418 resistance and acyclovir or gancyclovir resistance. While the presence of the G418 marker gene in the genome will indicate that integration has occurred, it will still be necessary to determine whether homologous integration has occurred. Those cells which show the desired phenotype may then be further analyzed which can be achieved in a number of ways, including restriction analysis, electrophoresis, Southern analysis, polymerase chain reaction (PCR), or the like. By identifying fragments which show the presence of the genetic alteration(s) at the target locus, one can identify cells in which homologous recombination has occurred to inactivate a copy of the target locus. For the most part, DNA analysis will be employed to establish the location of the integration.

Preferably, the PCR may be used with advantage in detecting the presence of homologous recombination. Probes may be used which are complementary to a sequence within the construct and complementary to a sequence outside the construct and at the target locus. In this way, one can only obtain DNA chains having both the primers present in the complementary chains if homologous recombination has occurred. By demonstrating the presence of the probes for the expected size sequence, the occurrence of homologous recombination is supported.

Generally, a DNA oligonucleotide primer for use in the PCR methods will be between approximately 12 to 50 nucleotides in length, preferably approximately 20-25 nucleotides in length. The PCR oligonucleotide primers may suitably include restriction sites to add specific restriction enzyme cleavage sites to the PCR product as needed, *e.g.*, to introduce a ligation site. Exemplary primers are provided in the Examples and Drawings that follow. The PCR products produced will include amplified TCR α and β chain sequences and can be modified to include, as desired, ribosome binding, intron, leader and promoter sequences for optimal analysis of the targeted locus.

In constructing the subject constructs for homologous recombination, a DNA vector for prokaryotes, particularly *E*. *coli,* may be included, for preparing the construct, cloning after each manipulation, analysis, such as restriction mapping or sequencing, expansion and isolation of the desired sequences. The term "vector" as used herein means any nucleic acid sequence of interest capable of being incorporated into a host cell resulting in the expression of a nucleic acid segment of interest such as those segments or sequences described above.

Vectors may include *e.g.*, linear nucleic acid segments or sequences, plasmids, cosmids, phagemids and extra-chromosomal DNA. Specifically, the vector can be recombinant DNA. Where the construct is large, generally exceeding about 50 kbp, usually exceeding 100 kbp, and usually not more than about 1000 kbp, a yeast artificial chromosome (YAC) may be used for cloning of the construct.

As mentioned previously, the process of inactivation of endogenous loci may be performed first with the α chain locus in non-human embryonic stem cells that can then be used to reconstitute blastocysts and generate chimeric non-human animals. Continuous cross-breeding of these animals can result in the production of homozygous animals that can be used as a source of non-human embryonic stem cells. These non-human embryonic stem cells may be isolated and transformed to inactivate the β locus, and the process repeated until all the desired loci have been inactivated. Alternatively, the β chain locus may be the first.

In addition to the above described methods of inactivation of endogenous loci, additional preferred methods of inactivation are available and may include for example, use of the *tet* transcription system to utilize temporal control of specific genes of interest (Proc. Natl. Acad. Sci. (1994) 91:9302-9306) or introduction of deoxycycline transcriptional regulatory controls for tissue specific control (Proc. Natl. Acad. Sci. (1996) 93:10933-10938).

An additionally preferred method for functional inactivation includes employment of the *cre-lox* deletion, site specific recombination system for targeted knock-out of genetic loci, wherein *loxP* sites are inserted to flank genes of interest and *cre* recombinase activated to delete genes (Curr. Opin. Biotechnol. (1994) 5:521-527).

Alternatively, antisense methods may be utilized in order to inhibit transcription of the desired loci, thus resulting in functional disruption of endogenous loci. In such a situation, antisense oligonucleotides will be generated which target specific sequences of the designated locus of interest, such as the TCRα or TCRβ locus, wherein successful antisense targeting results in inhibited production of the functional protein.

Endogenous loci inactivation could also be created by crossing two commercially available homozygous mice strains (The Jackson Laboratory, Maine). The first strain, B6.129P2-*Tcr^{btm1Mom}*, contains a deletion of the D and C gene segments of the TCRβ locus, while the second strain, B6.129S2-*Tcrα^{tm1Mom}*, contains a deletion of the TCRα C gene segment [Momberts, et al. (1991) PNAS 88: 3084-3087; Momberts, et al. (1992) Nature 360: 225-231]. Both animal strains fail to produce functional α/β TCRs, and when crossed together should yield an animal that has both endogenous TCR loci inactivated.

Additional preferred transgenes of the invention include DNA sequences that comprise heterologous molecules. Preferred heterologous transgenes of the invention include heterologous TCR subunits. Further, incorporation of such transgenes into the genome of the host is capable of conferring to the host the ability to express a repertoire of heterologous TCRs. Used herein the term "expression," or "gene expression", is meant to refer to the production of the protein product of the nucleic acid sequence of interest including transcription of the DNA and translation of the RNA transcription.

The genes encoding the various segments and regions that may be used in the invention have been well characterized. TCRs represent an enormous percent of clonally varying molecules with the same basic structure. The TCR is a heterodimer of 90 kd consisting of two transmembrane polypeptides of 45 kd each connected by disulfide bridges (Samuelson, et al. (1983) Proc. Natl. Acad. Sci. 80: 6972; Acuto, et al. (1983) Cell 34: 717; MacIntyre, et al. (1983) Cell 34: 737). For most T-cells, the two polypeptides are referred to as the α and β chain. Using subtractive hybridization procedures, cDNA clones encoding the TCR polypeptide chains have been isolated (Hendrick, et al. (1984) Nature 308: 149; Hendrick, et al. (1984) Nature 308: 153; Yanagi, et al. (1984) Nature 308: 145; Saito, et al. (1987) Nature 325: 125; Chien, et al. (1984) Nature 312: 314). Sequence analysis of these cDNA clones is employed to reveal the complete primary sequence of the TCR polypeptides. The TCR polypeptides are similar to each other and resemble the structure of the immunoglobulin polypeptides. (For review see Davis and Bjorkman (1988) *supra*.; and Kronenberg, et al. (1986) Ann. Rev. Immunol. 4:529).

Like the heavy and light chains of the immunoglobulins, the α and β chains have V and C regions (Acuto, *et al.* (1983) *supra*; Kappler, et al. (1983) Cell 35: 295). The V region is responsible for antigen recognition and the C region is involved in membrane anchoring and signal transmission. The V region of the TCR chains is further subdivided into V and J segments. In addition, the variable region of the β chains also contains a D segment interposed between the V and J segments. The constant region of the TCR chains is composed of four functional regions often encoded by different exons (Davis and Bjorkoran (1988) *supra.*)*.*

The availability of TCR cDNAs permits an analysis of the genomic organization of the murine and human TCR genes. The TCR genes show a segmental organization similar to the immunoglobulin genes. In the β chain gene locus, two nearly identical Cβ regions are tandemly arranged, each preceded by one D and six J segments (Rowen, et al. (1996), Science 272:1755). The β locus also contains approximately 65V gene segments, 46 of which appear functional, one of which is located 3' to the C regions in opposite orientation (Rowen, et al. (1996) Science 272:1755). During somatic development of the T-cell, a functional TCR gene is formed by rearrangement of these segments and regions. This process, depicted in FIG. 6, is the basis for T-cell receptor diversity.

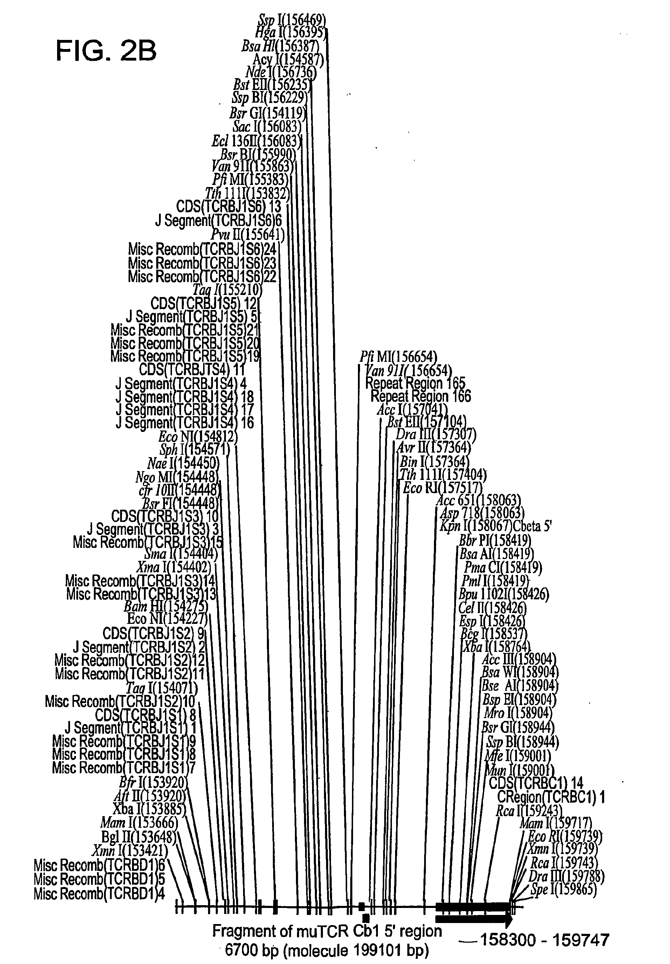

As shown schematically in FIGS. 2 and 4, the encoding segments for the TCR genes are scattered over large arrays of chromosomal DNA. Specific V, D and J segments are fused together to generate a complete V coding region next to a C region. B and T-cells probably use the same machinery for the assembly of Ig and TCR since B cells rearrange transfected TCR segments in the same way as transfected Ig gene segments, and the rearrangements are mediated by similar sequences flanking the segments to be fused (Akira (1987) Science 238:1134; Yancopoulos, *et al.* (1986) *supra*). The TCR β genes are rearranged and transcribed first, followed by the TCR α gene (Chien, *et al.* (1987) supra; Pardoll, et al. (1987) Nature 326: 79; Raulet, et al. (1985) Nature 312: 36; Samelson, et al. (1985) Nature 315: 765; Snodgrass, et al. (1985) Nature 315: 232).

In order to provide for the production of human TCRs in a heterologous host, it is necessary that the host be competent to provide the necessary enzymes and other factors involved with the production of TCRs, while lacking competent endogenous genes for the expression of alpha and beta chain TCRs. Thus, those enzymes and other factors associated with germ line rearrangement, splicing, and the like, must be functional in the heterologous host. What will be lacking is a functional natural region comprising the various exons associated with the production of endogenous TCR chains, as described above.

Thus, germline sequence TCR loci, or functionally unrearranged β and α genes from human TCR loci are preferred for making transgenes for use in the present invention. Such heterologous sequences include regulatory sequences as well as structural DNA sequences which, when processed, encode heterologous TCR polypeptide variants capable of representing the TCR repertoire. The only limitation on the use of such heterologous sequences is functional. The heterologous regulatory sequences must be utilized by the transgenic non-human animal to efficiently express sufficient amounts of the TCR polypeptides, such that it is able to produce a repertoire of TCRs. Further, the heterologous TCRs when properly expressed in the transgenic animal must be capable of producing the desired immune response. Still further, it should be possible to mix homologous and heterologous DNA sequences (*e.g.*, homologous regulators with heterologous structural genes and *vice versa*) to produce functional transgenes that may be used to practice the invention.

Strategies of the present invention are based on the known organization of the TCR α and β chain loci. Transgenes are derived, for example, from DNA sequences encoding at least one polypeptide chain of a TCR. Preferably, germline sequences of the α or β chain locus of the TCR are used as transgenes. As indicated, the TCR α and β chain loci have been well characterized. Transgenes of the present invention are derived from such DNA sequences.

Such DNA may be obtained from the genome of somatic cells and cloned using well-established technology. Such cloned DNA sequences may thereafter be further manipulated by recombinant techniques to construct the transgenes of the present invention.

Such heterologous transgenes preferably comprise operably linked germline DNA sequences of the loci that may be expressed in a transgenic non-human animal. Alternatively, operably linked partially rearranged sequences or fully rearranged sequences of the TCR α or β chains may be used for transgene preparation.

By the term "operably linked" is meant a genetic sequence operationally (*i.e*., functionally) linked to a nucleic acid segment, or sequences upstream (5') or downstream (3') from a given segment or sequence. Those nearby sequences often impact processing and/or expression of the nucleic acid segment or sequence in a desired cell type.

Typically, a DNA segment encoding a heterologous protein of the invention is inserted into a vector, preferably a DNA vector, in order to replicate the DNA segment in a suitable host cell.

In order to isolate, clone and transfer the TCR α or β chain locus, a yeast artificial chromosome may be employed. The entire locus can be cloned and contained within one or a few YAC clones. If multiple YAC clones are employed and contain regions of overlapping homology, they can be recombined within yeast host strains to produce a single construct representing the entire locus. YAC arms can be additionally modified with mammalian selection cassettes by retrofitting to assist in the introduction of the constructs into non-human embryonic stem cells or non-human embryos by the previously outlined methods.

In order to obtain a broad spectrum of TCRs produced, it is preferable to include all or almost all of the germline sequence of the TCR loci. However, in some instances, it may be preferable that one includes a subset of the entire V region. Various V region gene families are interspersed within the V region cluster. Thus, by obtaining a subset of the known V region genes of the human α and β chain TCR loci (Berman et al., EMBO J. (1988) 7:727-738) rather than the entire complement of V regions, the transgenic host may be immunized and be capable of mounting a strong immune response and provide diverse TCRs.

As discussed above, prepared human transgenes of the invention may be introduced into the pronuclei of fertilized non-human oocytes or embryonic stem cells. Genomic integration may be random or homologous depending on the particular strategy to be employed. Thus, by using transformation, using repetitive steps or in combination with breeding, transgenic non-human animals may be obtained which are able to produce human TCRs in the substantial absence of host TCR subunits.

Once the human loci have been introduced into the host genome, either by homologous recombination or random integration, and host non-human animals have been produced with the endogenous TCR loci inactivated by appropriate breeding of the various transgenic or mutated animals, one can produce a host which lacks the native capability to produce endogenous TCR subunits, but has the capacity to produce human TCR with a substantial TCR repertoire.

Such a host strain, upon immunization with specific antigens, would respond by the production of mouse T-cells producing specific human TCRs. It will then be possible to isolate particular T-cells that produce TCRs with particular preferred specificity. Such T-cells could be fused with mouse myeloma cells or be immortalized in any other manner for the continuous stable production of specific human TCRs.

Antigen specific human TCRs produced by an immortal cell line as described may be isolated and used for development for therapeutic use.

Additionally, isolation of nucleic acids encoding antigen specific TCR subunits may be isolated from these produced immortal cell lines. Isolated nucleic acids may be used in the production and development of TCR-based therapeutics.

Isolated nucleic acids may also be useful in the preparation and production of soluble single chain TCRs, which have been described in pending patent applications U.S.S.N 09/422,375, U.S.S.N. 08/943,086, and U.S.S.N. 08/813,781, which are incorporated herein by reference.

The subject methodology and strategies of the present invention need not be limited to producing transgenic non-human animals producing heterologous TCRs, but also provides the opportunity to provide for production of additional heterologous immune system components. For example, TCRs are known to function in the context of and by interaction with additional molecules such a major histocompatibility complex proteins (MHC), as well as co-receptor molecules CD4/CD8.

MHC loci have been well characterized. Transgenes encoding for MHC molecules can be prepared similarly to methods described for the TCR loci. MHC transgenes can then be additionally incorporated into cells and transgenic non-human animals produced which co-express heterologous TCRs in conjunction with MHC molecules. Preferable heterologous MHC transgenes comprise rearranged, operably linked DNA sequences, wherein incorporation into the transgenic host confers non-human animals capable of expressing heterologous MHCI and/or MHCII molecules.

Additionally, human co-receptor molecules CD4 and CD8 have been previously created which are functional in mice and have the ability to interact witch human MHC molecules expressed in mice (Fugger, et al. (1994) PNAS 91:6151-6155; Medsen, et al. (1999) Nature Genetics 23: 343-347; Kieffer, et al. (1997) J. Immunol. 159:4907-4912). Chimeric murine-human CD4 or CD8 co-receptors, where the extracellular domain of the co-receptor is human and the transmembrane and intracellular domains are murine, could also be used when special aspects of signaling in the murine cells necessitate use of such chimeric co-receptors; but for most uses, the human co-receptors function well.

Thus, further embodiments of the invention include incorporation of transgenes comprising co-receptor molecules CD4 and CD8 in transgenic non-human animals produced and described above. Such molecules are functional for interaction with human TCRs in the mouse host Co-receptors may be expressed in TCR-transgenic hosts either in conjunction with or without heterologous MHC molecules.

The following non-limiting examples are illustrative of the present invention.

### Methods and Materials

Transgenic mice, embryos, and embryonic stem cells are derived and manipulated according to Hogan, et al., "Manipulating the Mouse Embryo: A Laboratory Manual", Cold Spring Harbor Laboratory, Teratocarcinomas and embryonic stem cells: a practical approach, E. J. Robertson, ed., IRL Press, Washington, D.C., 1987; Zjilstra, et al. (1989) Nature 342:435-438; and Schwartzberg et al. (1989) Science 246:799-803,
().

DNA cloning procedures and YAC manipulations are carried out according to J. Sambrook, et al. in Molecular Cloning: A Laboratory Manual, 2d ed. (1989), Cold Spring Harbor Laboratory Press, Cold Spring Harbor, N.Y., and Genome Analysis: A Laboratory Manual, Volume 3 (1999), Cold Spring Harbor Laboratory Press, Cold Spring Harbor, N.Y.

Additional resources such as transgenic or wild-type mouse strains, human YAC resource libraries and oligonucleotides are purchased from outside vendors. For example resources may be obtained from the Jackson Laboratory (Bar Harbor, Maine), ResGen (Huntsville, Alabama), HGMP Resource Centre (Cambride, United Kingdom), and Sigma Genosys (The Woodlands, Texas).

Hybridoma cells and antibodies are manipulated according to "Antibodies: A Laboratory Manual", Ed Harlow and David Lane, Cold Spring Harbor Laboratory (1988).

### Example 1

### Inactivation of the Mouse TCRα Chain Gene by Homologous Recombination

This example describes the inactivation of the mouse endogenous TCRα locus by homologous recombination in embryonic stem (ES) cells followed by introduction of the mutated gene into the mouse germ line by injection of targeted ES cells bearing an inactivated α allele into early mouse embryos (blastocysts).

The strategy is to delete the α chain constant region (Cα) by homologous recombination with a vector containing DNA sequences homologous to the mouse α locus in which a 3.7 kb segment of the locus, spanning the Cα segments, is deleted and replaced by the puromycin selectable marker *pu*r.

### Construction of the α targeting vector:

The plasmid pPur (Clonetech; Palo Alto, CA) contains the puromycin resistance gene (*pur*), used for drug selection of transfected ES cells, under the transcriptional control of the SV40 promoter. The plasmid also includes an SV40 polyadenylation site for the *pur* gene. This plasmid is used as the starting point for construction of the α-targeting vector. The first step is to insert sequences encoding the thymidine kinase gene.

The Herpes Simple Virus thymidine kinase (HSV-tk) gene is included in the construct in order to allow for enrichment of ES clones bearing homologous recombinants, as described by Mansour, et al (1988), Nature 336:348-352.

The HSV-tk cassette is obtained from the plasmid pHSV-106 (GibcoBRL), which contains the structural sequences for the HSV-tk gene bracketed by the tk promoter and polyadenylation sequences. The tk cassette is amplified from pHSV-106 by PCR using primers that cover the *Bam*HI site (TKf, see below) and a site located near the polyadenylation site and which encodes a *Not*I site (TKr, see below). The resulting fragment is ligated into pGEM T-Easy, sequenced and excised with *Bam*HI and *Not*I. The pPUR vector is modified to include a unique *Not*I site by cutting with EcoRI and ligating in the oligonucleotide, AATTGCGGCCGC. The resulting plasmid, pPURtk contains unique *Nde*I, *Not*I and *Bam*HI sites (FIG. 1a).

| | |
|---|---|
| TKf. | ACTG GGATCCAAAT GAGTCTTCGG |
| TKr: | ACTG GCGGCCGC CAAACGACCC AACACCCGTG |

Mouse α chain sequences (FIG. 1b) are isolated from a genomic phage library derived from liver DNA using oligonucleotide probes specific for the Cα locus:
5'-CC CACCTGGATC TCCCAGATTT GTGAGGAAGG TTGCTGGAGA GC-3' (MUSTCRA 89394-39437, Cα exon 4)
and for the region 5' to Cα exon 1:
5'-GGAAA GCCCTGCTGG CTCCAAGATGGCTGAGGGAA AGGTCTACGG-3' (MUSTCRA 81681-81725, 5' to Cα exon 1)

A 4.1 kb *Bam*HI fragment extending 3' of the mouse Casegment is isolated from a positive phage clone by PCR amplification with oligonucleotide primers PCa3'f and PCa3'r (sequence provided below), and subcloned into *Bam*HI digested pPURtk to generate the plasmid pPURtk-Ca3' (FIG. 1c).
PCa3'f: 5'-TAGT*GGATCC* CATGCAGAGAGAAACCGAAGTACGTG-3'
PCa3'r : 5'-GCTACAGAGTGAAGTCATGGATCCT'G-3'

A 4.8 kb *NdeI* fragment extending 5' of the Cα region is also isolated from a positive phage clone by PCR amplification with oligonucleotide primers PCa5'f and PCa5'r. The resulting fragment is digested with *Nde*I and ligated into *Nde*I digested pPURtk-Ca3', in the same 5' to 3' orientation as the *pur* gene and the downstream 3' Cα sequences, to generate pPURtk-Ca5'3' (FIG. 1d).
PCa5'f: 5'-GGTCT GTGTTCCATA TGACGTCAGT ACG-3'
PCa5'r: 5'-ATTA*CATATG* GGTCCTAACTTAGGTCAGAACTCAGATGC-3'

This results in a plasmid with the flanking regions of the Cα but, when integrated, results in a deletion of Cα thus making the locus inactive.

Generation and analysis of non-human ES cells with targeted inactivation of a Cα allele: The non-human ES cells used are the AB-1 line grown on mitotically inactive SNL76/7 cell feeder layers (McMahon and Bradley (1990), Cell 62:1073-1085) essentially as described (Robertson, E. J. (1987) in Teratocarcinomas and Embryonic Stem Cells: A Practical Approach. E. J. Robertson, ed. (Oxford: IRL Press), p. 71-112).

Other suitable non-human ES lines include, but are not limited to, the E14 line (Hooper, et al. (1987) Nature 326:292-295), the D3 line (Doetschman, et al. (1985) J. Embryol. Exp. Morph. 87:27-45), and the CCE line (Robertson, et al. (1986) Nature 323:445-448). The success of generating a mouse line from ES cells bearing a specific targeted mutation depends on the pluripotency of the ES cells (*i.e.*, their ability, once injected into a host blastocyst, to participate in embryogenesis and contribute to the germ cells of the resulting animal).

The pluripotency of any given ES cell line can vary with time in culture and the care with which it has been handled. The only definitive assay for pluripotency is to determine whether the specific population of non-human ES cells to be used for targeting can give rise to chimeras capable of germline transmission of the ES genome. For this reason, prior to gene targeting, a portion of the parental population of AB-1 cells is injected into C57B1/6J blastocysts to ascertain whether the cells are capable of generating chimeric mice with extensive ES cell contribution and whether the majority of these chimeras can transmit the ES genome to progeny.

The α chain inactivation vector pPURtk-Ca5'3' is digested with *Not*I and electroporated into AB-1 cells by the methods described (Hasty, et al. (1991), Nature, 350:243-246). Electroporated cells are plated onto 100mm dishes at a density of 1-2 x 10⁶ cells/dish. After 24 hours, G418 (200 µg/ml of active component - to select for neomycin resistant cells) and fialuridine (1-(2-deoxy-2-fluoro-(beta)-d-arabinofuranosyl)-5-iodouracil, or FIAU) (0.5 mM - to select for HSV-tk positive cells) are added to the medium, and drug-resistant clones are allowed to.develop over 10-11 days. Clones are picked, trypsinized, divided into two portions, and further expanded. Half of the cells derived from each clone are then frozen and the other half analyzed for homologous recombination between vector and target sequences.

DNA analysis is carried out by Southern blot hybridization. DNA is isolated from the clones as described (Laird, et al. (1991), Nucl. Acids Res. 19:4293) digested with *Bam*HI and probed with the 730 bp *Hind*III fragment indicated in FIG. 1d as probe A. This probe detects a 8.9 kb *Bam*HI fragment in the wild type locus, and a diagnostic 2.4 kb band in a locus which has homologously recombined with the targeting vector (see FIG. 1d and 1e). Positive puromycin and FIAU resistant clones screened by Southern blot analysis which displayed the 2.4 kb *Bam*HI band indicative of a homologous recombination into one of the Cα genes digested with the restriction enzymes *Afl*II to verify that the vector integrated homologously into one of the Cα genes. The probe detects a 12.3 kb fragment in the wild-type locus and a 9.9 kb fragment in the locus that has homologously recombined.

Generation of mice bearing the inactivated TCRα chain:
Five of the targeted non-human ES clones described in the previous section are thawed and injected into C57B1/6J blastocysts as described (Bradley, A. (1987) in Teratocarcinomas and Embryonic Stem Cells: A Practical Approach. E. J. Robertson, ed. (Oxford: IRL Press), p. 113-151) and transferred into the uteri of pseudopregnant females to generate chimeric mice resulting from a mixture of cells derived from the input ES cells and the host blastocyst. The extent of ES cell contribution to the chimeras can be visually estimated by the amount of agouti coat coloration, derived from the ES cell line, on the black C57B1/6J background. Approximately half of the offspring resulting from blastocyst injection of the targeted clones are expected to be chimeric (*i.e.*, showed agouti as well as black pigmentation) and of these, the majority should show extensive (70 percent or greater) ES cell contribution to coat pigmentation. The AB1 ES cells are an XY cell line and a majority of these high percentage chimeras are male due to sex conversion of female embryos colonized by male ES cells. Male chimeras derived from 4 of the 5 targeted clones are bred with C57BL/6J females and the offspring monitored for the presence of the dominant agouti coat color indicative of germline transmission of the ES genome. Chimeras from some of these clones should consistently generate agouti offspring. Since only one copy of the Cα locus is targeted in the injected ES clones, each agouti pup had a 50 percent chance of inheriting the mutated locus. Screening for the targeted gene is carried out by Southern blot analysis of *Bam*HI-digested DNA from tail tip biopsies, using the probe utilized in identifying targeted ES clones (probe A, FIG. 1d).

Approximately 50 percent of the agouti offspring should show a hybridizing *Bam*HI band of 2.4 kb in addition to the wild-type band of 8.9 kb, demonstrating the germline transmission of the targeted Cα locus. In order to generate mice homozygous for the mutation, heterozygotes are bred together and the Cα genotype of the offspring determined, as described above.

Three genotypes can be derived from the heterozygote matings: (*i*) wild-type mice bearing two copies of a normal Cα locus, (*ii*) heterozygotes carrying one targeted copy of the Cα gene and one normal murine Cα gene, and (*iii*) mice homozygous for the Cα mutation. The deletion of Cα sequences from these latter mice is verified by hybridization of the Southern blots with a probe specific for Cα exon 2. Whereas hybridization of the Cα exon 2 probe is observed to DNA samples from heterozygous and wild-type siblings, no hybridizing signal is present in the homozygotes, attesting to the generation of a novel mouse strain in which both copies of the Cα locus have been inactivated by deletion as a result of targeted mutation.
Cα exon2 probe: 5'-CG TTCCCTGTGA TGCCACGTTG ACTGAGAAAA GCTTTG-3'

### Example 2

### Inactivation of the Mouse TCRβ Gene by Homologous Recombination:

This example describes the inactivation of the endogenous murine TCRβ chain locus by homologous recombination in non-human ES cells. The strategy is to delete the endogenous β chain constant region (Cβ) segments by homologous recombination with a vector containing Cβ chain sequences from which the Cβ regions have been deleted and replaced by the gene for the neomycin selectable marker neo.

### Construction of a Cβ chain targeting vector

The plasmids pGT-N28 and pGT-N39 (New England Biolabs) contain the neomycin resistance gene (*neo*), used for drug selection of transfected EPS cells, under the transcriptional control of the phosphoglycerate kinase (*pgk*) gene promoter. The neo gene is followed by the *pgk* polyadenylation site. In order to construct the cloning vector for the Cβ chain constructs, pNeo, pGT-N28 and pGT-N39 are cut with *Spe*I and *Afl*II and the 2.8 kb fragment from pGT-N28 is isolated and purified and ligated to the 1.6 kb fragment isolated and purified from the digest of pGT-N39. The resultant plasmid, pNeo, contains the *neo* gene flanked by the unique restriction sites *Not*I, *Eco*RI and *Hind*III.

Mouse Cβ chain sequences containing regions 5'to Cβ1 and 3'to Cβ 2 (FIG. 2a) are isolated from a murine genomic phage library derived from liver DNA using the following oligonucleotide probes specific for the Cβ chain constant region.
Cβ1: 5'- TGAGAAAGTC CAAAAACTCG GGGTACCATT CCACCATAGA-3' (AE000665 158041-158080)
Cβ2: 5'-GGAGT TAACCTGGTT GTGTCTCAGC AGTTTCTTTG GACTCCTGTG-3' (AE000665 168427-168471)

A 3.0 kb genomic *Bam*HI/*Eco*RI fragment, located 5' to the Cβ1 region is isolated from a phage which is identified using probe Cβ1. The fragment cloned into the Cβ knockout vector is generated in the following manner; the phage-DNA is first digested with *Bam*HI and a *Bam*HI/NotI linker (see B/N # 1 and #2 below) is annealed and ligated prior to digestion with *Eco*RI. This fragment is then cloned into pNeo which had been digested with *Not*I and *Eco*RI resulting in a plasmid designated pNeo Cb5'.
B/N #1 (top): 5'-GAT CCG TTA ACG C-3'
B/N #2 (bottom): 3'-GC AAT TGC GCC GG-5'

The next step in the construction involves the excision from pPURtk (see example 1) of the HSV thymidine kinase cassette as a *Bam*HI/*Not*I fragment and ligating it into pNeo Cb5' cut with BamHI and NotI. The resulting plasmid carries the HSV-tk gene, 3 kb of sequence 5' to the Cβ1 region and the neo selectable marker and is designated pNEOtk Cb5'.

The final step in the process of building the Cβ knockout vector is accomplished by isolating a 3.4 kb HindIII fragment from a phage positive for hybridization with probe Cβ2. This fragment is cloned into pNEOtk-C5' cut with *Hind*III. The resulting construct, pNEOtk-Cb5'3' (FIG. 2a and 2d), contains 6.4 kb of genomic sequences flanking the Cβ 1 and 2 loci, with a 11.3 kb deletion spanning the Cβ1 and Cβ2 regions into which the neo gene has been inserted.

Generation and analysis of ES cells with targeted inactivation of a Cβ allele:
AB-1 ES cells (McMahon and Bradley (1990), Cell 62:1073-1085) are grown on mitotically inactive SNL76/7 cell feeder layers essentially as described (Robertson, E. J. (1987) Teratocarcinomas and Embryonic Stem Cells: A Practical Approach. E. J. Robertson, ed. (Oxford: IRL Press), pp. 71-112). As described in the previous example, prior to electroporation of ES cells with the targeting construct pNEOtk-Cab5'3', the pluripotency of the ES cells is determined by generation of AB-1 derived chimeras which are shown to be capable of germline transmission of the ES genome.

The Cβ chain inactivation vector pNEOtk-Cb5'3' is digested with *Not*I and electroporated into AB-1 cells by the methods described (Hasty et al. (1991) Nature 350:243-246). Electroporated cells are plated into 100mm dishes at a density of 1-2x10⁶, cells/dish. After 24 hours, G418 (200 µg/ml of active component) and FIAU (0.5 mM) are added to the medium, and drug-resistant clones are allowed to develop over 8-10 days. Clones are picked, trypsinized, divided into two portions, and further expanded. Half of the cells derived from each clone are then frozen and the other half analyzed for homologous recombination between vector and target sequences.

DNA analysis is carried out by Southern blot hybridization. DNA is isolated from the clones as described (Laird, et al. (1991) Nucleic Acids Res. 19: 4293), digested with *Bam*HI and probed with the 800 bp. PCR fragment generated from pNEOtk-Cb5'3' with primers PRIMb5'f and PRIMb5'r as probes A and B in FIG. 2a. This probe detects a *Bam*HI fragment of 10.4 kb in the wild-type locus, whereas a 7.4 kb band is diagnostic of homologous recombination of endogenous sequences with the targeting vector. The G418 and FIAU doubly-resistant clones screened by Southern blot hybridization and found to contain the 7.4 kb fragment diagnostic of the expected targeted events at the Cβ locus is confirmed by further digestion with *Hind*HIII, *Eco*RV and *Tth*111I. Hybridisation of probes A and B to Southern blots of *Hind*III, *Eco*RV and *Tth*III digested DNA produces bands of 8.7 kb, 3.6 kb, and 3.4 + 3.9 kb, respectively, for the wild-type locus, whereas bands of 8.3 kb, 2.8 kb, and 0.9 + 3.4 kb, respectively, are expected for the targeted heavy chain locus.
PRIMb5'f: 5'-GGATTCA AAGGTTACCT TATGTGGCCA C-3'
PRIMb5'r: 5'-GCCCC AAAGGCCTAC CCGCTTCC -3'

### Generation of mice carrying the Cβ deletion

Three of the targeted non-human ES clones described in the previous section are thawed and injected into C57BL/6J blastocysts as described (Bradley, A. (1987) in Teratocarcinomas and Embryonic Stem Cells: A Practical Approach, E. J. Robertson, ed., Oxford: IRL Press, p.113-151) and transferred into the uteri of pseudopregnant females. The extent of ES cell contribution to the chimera is visually estimated from the amount of agouti coat coloration, derived from the ES cell line, on the black C57BL/6J background. Half of the offspring resulting from blastocyst injection of two of the targeted clones should be chimeric (*i.e.*, show agouti as well as black pigmentation. The majority of the chimeras should show significant (approximately 50 percent or greater) ES cell contribution to coat pigmentation. Since the AB-1 ES cells are an XY cell line, most of the chimeras are male, due to sex conversion of female embryos colonized by male ES cells. Male chimeras are bred with C57BL/6J females and the offspring monitored for the presence of the dominant agouti coat color indicative of germline transmission of the ES genome. Chimeras from both of the clones should consistent generate agouti offspring. Since only one copy of the heavy chain locus is targeted in the injected ES clones, each agouti pup had a 50 percent chance of inheriting the mutated locus. Screening for the targeted gene is carried out by Southern blot analysis of BamHI-digested DNA from tail biopsies, using the probe utilized in identifying targeted ES clones (probe A, FIG. 2a). Approximately 50 percent of the agouti offspring should show a hybridizing BamHI band of approximately 7.4 kb in addition to the wild-type band of 10.4 kb, demonstrating germline transmission of the targeted Cβgene (CβKO) segment.

In order to generate mice homozygous for the CβKO, heterozygotes are bred together and the β chain genotype of the offspring determined as described above. Three genotypes are derived from the heterozygote matings: wild-type mice bearing two copies of the normal Cβ locus, heterozygotes caring one targeted copy of the gene and one normal copy, and mice homozygous for the CβKO mutation. The absence of Cβ sequences from these latter mice is verified by Southern blot analysis of a BamHI digest of positive clones using Cβ1 or Cβ2 as probes. These probes should generate no signal from mice with the CβKO locus while those with the wild-type locus generated fragments of 10.4 and 6.2 kb respectively attesting to the generation of a novel mouse strain in which both copies of the heavy chain gene have been mutated by deletion of the Cβ sequences.

### Example 3

### Vector construction and modification for cloning human TCR loci

### pYAC4-neo vector:

Yeast is an excellent host in which to clone large fragments of exogenous DNA as yeast artificial chromosomes (YACs). Linear DNA molecules of up to 1.2 megabase pairs (Mbp) in length have been constructed *in vitro*, transformed into host yeast cells, and propagated as faithful replicas of the source genomic DNA (Burke, D.T., Carle, G.F. and Olson, M.V. (1987) Science 236: 806; Bruggemann, M, and Neuberger, M.S. (1996) Immunol. Today 7:391). One of the most widely used YAC vector has been pYAC4 (Burke, D.T., *et al.*; FIG. 3a). This vector can be transformed into either *Escherichia coli* or yeast and will replicate as a circular molecule in either host. However, pYAC4 preferentially is used to clone large inserts as linear yeast chromosomes. The first generation pYAC4 vector has been modified to contain the neomycin gene to facilitate selection of pYAC transfectants that are G418 resistant (Cooke, H. and Cross, S. (1988) Nucleic Acids Res. 16: 11317). We will further modify the pYAC4-neo vector by adding a polylinker region containing the restriction sites *Eco*RI, *Fse*I, *Ksp*I, *Asc*I, and *Eco*RI. The polylinker will be cloned into the *Eco*RI site of the *SUP4*-o gene, an ochre-suppressing allele of a tRNA^{Tyr} gene. Because of the infrequent cutting by these restriction endonucleases, digesting human DNA with them will generate large fragments of DNA that include much of the TCR alpha and beta loci.

To add the polylinker sequence to the pYAC4-neo vector, DNA is isolated and digested with *Eco*RI and then ligated in the presence of annealed oligonucleotides encoding the polylinker sequence to yield mod-pYAC4-neo. The oligonuleotide sequences are as follows: 
FseI KspI AscI
pYAC4 Oligo-(1) 5'-AATTCggCCggCCCCgCggggCgCgCCg-3'
 pYAC4 Oligo-(2) 5'AATTCggCgCgCCCCgCggggCCggCCg-3'

The mod-pYAC4-neo vector is then used to transform *E. coli* cells to generate large amounts of the vector DNA for cloning and manipulation of large human DNA fragments that are >500 Kbp in length.

### Example 4

### Cloning of Human TCRα Locus into mod-pYAC⁴-neo vector:

High molecular weight DNA is prepared from circulating leukocytes that are harvested from whole blood by a modification of the method of Luzzatto (Luzzatto, L. (1960) Biochem. Biophys. Res. Commun. 2:402). The DNA is purified by a sucrose step-gradient procedure originally developed for the isolation of intact chromosomal DNA molecules from yeast spheroplasts. Although this protocol involves only a one-step purification of a crude lysate, it produces DNA samples that are free of contaminating nucleases and readily cleaved by most restriction endonucleases. A detailed protocol for isolating high molecular weight human DNA can be found in Methods in Enzymology (1991) 194:251-270. The human TCRα locus is located on chromosome 14q11.2 and has been sequenced in its entirety and deposited into the National Center for Biotechnology Information (NCBI) nucleotide database (FIG. 4a). Our primary objective is to create a human TCR expressing transgenic animal that displays extensive TCR diversity. Therefore, we believe it would be best to use restriction endonucleases that cut infrequently to generate a large but manageable DNA fragment that would include most if not all of the TCR variable exons and all the joining segments. The access to nucleotide sequence information, particularly that of the human α/β TCR loci, has greatly enhanced the ability to identify unique cutting restriction endonuclease enzymes for digesting the human DNA into fragments that encode for the locus of interest for cloning into YAC vectors. Using the NCBI database and Vector NTI software, we are able to generate a restriction map of the TCRα locus (see FIG. 4B-F). The analysis revealed that one enzyme, *Ksp*I, will digest the human TCRα locus at bp 72426 or 5' of the first variable exon (TCRAV1). It also cuts the DNA a second time downstream of the 3' enhancer at 1,060,946 bp. The *Ksp*I fragment product is 988,520 bp or almost 1 megabase (Mbp) in length. By having this information we will size fractionate the DNA and isolate DNA fragments of approximately 1 mega base for cloning into mod-pYAC4 vector. Briefly, the digested sample will be size fractionated and purified using pulse field gel electrophoresis (PFGE). Using this protocol we should eliminate contaminating *Ksp*I digested fragments that are smaller than 1 Mbp in length. This will increase the efficiency of cloning into the YAC vector as well as facilitate the isolation of a DNA fragment containing the human TCRα locus. After PFGE, the gel will be stained with ethidium bromide and the 1 Mbp band will be excised and the DNA will be isolated using GELase and ethanol precipitation (EpiCenter, Madison, WI). Highly pure, intact DNA that is recovered will then be cloned into the mod-pYAC4-neo vector.

### Mod-pYAC4-neo(α):

The generation of HuTCRα YAC vector is accomplished by digesting the cloning vector with BamHI and with *Ksp*I to yield left and right arm products that are then dephosphorylated. The function of the phosphatase treatment is to prevent the formation of concatenated vector fragments that would later be difficult to separate from the desired ligation products by size fractionation. In more specific terms, 40-100 µg of insert DNA is mixed with an equal weight of prepared mod-pYAC4-neo vector. Adjust the volume to 250 µl and the buffer composition with New England Biolabs (NEB) ligation reaction buffer and then add 1000 units of NEB T4 DNA ligase and allow ligation reaction to incubate at 15°C for 10 hours.

Transformation:The ligated material will then be used to transform yeast spheroplasts (Burgers, P.M.J. and Percival, K.J. (1987) Anal. Biochem. 163: 391-397). We have chosen the yeast strain AB1380 for a transformation host since it has been widely used as a host by others, however, other yeast host strains, such as YPH925, may also be suitable. Transformants will be selected on a synthetic medium that lacks uracil; these plates are prepared following standard recipes. These transformants will be screened to identify positives which carry the HuTCRα YAC vector, also designated mod-pYAC-neo(α).

### Colony Screening:

The colony screening protocol involves growing the colonies on the surface of a nylon membrane, spheroplasting the yeast, lysing the spheroplasts with detergent, and denaturing the DNA with base. We will use the protocol described by Brownstein, *et al.* (Brownstein, B.H., Silverman, R.D., Little, R.D., Burke, D.T., Korsmeyer, S.J., Schlessinger, D., and Olson, M.V. (1989) Science 244: 1348). Briefly, this protocol uses the technique of colony lift replica plating to make duplicate filters, one which will provide colonies for probing and a duplicate which will provide viable cells for the propagation of positive clones. Yeast colonies are grown to the appropriate size for screening colonies on the nylon filter. This requires approximately 2 days of growth at 30°C. One of the duplicate filters containing colonies is transferred to a thick paper filter saturated with 2 mg/ml of yeast lytic enzyme [ICN #152270, >70,000 units (U)/g], in 1.0 M sorbitol, 0.1 M sodium citrate, 50 mM EDTA, and 15 mM dithiothreitol (pH of the enzyme buffer adjusted to 7) and incubated overnight at 30°C. The membrane is transferred to a paper filter saturated with 10% sodium dodecyl sulfate for 5 minutes at room temperature. The membrane is then transferred to a paper filter saturated with 0.5 M NaOH for 10 minutes and is neutralized by transferring it to three successive paper filters saturated with 0.3 M NaCl, 30 mM sodium citrate, 0.2 M TrisHCl, pH 7.5 for 5 minutes each time. After the filters have air dried, probing will be carried out using labeled oligonucleotides and standard hybridization and autoradiography techniques. To identify human TCRα locus positive colonies, we will screen colonies using two oligonucleotide probes specific to the 5' and 3' ends of the DNA insert. These oligonucleotides anneal to sites approximately 100 bp downstream of the 5' end *KspI* site and 100 bp upstream of the 3' end *KspI* site respectively and their sequences are as follows:

Screening oligo #1- 5'- GTCTCTACTT TACTAAAAAT ACAAAAATTA GCCAGGTGTG
GTGGTG-3'

Screening oligo #2-5'- GTCACAGGGC TGAGGGAAGG AGACAAGAGC
CTGGACAGCA-3'

### The transgenic Human TCRα Locus:

After assembling the human TCRα transgene locus in mod-pYAC-neo(α), which may contain the entire Vα and Jα exons, the single Cα exon, and the 3' enhancer, we can introduce the HuTCRα YAC sector into non-human embryonic stem cells (ES) by spheroplast fusion with the yeast host strain (Pachnis, V., Pevny, L., Rothstein, R., and Constantini, F. (1990) PNAS 87: 5109-5113; Huxley, C. and Gnirke, A., (1991) Bioessays, 13: 545-550; Davies, N.P and Huxley, C. (1996) in Methods in Molecular Biology, Vol.54: YAC Protocols. Eds. D. Markie. Humana Press Inc., Tolowa, NJ.).

G418 resistance will be used to monitor non-human ES cells that fused successfully with the yeast containing the HuTCRα YAC. Selection of neomycin resistant HuTCRα YAC positive non-human ES cells will be analyzed 2-3 weeks after spheroplast fusion. Following PCR and southern blot analysis identification of the appropriately modified ES cells, five clones will be expended and introduced into blastocysts (Hogan, B.R, Beddington, F., Costantini, F. and Lacy, E. (1994) Manipulating the Mouse embryo: A laboratory Manual. Cold Spring Harbor Laboratory Press, Cold Spring Harbor, NY. pp. 477), and implanted into a pseudo-pregnant female host strain. Breeding of chimeric mice with mice that are MuTCRα negative/ MuTCRβ negative (see Example 10) should result in mice that produce only human TCRα.

### Example 5a

### Cloning of Human TCRβ Locus into mod-pYAC-neo vector.

The human DNA for digestion and cloning will be prepared as described in the Example above.The human TCRβ beta locus is located on chromosome 7q35 and has been sequenced in its entirety and deposited into the NCBI nucleotide database. Using the NCBIdatabase and Vector NTI software, we assembled the human TCRβ locus and carried out nucleotide mapping (see FIG. 5b-h). The analysis of the mapping exercise revealed that digesting human genomic DNA with both FseI and AscI restriction endonucleases will generate a large DNA fragment that would contain 21 out of 30 variable exons, all of the joining and diversity segments, both constant exons and the 3' enhancer. The length of the TCRβ DNA fragment is determined to be 598,054 bp (see Figure 5b-h). By having this information available, we will be able to size fractionate the DNA and isolate DNA fragments in the 500-600 kbp length for cloning into the pYAC vector.

After isolating the DNA, we will digest 100 µg using the two specific restriction endonucleases to generate a fragment containing the majority of the human TCRβ locus. As described in the Example 4, the digested DNA sample will be size fractionated and purified using PFGE. This will increase the efficiency of cloning and the likelihood of isolating a DNA fragment containing the majority of human TCRβ locus. After running the PFGE to completion the 600 Kbp band will be excised from the gel and the DNA will be isolated using GELase (EpiCenter, Madison, WI) and ethanol precipitation. Highly pure and intact DNA will be recovered and then cloned into the mod-pYAC4-neo vector.

### Mod-pYAC4-neo(β)

The preparation of the mod-pYAC4-neo(β) vector is similar to the procedure used for cloning the human TCRα locus except that the vector DNA is first digested with *Bam*HI and then with *Fse*I and *Asc*I. Ligation of insert DNA into the pYAC vector is similar to that described for the human TCRα locus described in Example 4. After transformation of yeast with the pYAC vector containing the human TCRβ locus, we will carry out screening of colonies as described previously.

### The transgenic Human TCRβ Locus:

After assembling the human TCRβ translocus in mod-pYAC-neo(β), the construct, HuTCRβ YAC, which may contain the majority of Vβ and the entire Jβ and Dβ segments, the two Cβ exons, and the 3' enhancer, we will introduce the HuTCRβ YAC into non-human embryonic stem cells (ES) by spheroplast fusion (Pachnis, V., Pevny, L., Rothstein, R., and Constantini, F. (1990) PNAS 87: 5109-5113; Huxley, C. and Gnirke, A., (1991) Bioessays, 13: 545-550; Davies, N.P and Huxley, C. (1996) in Methods in Molecular Biology, Vol.54: YAC Protocols. Eds. D. Markie. Humana Press Inc., Tolowa, NJ.) and chimeric mice will be produced by blastocyst injection (Hogan, B.R., Beddington, F., Costantini, F. and Lacy, E. (1994) Manipulating the Mouse embryo: A laboratory Manual. Cold Spring Harbor Laboratory Press, Cold Spring Harbor, NY. pp. 477).

G418 resistance will be used to monitor for HM-1 embryonic stem cells that have been fused with the YAC containing yeast. Selection of neomycin resistant HuTCRβ YAC positive HM-1 ES cells will then be analyzed 2-3 weeks after fusion. ES cells containing a complete HuTCRβ YAC copy will be confirmed by Southern hybridization, and HuTCRβ YAC positive clones will be used to reconstitute blastocysts to produce chimeric animals. Breeding of chimeric animals with C57BL/6J mice that are MuTCRα negative/ MuTCRβ negative (see Example 10) should result in germline transmission and mice that have HuTCRβ locus integrated into their genomes. Gene transmission can be confirmed by Southern blot analysis of tail DNA.

### Example 5b

Mice expressing the human TCRβ or the TCRα gene could also be constructed by alternative methods. For instance, it is possible to reconstruct the TCRβ chain locus with several human YAC clones using information obtained from the NCBI and National Human Genome Research Institute databases. These identified human YACs, which are available from ResGen, contain TCRβ chain sequence and overlapping homology. The first YAC clone, D49H4, contains the 5' end of the TCRβ locus through to the trypsinogen gene repeats, while the second YAC, 940 a 12, contains the 3' end of the TCRβ locus (Figure 5i). Since the two clones have significant regions of overlapping homology, they can be used to assemble a single human TCRβ YAC (HuTCRβ YAC) via homologous recombination. The recombination event, as well, as the lack of random deletions or chimerism, can be confirmed by PCR using primer sets that flank the regions of sequence homology between the two genes, PFGE, and/or Southern blot analysis.

Before introduction of the HuTCRβ YAC into mammalian cells or non-human embryos, the arms of the YAC construct can be modified. The arms of the HuTCRβ YAC are altered to include mouse regulatory sequences and/or mammalian selection cassettes by a technique called 'retrofitting' (Figure 5j). The YAC arms are retrofitted with vectors, such as pRAN4 (Markie, D. et al., (1993) Somatic Cell and Molecular Genetics, 19: 161-169), by a variety of transformation methods described by Eric D. Green in Genome Analysis: A Laboratory Manual, Volume 3 (1999), Cold Spring Harbor Laboratory Press, Cold Spring Harbor, N.Y. The modification of the arms should assist in the selection of successful fusion events between the yeast host strain and non-human ES cells, in addition to boosting expression of the HuTCRβ transgene once it has integrated into the host genome.

After assembly and modification of the HuTCRβ YAC construct, which may contain the majority of the Vβ segments, the entire Jβ and Dβ segments, and the two Cβ exons, the HuTCRβ YAC is introduced into non-human ES cells by spheroplast fusion. Successfully fused ES cells can be used to reconstitute mouse blastocysts and generate chimeras. Alternatively, the construct could be isolated from the yeast host strain and introduced into one-cell stage non-human embryos via microinjection as outlined in Hogan, et al., "Manipulating the Mouse Embryo: A Laboratory Manual", Cold Spring Harbor Laboratory, with slight modifications to prevent shearing of the HuTCRβ YAC construct (Montolui, L. (1996) in Methods in Molecular Biology, Vol.54: YAC Protocols. Eds. D. Markie. Humana Press Inc., Tolowa, NJ.) Resulting offspring can be tested for integration of the HuTCRβ YAC construct in the mouse genome by Southern analysis of tail biopsies. Positively identified animals can be bred to homozygosity and crossed with other existing mouse strains.

### Example 6 Generation of Human TCRα/β mice:

A) Generating a Human TCRα/β mouse through genetic crossing between the HuTCRα mouse and the HuTCRβ mouse:
   Both of the HuTCRα and HuTCRβ containing mice will be breed in a C57BL/6J background. These mice still have functional murine TCRα/β loci that display murine TCR on their T-cells. Successful breeding of these mice should result in the generation of a C57B1/6J transloci mouse that contain functional murine and human TCR. To determine whether the mouse has both the human TCRα/β loci, we will carry out southern hybridization using tail DNA and probing the membranes with either an alpha or a beta specific oligonucleotide.
B) Generating a human TCRα/β positive mouse in a murine TCRα/β knockout background:
   Mice generated in A (that are positive for both mouse and human TCR loci) will then be used in the next round of breeding to put the human TCRα/β loci in to a C57BL/6J background that has the endogenous murine TCR loci deleted or inactivated. This will be carried out by crossing a murine TCRα/β knockout mouse with a mouse that is positive for both the murine TCRα/β loci and the human TCRα/β loci. Screening for positive mice will again be carried out using endonuclease treated DNA from tail snips and along with Southern hybridization techniques. DNA fragments run out on a gel and transferred to a nylon membrane will be probed with specific primers to the TCRα or -β chain respectively. Mice positive human TCRα/β and negative for murine TCR α/β) will be grown up to 8 weeks of age and several will be sacrificed to isolate spleens for staining of splenic T-cells. The identification of T-cells expressing human α/β TCRs is carried out using immunofluorescence and flow cytometry. The approach will rely on using the anti-human TCR specific mAb conjugated with phycoerythrin (TCR Pan α/β, clone BMA031 (IgG2b mouse) Coulter Immunotech, ME).
C) Generating a human TCRα/β positive mouse in a murine TCRα/β knockout background crossed with a C57BL/6J mouse containing the Human MHC class I molecule HLA-A2:
   This is an example of one type of mouse that will be generated to further our needs of creating human α/β TCR that are restricted by a human MHC class I molecule. In this example, we have chosen to use the human class I molecule known as HLA-A2.1. T-cells generated that are reactive to peptides restricted by this MHC molecule are generally of the CD8⁺ phenotype and are cytolytic in nature. The HLA-A2.1 allele is expressed in close to 50% of the population making it the most prevalent form of MHC expressed. To demonstrate reduction to practice, we have chosen to cross the human TCR α/β transgenic mouse with a HLA-A2.1 transgenic mouse generated previously by Dr. Linda Sherman (Sherman and Lustgarten, US Pat. App. 08/812,393 and WO97/32603). We will breed both mice to produce a new mouse that will have the HLA-A2.1 allele and the human TCRα/β loci. This mouse will also contain the endogenous murine MHC class I and II loci as we have not carried out any further modification of these loci. In the future it may be desirable to generate knockouts of the murine MHC class I and II loci. In the present discussion we have limited our description to generating knockouts of the murine TCRα/β which are also human TCRα/β and the HLA-A2 transgenic. We will screen for positive mice using Southern hybridization and flow cytometry. Furthermore, we will generate T-cell clones reactive to a defined peptide antigen presented by HLA-A2 molecules and then carry out PCR analysis of their VJ and VDJ rearrangements. This will also be followed by additional characterization of TCR expression on T-cells by immunofluorescent staining using Vα and Vβ family specific mAb (see Immunotech catalogue).

### Example 7

### Testing the human TCR transgenic mice for functional human TCR:

To assess the functionality of the human TCR transgenes, T-cells isolated from these mice will be stained with a panel of antibodies specific for human TCR α and β variable regions and analyzed by flow cytometry. In addition mRNA will be isolated from these cells and the structure of TCR cDNA clones will be examined.

Splenic T-cells will be isolated from mice that contain a deletion of the constant regions at the endogenous murine TCR α and β chain loci, and a single copy of the unrearranged human TCR α and TCR β chain transgene loci. These cells will be stained with a panel of antibodies specific for human α or β variable regions (from Coulter Immunotech) and analyzed by flow cytometry. This will allow analysis of the total number and diversity of T-cells with functionally rearranged α and β chains to be assessed. Evaluation of the proportional distribution of the various variable regions in relation to their expression in human T-cells will also be carried out.

Poly-adenylated RNA will also be isolated from an eleven-week old male second generation human TCR α/β transgenic mouse. This RNA will be used to synthesize single stranded cDNA primed with oligo-dT / SMART II oligonucleotide (Clonetech). The resulting cDNA will then be used as template for SMART RACE PCR amplifications using synthetic oligonucleotide primers specific for the human α or β constant regions and the Universal primer mix from Clonetech. Amplified fragments of the appropriate size will be isolated from agarose gels, cloned in pGEM T-Easy and sequenced.

The sequences will be examined for the overall diversity of the transgene encoded chains, focusing on D and J segment usage, N region addition, CDR3 length distribution, and the frequency of junctions resulting in functional mRNA molecules will be examined.

### Example 8

### Immunization and immune response in a transgenic TCR / HLA-A2 mouse:

This example demonstrates the successful immunization and immune response in a transgenic mouse of the present invention.

### Peptide priming of transgenic mouse (HuTCR α/β / muTCR α- /β- - HLA-A2.1) and propagation of CTL lines:

Mice will be injected subcutaneously at the base of the tail with 100 µg. of the 264 peptide (amino acids 264-272 from human p53 tumor suppressor protein) and 120 µg. of the I-Ab binding synthetic T-helper peptide representing residues 128-140 of the hepatitis B virus core protein (Sette, A., Vitiello, A., et al. (1994) J. Immunol. 153:5586) emulsified in 100 µL of incomplete Freund's adjuvant. After 10 days, spleen cells of primed mice will then be cultured with irradiated A2.1-transgenic, lipopolysaccharide (LPS)-activated spleen cell stimulators that will be pulsed with the indicated priming peptide at 5 µg/mL and human beta 2-microglobulin at 10 µg/mL. After 6 days, the resultant effector cells will be assayed in a 4-hr ⁵¹Cr-release assay at various E/T ratios for lytic activity against T2 cells that are pulsed with either the indicated priming peptide, an unrelated A2.1 binding peptide, or no peptide. Polyclonal CTL lines specific for 264 peptide (CTL A2 264) will be established by weekly restimulation of effector CTLs with irradiated JA2 cells that will be pulsed with 5 µg of the 264 peptide, irradiated C57BL/6 spleen filler cells and 2% (vol/vol) rat Con A supernatant. This protocol has been described by Theobald, M., et al. (1995) Proc. Natl. Acad. Sci. 92:11993.

### Analysis of Human TCR reactivity and clonal diversity:

TCR reactivity and specificity will be assessed using an *in vitro* cytotoxic killing assay and immunofluoresent staining with anti-Vα and anti-Vβ specific mAbs and 264/HLA-A2 tetramers (Altman, J., et al. (1996) Science 274:94-96). CTL lines will be propagated and then cloned using standard limiting dilution techniques. Individual clones will be assayed for specificity through staining with A2 tetramers containing the 264 peptide and with A2 tetramers containing an irrelevant peptide that should not be recognized by the 264 specific T-cell clones, and in cytotoxic killing assays. Results from these assays will be useful in demonstrating TCR specificity for the 264 peptide/HLA-A2.1 complex.

To characterize the diversity of the human TCR repertoire in these transgenic mice, we will further characterize the α and β variable family usage via antibody staining. Several Vα and Vβ specific mAbs are commercially available that will be used to determine overall variable family usage of the human TCRs. Furthermore, SMART RACE PCR analysis (see Example 7 and below) will be carried out on T-cell clones that demonstrate specificity for the 264 peptide/HLA-A2.1 complex. We will analyze the sequences for the characteristics mentioned in Example 7 and evaluate the effect of expression of the transgene on allelic exclusion.

### Generation of cell lines producing recombinant TCR molecules:

### A. Isolation of genomic clones corresponding to rearranged and expressed copies of TCR α and β chains.

Cells from an individual hybridoma clone that is reactive for the peptide antigen/MHC complex of interest will be used to prepare genomic DNA. Such cells may contain multiple alleles of a given TCR gene. For example, a hybridoma might contain four copies of the TCR genes (two TCR copies from the fusion partner cell line and two TCR copies from the original T-cell expressing the TCR of interest). Of these four copies, only one encodes the TCR of interest, despite the fact that several of them may be rearranged. The procedure described in this example allows for the selective cloning of the expressed copy of the TCR α and β chains.

### Double Stranded cDNA:

Cells from human hybridoma, or lymphoma, or other cell line that synthesizes the TCR are used for the isolation of total or polyA⁺ RNA. The RNA is then used for the synthesis of 5'-RACE-Ready cDNA using the enzyme reverse transcriptase and the SMART II oligo (Clonetech Laboratories, User Manual PT3269-1, March 1999). The single stranded cDNA is then used as template for second strand synthesis (catalyzed by Taq polymerase) using the following oligonucleotides as a primers:
Vβ (near C term) VW510: ATCCTTTCTCTTGACCATGGCCATC
Vα (near C term) VW512: GCTGGACCACAGCCGCAGCGTCATG

The double stranded cDNA is isolated, cloned and used for determining the nucleotide sequence of the mRNAs encoding the alpha and beta chains of the expressed TCR molecule. Genomic clones of these expressed genes are then isolated. The procedure for cloning the expressed alpha chain gene is outlined below.

### Alpha Chain:

Twenty to forty nucleotides of sequence that span the V-N-J junction will then be used to synthesize a unique probe for isolating the gene from which TCR message is transcribed. This synthetic nucleotide segment of DNA will be referred to below as o-alpha.

A Southern blot of DNA, isolated from the TCR expressing cell line and digested individually and in pairwise combinations with several different restriction endonucleases, is then probed with the ³²P labeled unique oligonucleotide o-alpha. A unique restriction endonuclease site is identified upstream of the rearranged V segment.

DNA from the TCR expressing cell line is cut with an appropriate restriction enzyme(s). The DNA is size fractionated by agarose gel electrophoresis. The fraction including the DNA fragment covering the expressed V segment is cloned into lambda Gem-12 or EMBL3 SP6/T7 (Promega, Madison, WI or Clonetech, Palo Alto, CA) or, if the fragment is small enough, directly into pGEM series vectors. V segment containing clones are isolated using the unique probe o-alpha. Large fragment DNA is isolated from positive clones and subcloned into the polylinker of pGEM (Promega) or the equivalent. The resulting clone is called pgTRAr.

### Beta Chain:

Twenty to forty nucleotides of sequence that span the V-N-D-N-J junction will then be used to synthesize a unique probe for isolating the gene from which TCR message is transcribed. This synthetic nucleotide segment of DNA will be referred to below as o-beta.

A Southern blot of DNA, isolated from the TCR expressing cell line and digested individually and in pairwise combinations with several different restriction endonucleases, is then probed with the ³²P labeled unique oligonucleotide o-beta. A unique restriction endonuclease site is identified upstream of the rearranged V segment.

DNA from the TCR expressing cell line is cut with an appropriate restriction enzyme(s). The DNA is size fractionated by agarose gel electrophoresis. The fraction including the DNA fragment covering the expressed V segment is cloned into lambda Gem-12 or EMBL3 SP6/T7 (Promega, Madison, WI or Clonetech, Palo Alto, CA) or, if the fragment is small enough, directly into pGEM series vectors. V segment containing clones are isolated using the unique probe o-alpha. Large fragment DNA is isolated from positive clones and subcloned into the polylinker of pGEM (Promega) or the equivalent. The resulting clone is called pgTRBr.

### Construction of three domain TCR expression vector and expression in mammalian cells:

The cloned inserts in pgTRAr and pgTRBr are then PCR amplified with the appropriate oligonucleotides and subcloned into a three-domain single chain Vα - Vβ/Cβ construct into pGem vector.

The three-domain single-chain TCR will then be cloned into the vector pSUN27 for expression as a single chain TCR kappa constant chain fusion protein. The resulting vector is used to transfect Chinese Hamster Ovary cells via electroporation to generate cell lines that produce soluble scTCR-κ fusion protein so that binding affinity to the peptide antigen/HLA-A2 molecule can be evaluated.

Alternatively, DNA encoding the alpha and beta chains, isolated from the cloned hybridoma cells described above, is used to construct the Vα, Vβ-Cβ fragments required for the three domain TCR cloned in pGEM. This construct is then transferred to pSUN27 and the protein is produced and evaluated as described above.

### Example 9

### Preparation of HLA-A2 minilocus:

### Cloning the HLA-A2.1 from LCL 721.

The genomic DNA is isolated from the human LCL 721 cell line and digested with the restriction enzyme *Hind*III. The *Hind*III-digested genomic DNA is size-selected on a 0.7% agarose gel and the fractionated DNA is purified. The purified genomic DNA is then ligated into the *Hind*III site of pBR3222. The ligated DNA is transformed into *E. coli* LE392. Recombinant bacteria containing the HLA-A2 gene are detected by colony hybridization (Hanahan, D, and M. Meselson (1980) Gene 10:63-67), using the synthetic oligonucleotide 5'-TGTCTCCCCGTCCCAAT-3' as a probe. Subsequently, the cloned 5.1 kb fragment containing the HLA-A2 gene is subcloned into the *Hind*III site pcDNA3.1(+) (InVitrogen, Carlsbad, CA).

### Production and Detection of HLA-A2.1 Transgenic Mice.

Transgenic mice are then produced using a standard protocol (Hogan, G. et al. (1986) Manipulating the Mouse Embryo: A Laboratory Manual, Cold Spring Harbor Laboratory, Cold Spring Harbor, NY) by injecting the linearized pcDNA3.1(+) carrying the HLA-A2 gene into fertilized eggs obtained by crossing (C57BL/6J x DBA/2)F1 mice. Transgenic lines are established from mice carrying the transgene as detected by tail DNA dot blot analysis. Two transgenic lines are selected based on cell surface expression of the transgene product. To detect cell surface expression of HLA-A2, spleen cells or peripheral blood (0.5 mL) collected from the tail vein of test mice are treated with Tris-buffered ammonium chloride (5 mL) to lyse red blood cells. Cells are washed and resuspended in RPMI 10% supplemented with 2.5 µg/mL ConA, 250 ng/mL ionomycin, 3 ng/mL PMA, and 5% culture supernatant of Con A-activated rat splenocytes. Samples are incubated at 3x10⁶ cells/well in a volume of 2 mL for 3 days at 37°C in a humidified 5% CO₂ atmosphere. HLA-A2.1 cell surface expression is assessed by flow cytometry (FACS; Becton Dickinson & Co, Mountain View, CA) using a biotinylated HLA-A2.1-specific mAb BB7.2 (Parham, P. et al. (1981) Hum. Immunol. 3:277) and PE-conjugated streptavidin (Biomeda, Foster City, CA). Cells are analyzed using a flow cytometer. One transgenic line is maintained by back-crossing to B10.D22 and the another transgenic line by back-crossing to C57BL/6J. Heterozygous offspring are back-crossed to C57BL/6J or B10.D22 animals and then intercrossed at the N2 generation to give rise to independent homozygous strains.

### Example 10

### Generation of mice that are negative for both murine TCR α and β loci (MuTCR α-/β- or MuTCR αKO/βKO):

This example describes the creation of a mouse strain that is negative for both the alpha and beta loci of the TCR. This will be accomplished by breeding a mouse homozygous for the TCRα chain knockout with one homozygous for the TCRβ chain knockout.

In order to generate mice homozygous for both the TCRα chain knockout (see Example 1) and the TCRβ chain knockout (see Example 2), mice homozygous for each knockout are bred together to generate offspring heterozygous at each locus. These heterozygotes are crossed and the resulting offspring screened by Southern blot analysis. Screening for the presence of the β chain knockout is carried out by Southern blot analysis of *Bam*HI-digested DNA from tail biopsies, using probe B described in Example 2 (see FIG. 2a). Those offspring showing a 7.4 kb band indicative of a β chain knockout and lacking the 10.4 kb wild-type band are further screened for the presence of inactivated α chain. Probe A from Example 1 (α chain probe, see FIG. 1d) was used to screen Southern blots of *Bam*HI-digested DNA. This probe detects a 8.9 kb fragment in the wild-type locus, and a diagnostic 2.4 kb band in an α chain knockout. The absence of wild-type DNA sequences is confirmed by probing the *Bam*HI digested DNA with the Cα exon 2 probe and the Cβ 1 and/or Cβ 2 probe(s) and finding no bands which hybridize. This combination of diagnostic tests would indicate the generation of a novel mouse in which both copies of the murine TCR α and β loci have been inactivated by deletion as a result of targeted mutation. This mouse would be referred to as a MuTCR α-/β- or MuTCR αKO/βKO mouse.

### REFERENCES

U.S.S.N 09/422,375, U.S.S.N. 08/943,086, and U.S.S.N. 08/813,781 WO97/32603 Sept. 12, 1997
US Pat. 5,877,397
US Pat. 6,150,584 A
WO 98/24893 A2, June 11, 1998
USSN 08/812,393
Janeway and Travers, Immunobiology 1997
Chung S. et al. (1994) Proc. Natl. Acad. Sci. 91: 12654-12658
Rothe J. et al (1993) International Immunology 5: 11-17
Viney J.L. et al. (1992) Hybridoma 11: 701-713
Evans, M. J., et al. (1981) Nature 292: 154-156
Bradley, M. O., et al. (1984) Nature 309: 255-258
Gossler, et al. (1986) Proc. Natl. Acad. Sci. 83: 9065-9069
Robertson, et al. (1986) Nature 322: 445-448
Jaenisch, R. (1988) Science 240: 1468-1474
Jaenich, R. (1976) Proc. Natl. Acad. Sci. 73: 1260-1264
Hogan, et al. (1986) in Manipulating the Mouse Embryo, Cold Spring Harbor Laboratory Press, Cold Spring Harbor, N.Y.
Brinster, et al. (1985) Proc. Natl. Acad. Sci. 82: 4438-4442
Proc. Natl. Acad. Sci. (1994) 91: 9302-9306
Proc. Natl. Acad. Sci. (1996) 93: 10933-10938
Curr. Opin. Biotechnol. (1994) 5: 521-527
Momberts, et al. (1991) PNAS 88:3084-3087
Momberts, et al. (1992) Nature 360: 225-231
Samuelson, et al. (1983) Proc. Natl. Acad. Sci. 80: 6972
Acuto, et al. (1983) Cell 34: 717
MacIntyre, et al. (1983) Cell 34: 737
Hendrick, et al. (1984) Nature 308: 149
Hendrick, et al. (1984) Nature 308: 153
Yanagi, et al. (1984) Nature 308: 145
Saito, et al. (1987) Nature 325: 125
Chien, et al. (1984) Nature 312: 314
Davis and Bjorkman (1988) *supra.*
Kronenberg, et al. (1986) Ann. Rev. Immunol. 4: 529
Acuto, *et al.* (1983) *supra*
Kappler, et al. (1983) Cell 35: 295
Rowen, et al. (1996), Science 272: 1755
Akira (1987) Science 238: 1134
Yancopoulos, *et al.* (1986) *supra*
Chien, *et al.* (1987) *supra*
Pardoll, et al. (1987) Nature 326: 79
Raulet, et al. (1985) Nature 312: 36
Samelson, et al. (1985) Nature 315: 765
Snodgrass, et al. (1985) Nature 315: 232
Berman et al., EMBO J. (1988) 7: 727-738
Fugger, et al. (1994) PNAS 91: 6151-6155
Medsen, et al. (1999) Nature Genetics 23: 343-347
Kieffer, et al. (1997) J. Immunol. 159: 4907-4912
Teratocarcinomas and embryonic stem cells: a practical approach, E. J. Robertson, ed., IRL Press, Washington, D.C., 1987
Zjilstra, et al. (1989) Nature 342:435-438
Schwartzberg et al. (1989) Science 246:799-803
J. Sambrook, et al. in Molecular Cloning: A Laboratory Manual, 2d ed. (1989), Cold Spring Harbor Laboratory Press, Cold Spring Harbor, N.Y.
"Antibodies: A Laboratory Manual", Ed Harlow and David Lane, Cold Spring Harbor Laboratory (1988)
Mansour, et al. (1988), Nature 336:348-352
McMahon and Bradley (1990), Cell 62:1073-1085
Robertson, E. J. (1987) in Teratocarcinomas and Embryonic Stem Cells: A Practical Approach. E. J. Robertson, ed. (Oxford: IRL Press), p. 71-112
Genome Analysis: A Laboratory Manual, Volume 3 (1999), Cold Spring Harbor Laboratory Press, Cold Spring Harbor, N.Y.
Hooper, et al. (1987) Nature 326:292-295
Doetschman, et al. (1985) J. Embryol. Exp. Morph. 87:27-45
Robertson, et al. (1986) Nature 323:445-448
Hasty, et al. (1991), Nature, 350:243-246
Laird, et al. (1991), Nucl. Acids Res. 19:4293
Bradley, A. (1987) in Teratocarcinomas and Embryonic Stem Cells: A Practical Approach. E. J. Robertson, ed. (Oxford: IRL Press), p. 113-151
Burke, D.T., Carle, G.F. and Olson, M.V. (1987) Science 236: 806
Bruggemann, M, and Neuberger, M.S. (1996) Immunol. Today 7:391
Cooke, H. and Cross, S. (1988) Nucleic Acids Res. 16: 11817
Luzzatto, L. (1960) Biochem. Biophys. Res. Commun. 2:402
Methods in Enzymology (1991) 194:251-270
Burgers, P.M.J. and Percival, K.J. (1987) Anal. Biochem. 163: 391-397
Brownstein, B.H., Silverman, R.D., Little, R.D., Burke, D.T., Korsmeyer, S.J., Schlessinger, D., and Olson, M.V. (1989) Science 244: 1348
Pachnis, V., Pevny, L., Rothstein, R., and Constantini, F. (1990) PNAS 87: 5109-5113
Huxley, C. and Gnirke, A., (1991) Bioessays, 13: 545-550; Davies, N.P and Huxley, C. (1996) in Methods in Molecular Biology, Vol.54: YAC Protocols. Eds. D. Markie. Humana Press Inc., Tolowa, NJ.
Montolui, L. (1996) in Methods in Molecular Biology, Vol.54: YAC Protocols. Eds. D. Markie. Humana Press Inc., Tolowa, NJ
Davies, N.P., Popov, A.V., Zou, X., and Bruggemann, M. (1996) in Antibody Engineering: A practical Approach. Eds. J. McCafferty, H.R. Hoogenboom, and D.J. Chiswell. IRL Press, Oxford. pp. 59-76
Hogan, B.R., Beddington, F., Costantini, F. and Lacy, E. (1994) Manipulating the Mouse embryo: A laboratory Manual. Cold Spring Harbor Laboratory Press, Cold Spring Harbor, NY. pp. 477
Sette, A., Vitiello, A., et al. (1994) J. Immunol. 153:5586
Theobald, M., et al. (1995) Proc. Natl. Acad. Sci. 92:11993
Altman, J., et al. (1996) Science 274:94-96
Clonetech Laboratories, User Manual PT3269-1, March 1999
Hanahan, D, and M. Meselson (1980) Gene 10:63-67
Parham, P. et al. (1981) Hum. Immunol. 3:277

## Claims

1. A non-human transgenic animal capable of producing human TCR with a substantial TCR repertoire, comprising:
inactivated endogenous T-cell receptor loci which are α and β chain receptor loci; and
transgenes contained within its genome composed of human T-cell receptor loci which are α and β chain receptor loci,
wherein said human T-cell receptor loci are unrearranged.

2. The non-human transgenic animal of claim 1, wherein said human T-cell receptor loci are composed, in operable linkage, of a plurality of human T-cell receptor V genes, and D and/or J and C genes.

3. The non-human transgenic animal of one of claims 1 or 2, wherein said animal is capable of productive VDJC rearrangement and expressing human T-cell receptors.

4. The non-human transgenic animal of any one of claims 1-3, wherein said transgenes undergo productive VDJC rearrangement in lymphocytes of said non-human transgenic animal and wherein T-cells express detectable amounts of transgenic TCR in response to antigenic stimulation.

5. The non-human transgenic animal of any one of claims 1-4, wherein said non-human transgenic animal produces an immune response to an antigen, said immune response comprising a population of T-cells reactive to an antigen and wherein the T-cell receptors comprise a human T-cell receptor.

6. The non-human transgenic animal of any one of claims 1-5, wherein a produced human T-cell receptor is composed of human α and β chains.

7. The non-human transgenic animal of any one of the preceding claims, further comprising:
transgenes contained within its genome composed of human HLA genes of human MHC loci.

8. The non-human transgenic animal of claim 7, wherein said MHC loci contains all human HLA genes.

9. The non-human transgenic animal of claim 7, wherein said MHC loci contains a portion of human HLA genes.

10. The non-human transgenic animal of any one of claims 7-9, wherein said human HLA genes are MHC class I and MHC class II.

11. The non-human transgenic animal of any one of claims 7-10, wherein said non-human transgenic animal produces an immune response to an antigen, said immune response comprising a population of T-cells reactive to antigen presented by the human MHC class I receptors and/or reactive to antigen presented by the human MHC class II receptors.

12. The non-human transgenic animal of any one of claims 7-11, wherein said human HLA genes are MHC class I.

13. The non-human transgenic animal of any one of claims 7-12, wherein said human HLA genes are HLA-A2.

14. The non-human transgenic animal of any one of claims 7-13, wherein said non-human transgenic animal produces an immune response to an antigen, said immune response comprising a population of T-cells reactive to antigen presented by the human MHC class I receptors.

15. The non-human transgenic animal of any one of claims 7-11, wherein said human HLA genes are MHC class II.

16. The non-human transgenic animal of claim 15, wherein said non-human transgenic animal produces an immune response to an antigen, said immune response comprising a population of T-cells reactive to antigen presented by the human MHC class II receptors.

17. The non-human transgenic animal of any one of claims 7-16, wherein said non-human transgenic animal produces an immune response to an antigen, said immune response comprising a population of T-cells reactive to the antigen and wherein the T-cell receptors comprise human α and β chains.

18. A non-human transgenic animal of any one of the preceding claims, further comprising genes contained within its genome a human co-receptor.

19. The non-human transgenic animal of claim 18, wherein said genes encode a CD8 co-receptor and/or a CD4 co-receptor.

20. The non-human transgenic animal of claim 18 or claim 19, wherein said non-human transgenic animal produces an immune response to an antigen, said immune response comprising a population of T-cells reactive to the antigen and wherein the T-cell receptors comprise human T-cell receptors and co-receptor molecules.

21. The non-human transgenic animal of any one of claims 18-20, wherein said non-human transgenic animal produces an immune response to an antigen, said immune response comprising a population of T-cells reactive to antigen presented by human MHC class I receptors and/or reactive to antigen presented by human MHC class II receptors.

22. The non-human transgenic animal of any one of claims 18-21, wherein said co-receptor is a CD8 co-receptor.

23. The non-human transgenic animal of any one of claims 18-22, wherein said non-human transgenic animal produces an immune response to an antigen, said immune response comprising a population of T-cells reactive to the antigen and wherein the T-cells express on their cell-surface human T-cell receptors and co-receptor CD8 molecules.

24. The non-human transgenic animal of any one of claims 18-23, wherein said non-human transgenic animal produces an immune response to an antigen, said immune response comprising a population of T-cells reactive to antigen presented by human MHC class I receptors.

25. The non-human transgenic animal of any one of claims 18-21, wherein said co-receptor is a CD4 co-receptor.

26. The non-human transgenic animal of any one of claims 18-21 and 26, wherein said non-human transgenic animal produces an immune response to an antigen, said immune response comprising a population of T-cells reactive to the antigen and wherein the T-cells express on their cell surface human T-cell receptors and co-receptor CD4 molecules.

27. The non-human transgenic animal of any one of claims 18-21, 25 and 26, wherein said non-human transgenic animal produces an immune response to an antigen, said immune response comprising a population of T-cells reactive to antigen presented by human MHC class II receptors.

28. The non-human transgenic animal of any one of the preceding claims, wherein said animal is any animal which can be manipulated transgenically.

29. The non-human transgenic animal of any one of claims 1-28, wherein said animal is a mouse.

30. The non-human transgenic animal of any one of claims 1-28, wherein said animal is a rat.

31. The non-human transgenic animal of any one of claims 1-28, wherein said animal is a primate.

32. The non-human transgenic animal of any one of claims 1-28, wherein said animal is a chimpanzee.

33. The non-human transgenic animal of any one of claims 1-28, wherein said animal is a goat.

34. The non-human transgenic animal of any one of claims 1-28, wherein said animal is a pig.

35. The non-human transgenic animal of any one of claims 1-28, wherein said animal is a zebrafish.

36. A method of producing a non-human transgenic animal according to claim 1 comprising the steps of:
inactivating endogenous T-cell receptor loci which are α and β chain T-cell receptor loci in a non-human embryo or non-human embryonic stem cell;
inserting transgenes containing active human T-cell receptor loci which are α and β chain receptor loci in said non-human embryo or non-human embryonic stem cell;
producing a transgenic animal from said non-human embryo or non-human embryonic stem cell which contains the active human transgene wherein the animal is capable of producing T-cells that express human T-cell receptors; and
breeding the transgenic animal as needed to produce the transgenic animal of claim 1.

37. A method of producing a non-human transgenic animal according to claim 1 comprising the steps of:
inactivating endogenous T-cell receptor loci in a non-human embryo or non-human embryonic stem cell, wherein said loci are T-cell receptor α or T-cell receptor ß loci;
producing a transgenic animal from said non-human embryo or non-human embryonic stem cell which contains inactivated loci wherein the animal is incapable of expressing said endogenous loci;
crossing a produced transgenic animal having inactivated endogenous T-cell receptor α loci with a produced transgenic animal having inactivated endogenous T-cell receptor ß loci;
selecting progeny having both inactivated endogenous T-cell receptor α and T-cell receptor ß loci;
inserting transgenes containing active human T-cell receptor loci in a non-human embryo or non-human embryonic stem cell wherein said human T-cell receptor loci are human T-cell receptor α or T-cell receptor ß loci;
producing a transgenic animal from said non-human embryo or non-human embryonic stem cell which contains the active human transgene;
crossing a produced transgenic animal having active human T-cell receptor α transgenes with a produced transgenic animal having active human T-cell receptor ß transgenes;
selecting progeny having both active human T-cell receptor α and T-cell receptor ß transgenes wherein the animal is capable of producing T-cells that express human T-cell receptors;
crossing a produced transgenic animal having both inactivated endogenous T-cell receptor α and T-cell receptor β loci with a produced transgenic animal having both active human T-cell receptor α and T-cell receptor ß transgenes;
selecting progeny having inactivated endogenous T-cell receptor α and T-cell receptor ß loci and containing active human T-cell receptor α and T-cell receptor ß transgenes; and
breeding the transgenic animal as needed to produce the transgenic animal of claim 1.

38. The method of any one of claims 36-37 wherein said endogenous T-cell receptor loci are inactivated by a functional limitation of the loci.

39. The method of any one of claims 36-37 wherein said endogenous T-cell receptor loci are inactivated by deleting J segment genes from said loci.

40. The method of any one of claims 36-37, wherein said endogenous T-cell receptor loci are inactivated by deleting D segment genes from said loci.

41. The method of any one of claims 36-37, wherein said endogenous T-cell receptor loci are inactivated by deleting C segment genes from said loci.

42. The method of any one of claims 36-37, wherein said human T-cell receptor loci are unrearranged.

43. The method of any one of claims 36-37, wherein said transgenes containing the active human T-cell receptor loci comprise, in operable linkage, a plurality of human T-cell receptor V genes, and D and/or J and C genes.

44. A method of producing a non-human transgenic animal according to claim 1, further capable of producing heterologous MHC molecules, comprising the steps of:
crossing a transgenic animal expressing human T-cell receptors produced by the method of claim 36 with a transgenic animal containing human MHC loci and expressing human MHC molecules;
selecting progeny transgenic animals which express human T-cell receptors and heterologous MHC molecules; and
breeding the transgenic animal as needed to produce the transgenic animal of claim 1, further capable of producing heterologous MHC molecules.

45. The method of claim 44, wherein said MHC loci contains all human HLA genes.

46. The method of claim 44, wherein said MHC loci contains a portion of human HLA genes.

47. The method of any one of claims 44-46, wherein said human HLA genes are MHC class I and MHC class II.

48. The method of any one of claims 44-47, wherein said human HLA genes are MHC class I.

49. The method of any one of claims 44-47, wherein said human HLA genes are MHC class II.

50. A method of producing a non-human transgenic animal according to claim 1, further capable of producing heterologous MHC molecules, and heterologous co-receptor molecules, comprising the steps of:
crossing a transgenic animal expressing human T-cell receptors and heterologous MHC molecules produced by the method of claim 44 with a transgenic animal containing a heterologous co-receptor genes;
selecting progeny transgenic animals which express human T-cell receptors, heterologous MHC molecules, and heterologous co-receptor molecules; and
breeding the transgenic animal as needed to produce the transgenic animal of claim 1, further capable of producing heterologous MHC molecules, and heterologous co-receptor molecules.

51. The method of claim 50, wherein said heterologous co-receptor is a CD8 co-receptor and a CD4 co-receptor.

52. The method of claim 50, wherein said heterologous co-receptor is a CD8 co-receptor.

53. The method of claim 50 wherein said heterologous co-receptor is a CD4 co-receptor.

54. A method of generating an immortal cell line capable of producing human T-cell receptors, comprising the steps of:
producing a transgenic animal capable of producing human T-cell receptors by the method of claim 37;
inducing an immune response in said animal;
isolating a T-cell expressing human T-cell receptors; and
fusing the isolated T-cell with an immortalizing cell line to generate an immortal cell line capable of producing human T-cell receptors.

55. The method of claim 54, wherein said isolated T-cell expresses TCR specific for a particular antigen of interest.

56. The method of claim 54 or claim 55, wherein said isolated T-cell expresses TCR capable of reacting with a chosen peptide/MHC complex of interest.

57. The method of any one of claims 54-56, wherein said immortalizing cell line is a myeloma cell line.

58. The non-human transgenic animal of claim 1, further containing in its genome transgenes comprising, in operable linkage, a plurality of human T-cell receptor V genes, and their D and/or J and C genes.

59. The non-human transgenic animal of claim 1, wherein the transgenes contained within its genome are contained within the germline.

## Patentansprüche

1. Nicht-humanes transgenes Tier, das dazu in der Lage ist, humanen TCR mit einem umfangreichen TCR-Repertoir herzustellen, umfassend:
inaktivierte endogene T-Zell-Rezeptor-Loci, die α- und β-Ketten-Rezeptor-Loci sind; und
Transgene, die in seinem Genom enthalten sind, zusammengesetzt aus humanen T-Zell-Rezeptor-Loci, die α- und β-Ketten-Rezeptor-Loci sind,
wobei die humanen T-Zell-Rezeptor-Loci nichtrearrangierte Loci sind.

2. Nicht-humanes transgenes Tier gemäß Anspruch 1, wobei die humanen T-Zell-Rezeptor-Loci in funktionaler Verknüpfung aus einer Vielzahl von humanen T-Zell-Rezeptor V-Genen, und D-und/oder J- und C-Genen zusammensetzt sind.

3. Nicht-humanes transgenes Tier gemäß einem der Ansprüche 1 oder 2, wobei das Tier zu einem produktiven VDJC-Rearrangement und zur Expression humaner T-Zell-Rezeptoren in der Lage ist.

4. Nicht-humanes transgenes Tier gemäß einem der Ansprüche 1 bis 3, wobei die Transgene produktives VDJC-Rearrangement in Lymphozyten des nicht-humanen transgenen Tiers erfahren, und wobei T-Zellen nachweisbare Mengen an transgenem TCR in Antwort auf antigenische Stimulierung exprimieren.

5. Nicht-humanes transgenes Tier gemäß einem der Ansprüche 1 bis 4, wobei das nicht-humane transgene Tier eine Immunantwort gegen ein Antigen hervorbringt, wobei die Immunantwort eine Population von T-Zellen umfasst, die reaktiv für ein Antigen sind, und wobei die T-Zell-Rezeptoren einen humanen T-Zell-Rezeptor umfassen.

6. Nicht-humanes transgenes Tier gemäß einem der Ansprüche 1 bis 5, wobei ein hergestellter humaner T-Zell-Rezeptor aus humanen α- und β-Ketten zusammengesetzt ist.

7. Nicht-humanes transgenes Tier gemäß einem der vorhergehenden Ansprüche, weiter umfassend:
Transgene, die innerhalb seines Genoms enthalten sind, und die aus humanen HLA-Genen von humanen MHC-Loci zusammengesetzt sind.

8. Nicht-humanes transgenes Tier gemäß Anspruch 7, wobei die MHC-Loci alle humanen HLA-Genen enthalten.

9. Nicht-humanes transgenes Tier gemäß Anspruch 7, wobei die MHC-Loci einen Teilbereich von humanen HLA-Genen enthalten.

10. Nicht-humanes transgenes Tier gemäß einem der Ansprüche 7 bis 9, wobei die humanen HLA-Gene MHC-Klasse-I und MHC-Klasse-II sind.

11. Nicht-humanes transgenes Tier gemäß einem der Ansprüche 7 bis 10, wobei das nicht-humane transgene Tier eine Immunantwort gegen ein Antigen hervorbringt, wobei die Immunantwort eine Population von T-Zellen umfasst, die reaktiv für Antigen sind, das von den humanen MHC-Klasse-I-Rezeptoren präsentiert wird, und/oder reaktiv für Antigen sind, das von den humanen MHC-Klasse-II-Rezeptoren präsentiert wird.

12. Nicht-humanes transgenes Tier gemäß einem der Ansprüche 7 bis 11, wobei die humanen HLA-Gene MHC-Klasse-I sind.

13. Nicht-humanes transgenes Tier gemäß einem der Ansprüche 7 bis 12, wobei die humanen HLA-Gene HLA-A2 sind.

14. Nicht-humanes transgenes Tier gemäß einem der Ansprüche 7 bis 13, wobei das nicht-humane transgene Tier eine Immunantwort gegen ein Antigen hervorbringt, wobei die Immunantwort eine Population von T-Zellen umfasst, die reaktiv für Antigen sind, das von den humanen MHC-Klasse-I-Rezeptoren präsentiert wird.

15. Nicht-humanes transgenes Tier gemäß einem der Ansprüche 7 bis 11, wobei die humanen HLA-Gene MHC-Klasse-II sind.

16. Nicht-humanes transgenes Tier gemäß Anspruch 15, wobei das nicht-humane transgene Tier eine Immunantwort gegen ein Antigen hervorbringt, wobei die Immunantwort eine Population von T-Zellen umfasst, die reaktiv für Antigen sind, das durch die humanen MHC-Klasse-II-Rezeptoren präsentiert wird.

17. Nicht-humanes transgenes Tier gemäß einem der Ansprüche 7 bis 16, wobei das nicht-humane transgene Tier eine Immunantwort gegen ein Antigen hervorbringt, wobei die Immunantwort eine Population von T-Zellen umfasst, die reaktiv für das Antigen sind und wobei die T-Zell-Rezeptoren humane α- und β-Ketten umfassen.

18. Nicht-humanes transgenes Tier gemäß einem der vorhergehenden Ansprüche, weiter Gene eines humanen Co-Rezeptors umfassend, die innerhalb seines Genoms enthalten sind.

19. Nicht-humanes transgenes Tier gemäß Anspruch 18, wobei die Gene einen CD8-Co-Rezeptor und/oder einen CD4-Co-Rezeptor codieren.

20. Nicht-humanes transgenes Tier gemäß Anspruch 18 oder Anspruch 19, wobei das nicht-humane transgene Tier eine Immunantwort gegen ein Antigen hervorbringt, wobei die Immunantwort eine Population von T-Zellen umfasst, die reaktiv für das Antigen sind, und wobei die T-Zell-Rezeptoren humane T-Zell-Rezeptoren und Co-Rezeptor-Moleküle umfassen.

21. Nicht-humanes transgenes Tier gemäß einem der Ansprüche 18 bis 20, wobei das nicht-humane transgene Tier eine Immunantwort gegen ein Antigen hervorbringt, wobei die Immunantwort eine Population von T-Zellen umfasst, die reaktiv für Antigen sind, das von humanen MHC-Klasse-I-Rezeptoren präsentiert wird, und/oder reaktiv sind für Antigen, das von humanen MHC-Klasse-II-Rezeptoren präsentiert wird.

22. Nicht-humanes transgenes Tier gemäß einem der Ansprüche 18 bis 21, wobei der Co-Rezeptor ein CD8-Co-Rezeptor ist.

23. Nicht-humanes transgenes Tier gemäß einem der Ansprüche 18 bis 22, wobei das nicht-humane transgene Tier eine Immunantwort gegen ein Antigen hervorbringt, wobei die Immunantwort eine Population von T-Zellen umfasst, die reaktiv für das Antigen sind, und wobei die T-Zellen auf ihrer Zelloberfläche humane T-Zell-Rezeptoren und Co-Rezeptor-CD8-Moleküle exprimieren.

24. Nicht-humanes transgenes Tier gemäß einem der Ansprüche 18 bis 23, wobei das nicht-humane transgene Tier eine Immunantwort gegen ein Antigen hervorbringt, wobei die Immunantwort eine Population von T-Zellen umfasst, die reaktiv für Antigen sind, das von humanen MHC-Klasse-I-Rezeptoren präsentiert wird.

25. Nicht-humanes transgenes Tier gemäß einem der Ansprüche 18 bis 21, wobei der Co-Rezeptor ein CD4-Co-Rezeptor ist.

26. Nicht-humanes transgenes Tier gemäß einem der Ansprüche 18 bis 21 und 26, wobei das nicht-humane transgene Tier eine Immunantwort gegen ein Antigen hervorbringt, wobei die Immunantwort eine Population von T-Zellen umfasst, die reaktiv für das Antigen sind, und wobei die T-Zellen auf ihrer Zelloberfläche humane T-Zell-Rezeptoren und Co-Rezeptor-CD4-Moleküle exprimieren.

27. Nicht-humanes transgenes Tier gemäß einem der Ansprüche 18 bis 21, 25 und 26, wobei das nicht-humane transgene Tier eine Immunantwort gegen ein Antigen hervorbringt, wobei die Immunantwort eine Population von T-Zellen umfasst, die reaktiv für Antigen sind, das von humanen MHC-Klasse-II-Rezeptoren präsentiert wird.

28. Nicht-humanes transgenes Tier gemäß einem der vorhergehenden Ansprüche, wobei das Tier jegliches Tier ist, das transgen manipuliert werden kann.

29. Nicht-humanes transgenes Tier gemäß einem der Ansprüche 1 bis 28, wobei das Tier eine Maus ist.

30. Nicht-humanes transgenes Tier gemäß einem der Ansprüche 1 bis 28, wobei das Tier eine Ratte ist.

31. Nicht-humanes transgenes Tier gemäß einem der Ansprüche 1 bis 28, wobei das Tier ein Primat ist.

32. Nicht-humanes transgenes Tier gemäß einem der Ansprüche 1 bis 28, wobei das Tier ein Schimpanse ist.

33. Nicht-humanes transgenes Tier gemäß einem der Ansprüche 1 bis 28, wobei das Tier eine Ziege ist.

34. Nicht-humanes transgenes Tier gemäß einem der Ansprüche 1 bis 28, wobei das Tier ein Schwein ist.

35. Nicht-humanes transgenes Tier gemäß einem der Ansprüche 1 bis 28, wobei das Tier ein Zebrafisch ist.

36. Verfahren zur Herstellung eines nicht-humanen transgenen Tieres gemäß Anspruch 1, das die folgenden Schritte umfasst:
Inaktivieren endogener T-Zell-Rezeptor-Loci, die α- und β-Ketten-T-Zell-Rezeptor-Loci sind, in einem nicht-humanen Embryo oder in einer nicht-humanen embryonalen Stammzelle;
Einführen von Transgenen, die aktive humane T-Zell-Rezeptor-Loci enthalten, und die α- und β-Ketten-Rezeptor-Loci sind, in den nicht-humanen Embryo oder in die nicht-humane embryonale Stammzelle;
Herstellen eines transgenen Tiers aus dem nicht-humanen Embryo oder aus der nicht-humanen embryonalen Stammzelle, der/die das aktive humane Transgen enthält, wobei das Tier dazu in der Lage ist, T-Zellen herzustellen, die humane T-Zell-Rezeptoren exprimieren; und
Züchten des transgenen Tieres, wie es benötigt wird, um das transgene Tier gemäß Anspruch 1 herzustellen.

37. Verfahren zur Herstellung eines nicht-humanen transgenen Tieres gemäß Anspruch 1, das die folgenden Schritte umfasst:
Inaktivieren endogener T-Zell-Rezeptor-Loci in einem nicht-humanen Embryo oder in einer nicht-humanen embryonalen Stammzelle, wobei die Loci T-Zell-Rezeptor-α- oder T-Zell-Rezeptor-β-Loci sind;
Herstellen eines transgenen Tieres aus dem nicht-humanen Embryo oder aus der nicht-humanen embryonalen Stammzelle, der/das inaktivierte Loci enthält, wobei das Tier nicht dazu in der Lage ist, diese endogenen Loci zu exprimieren;
Kreuzen eines hergestellten transgenen Tieres, das inaktivierte endogene T-Zell-Rezeptor-α-Loci hat mit einem hergestellten transgenen Tier, das inaktivierte endogene T-Zell-Rezeptor-β-Loci hat;
Auswählen von Nachkommen, die sowohl inaktivierte endogene T-Zell-Rezeptor-α- als auch T-Zell-Rezeptor-β-Loci haben;
Einführen von Transgenen, die aktive humane T-Zell-Rezeptor-Loci enthalten, in einen nicht-humanen Embryo oder in eine nicht-humane embryonale Stammzelle, wobei die humanen T-Zell-Rezeptor-Loci humane T-Zell-Rezeptor-α- oder T-Zell-Rezeptor-β-Loci sind;
Herstellen eines transgenen Tieres aus dem nicht-humanen Embryo oder aus der nicht-humanen embryonalen Stammzelle, der/die das aktive humane Transgen enthält;
Kreuzen eines hergestellten transgenen Tieres, das aktive humane T-Zell-Rezeptor-α-Transgene hat, mit einem hergestellten transgenen Tier, das aktive humane T-Zell-Rezeptor-β-Transgene hat;
Auswählen von Nachkommen, die sowohl aktive humane T-Zell-Rezeptor-α-, als auch T-Zell-Rezeptor-β-Transgene haben, wobei das Tier dazu in der Lage ist, T-Zellen herzustellen, die humane T-Zell-Rezeptoren exprimieren;
Kreuzen eines hergestellten transgenen Tieres, das sowohl inaktivierte endogene T-Zell-Rezeptor-α- als auch T-Zell-Rezeptor-β-Loci hat, mit einem hergestellten transgenen Tier, das sowohl aktive humane T-Zell-Rezeptor-α- als auch T-Zell-Rezeptor-β-Transgene hat;
Auswählen von Nachkommen, die inaktivierte endogene T-Zell-Rezeptor-α- und T-Zell-Rezeptor-β-Loci haben und aktive humane T-Zell-Rezeptor-α- und T-Zell-Rezeptor-β-Transgene enthalten;
und
Züchten des transgenen Tieres, wie es benötigt wird, um das transgene Tier gemäß Anspruch 1 herzustellen.

38. Verfahren gemäß einem der Ansprüche 36-37, wobei die endogenen T-Zell-Rezeptor-Loci durch eine funktionelle Einschränkung der Loci inaktiviert werden.

39. Verfahren gemäß einem der Ansprüche 36-37, wobei die endogenen T-Zell-Rezeptor-Loci durch Entfernen von J-Segment-Genen aus den Loci inaktiviert werden.

40. Verfahren gemäß einem der Ansprüche 36-37, wobei die endogenen T-Zell-Rezeptor-Loci durch Entfernen von D-Segment-Genen aus den Loci inaktiviert werden.

41. Verfahren gemäß einem der Ansprüche 36-37, wobei die endogenen T-Zell-Rezeptor-Loci durch Entfernen von C-Segment-Genen aus den Loci inaktiviert werden.

42. Verfahren gemäß einem der Ansprüche 36-37, wobei die humanen T-Zell-Rezeptor-Loci nicht-rearrangiert werden.

43. Verfahren gemäß einem der Ansprüche 36-37, wobei die Transgene, die aktive humane T-Zell-Rezeptor-Loci enthalten, in funktionaler Verknüpfung eine Vielzahl von humanen T-Zell-Rezeptor-V-Genen und -D- und/oder -J- und -C-Gene umfassen.

44. Verfahren zur Herstellung eines nicht-humanen transgenen Tieres gemäß Anspruch 1, das weiter dazu in der Lage ist, heterologe MHC-Moleküle herzustellen, umfassend die folgenden Schritte:
Kreuzen eines transgenen Tieres, das humane T-Zell-Rezeptoren exprimiert und durch das Verfahren gemäß Anspruch 36 hergestellt wurde, mit einem transgenen Tier, das humane MHC-Loci enthält und humane MHC-Moleküle exprimiert;
Auswählen von Nachkommen von transgenen Tieren, die humane T-Zell-Rezeptoren und heterologe MHC-Moleküle exprimieren; und
Züchten des transgenen Tieres, wie es benötigt wird, um das transgene Tier gemäß Anspruch 1 herzustellen, das weiter dazu in der Lage ist, heterologe MHC-Moleküle hervorzubringen.

45. Verfahren gemäß Anspruch 44, wobei die MHC-Loci alle humanen HLA-Gene enthalten.

46. Verfahren gemäß Anspruch 44, wobei die MHC-Loci einen Teilbereich von humanen HLA-Genen enthalten.

47. Verfahren gemäß einem der Ansprüche 44-46, wobei die humanen HLA-Gene MHC-Klasse-I und MHC-Klasse-II sind.

48. Verfahren gemäß einem der Ansprüche 44-47, wobei die humanen HLA-Gene MHC-Klasse-I sind.

49. Verfahren gemäß einem der Ansprüche 44-47, wobei die humanen HLA-Gene MHC-Klasse-II sind.

50. Verfahren zur Herstellung eines nicht-humanen transgenen Tieres gemäß Anspruch 1, das weiter dazu in der Lage ist, heterologe MHC-Moleküle und heterologe Co-Rezeptor-Moleküle herzustellen, umfassend die folgenden Schritte:
Kreuzen eines transgenen Tieres, das humane T-Zell-Rezeptoren und heterologe MHC-Moleküle exprimiert und durch das Verfahren gemäß Anspruch 44 hergestellt wurde mit einem transgenen Tier, das heterologe Co-Rezeptor-Gene enthält;
Auswählen von Nachkommen von transgenen Tieren, die humane T-Zell-Rezeptoren, heterologe MHC-Moleküle und heterologe Co-Rezeptor-Moleküle exprimieren; und
Züchten des transgenen Tieres, wie es benötigt wird, um das transgene Tier gemäß Anspruch 1 herzustellen, das weiter dazu in der Lage ist, heterologe MHC-Moleküle und heterologe Co-Rezeptor-Moleküle herzustellen.

51. Verfahren gemäß Anspruch 50, wobei der heterologe Co-Rezeptor ein CD8-Co-Rezeptor und ein CD4-Co-Rezeptor ist.

52. Verfahren gemäß Anspruch 50, wobei der heterologe Co-Rezeptor ein CD8-Co-Rezeptor ist.

53. Verfahren gemäß Anspruch 50, wobei der heterologe Co-Rezeptor ein CD4-Co-Rezeptor ist.

54. Verfahren zur Herstellung einer unsterblichen Zelllinie, die dazu in der Lage ist, humane T-Zell-Rezeptoren herzustellen, umfassend die folgenden Schritte:
Herstellen eines transgenen Tieres, das dazu in der Lage ist humane T-Zell-Rezeptoren herzustellen, durch das Verfahren gemäß Anspruch 37;
Induzieren einer Immunantwort in dem Tier;
Isolieren einer T-Zelle, die humane T-Zell-Rezeptoren exprimiert;
und
Fusionieren der isolierten T-Zelle mit einer immortalisierenden Zelllinie, um eine unsterbliche Zelllinie herzustellen, die dazu in der Lage ist, humane T-Zell-Rezeptoren herzustellen.

55. Verfahren gemäß Anspruch 54, wobei die isolierte T-Zelle TCR exprimiert, der spezifisch für ein spezielles Antigen von Interesse ist.

56. Verfahren gemäß Anspruch 54 oder Anspruch 55, wobei die isolierte T-Zelle TCR exprimiert, der dazu in der Lage ist, mit einem ausgewählten Peptid/MHC-Komplex von Interesse zu reagieren.

57. Verfahren gemäß einem der Ansprüche 54-56, wobei die immortalisierende Zelllinie eine Myelom-Zelllinie ist.

58. Nicht-humanes transgenes Tier gemäß Anspruch 1, das in seinem Genom weiter Transgene enthält, die in funktionaler Verknüpfung eine Vielzahl von humanen T-Zell-Rezeptor-V-Genen und ihrer -D- und/oder -J- und -C-Gene umfassen.

59. Nicht-humanes transgenes Tier gemäß Anspruch 1, wobei die Transgene, die in seinem Genom enthalten sind, in der Keimbahn enthalten sind.

## Revendications

1. Animal transgénique non humain capable de produire des RCT humains avec un répertoire substantiel de RCT, comprenant :
des loci de récepteurs de cellules T endogènes inactivés qui sont des loci de récepteurs à chaînes α et β ; et
des transgènes contenus au sein de son génome composé de loci de récepteurs de cellules T humains qui sont des loci de récepteurs à chaînes α et β,
où lesdits loci des récepteurs de cellules T humains sont non réarrangés.

2. Animal transgénique non humain selon la revendication 1, où lesdits loci des récepteurs de cellules T humains sont composés, dans une liaison fonctionnelle, d'une pluralité de gènes V et de gènes D et/ou J et C des récepteurs de cellules T humains.

3. Animal transgénique non humain selon l'une quelconque des revendications 1 ou 2, où ledit animal est capable d'un réarrangement VDJC productif et d'exprimer des récepteurs de cellules T humains.

4. Animal transgénique non humain selon l'une quelconque des revendications 1 à 3, où lesdits transgènes subissent un réarrangement VDJC productif dans les lymphocytes dudit animal transgénique non humain et où les cellules T expriment des quantités détectables de RCT transgéniques en réponse à une stimulation antigénique.

5. Animal transgénique non humain selon l'une quelconque des revendications 1 à 4, où ledit animal transgénique non humain produit une réponse immunitaire contre un antigène, ladite réponse immunitaire comprenant une population de cellules T réactives à un antigène et où les récepteurs de cellules T comprennent un récepteur de cellule T humain.

6. Animal transgénique non humain selon l'une quelconque des revendications 1 à 5, où un récepteur de cellule T humain produit est composé de chaînes α et β humaines.

7. Animal transgénique non humain selon l'une quelconque des revendications précédentes, comprenant en outre :
des transgènes contenus au sein de son génome composés de gènes HLA humains des loci du CMH humain.

8. Animal transgénique non humain selon la revendication 7, où lesdits loci du CMH contiennent tous les gènes HLA humains.

9. Animal transgénique non humain selon la revendication 7, où lesdits loci du CMH contiennent une partie des gènes HLA humains.

10. Animal transgénique non humain selon l'une quelconque des revendications 7 à 9, où lesdits gènes HLA humains sont du CMH de classe I et du CMH de classe II.

11. Animal transgénique non humain selon l'une quelconque des revendications 7 à 10, où ledit animal transgénique non humain produit une réponse immunitaire contre un antigène, ladite réponse immunitaire comprenant une population de cellules T réactives à un antigène présenté par les récepteurs du CMH de classe I humain et/ou réactives à un antigène présenté par les récepteurs du CMH de classe II humain.

12. Animal transgénique non humain selon l'une quelconque des revendications 7 à 11, où lesdits gènes HLA humains sont du CMH de classe I.

13. Animal transgénique non humain selon l'une quelconque des revendications 7 à 12, où lesdits gènes HLA humains sont HLA-A2.

14. Animal transgénique non humain selon l'une quelconque des revendications 7 à 13, où ledit animal transgénique non humain produit une réponse immunitaire contre un antigène, ladite réponse immunitaire comprenant une population de cellules T réactives à un antigène présenté par les récepteurs du CMH de classe I humain.

15. Animal transgénique non humain selon l'une quelconque des revendications 7 à 11, où lesdits gènes HLA humains sont du CMH de classe II.

16. Animal transgénique non humain selon la revendication 15, où ledit animal transgénique non humain produit une réponse immunitaire contre un antigène, ladite réponse immunitaire comprenant une population de cellules T réactives à un antigène présenté par les récepteurs du CMH de classe II humain.

17. Animal transgénique non humain selon l'une quelconque des revendications 7 à 16, où ledit animal transgénique non humain produit une réponse immunitaire à un antigène, ladite réponse immunitaire comprenant une population de cellules T réactives à l'antigène et où les récepteurs de cellules T comprennent des chaînes α et β humaines.

18. Animal transgénique non humain selon l'une quelconque des revendications précédentes, comprenant en outre des gènes contenus au sein de son génome un corécepteur humain.

19. Animal transgénique non humain selon la revendication 18, où lesdits gènes codent pour un corécepteur CD8 et/ou un corécepteur CD4.

20. Animal transgénique non humain selon la revendication 18 ou la revendication 19, où ledit animal transgénique non humain produit une réponse immunitaire contre un antigène, ladite réponse immunitaire comprenant une population de cellules T réactif à l'antigène et où les récepteurs de cellules T comprennent des récepteurs de cellules T humains et des molécules de corécepteurs.

21. Animal transgénique non humain selon l'une quelconque des revendications 18 à 20, où ledit animal transgénique non humain produit une réponse immunitaire contre un antigène, ladite réponse immunitaire comprenant une population de cellules T réactives à un antigène présenté par des récepteurs du CMH de classe I humain et/ou réactives à un antigène présenté par des récepteurs du CMH de classe II humain.

22. Animal transgénique non humain selon l'une quelconque des revendications 18 à 21, où ledit corécepteur est un corécepteur CD8.

23. Animal transgénique non humain selon l'une quelconque des revendications 18 à 22, où ledit animal transgénique non humain produit une réponse immunitaire à un antigène, ladite réponse immunitaire comprenant une population dé cellules T réactives à l'antigène et où les cellules T expriment sur leur surface cellulaire des récepteurs de cellules T humains et des molécules de corécepteurs CD8.

24. Animal transgénique non humain selon l'une quelconque des revendications 18 à 23, où ledit animal transgénique non humain produit une réponse immunitaire contre un antigène, ladite réponse immunitaire comprenant une population de cellules T réactives à un antigène présenté par des récepteurs du CMH de classe I humain.

25. Animal transgénique non humain selon l'une quelconque des revendications 18 à 21, où ledit corécepteur est un corécepteur CD4.

26. Animal transgénique non humain selon l'une quelconque des revendications 18 à 21 et 25, où ledit animal transgénique non humain produit une réponse immunitaire contre un antigène, ladite réponse immunitaire comprenant une population de cellules T réactives à l'antigène et où les cellules T expriment sur leur surface cellulaire des récepteurs de cellules T humains et des molécules de corécepteurs CD4.

27. Animal transgénique non humain selon l'une quelconque des revendications 18 à 21, 25 et 26, où ledit animal transgénique non humain produit une réponse immunitaire à un antigène, ladite réponse immunitaire comprenant une population de cellules T réactives à un antigène présenté par des récepteurs du CMH de classe II humain.

28. Animal transgénique non humain selon l'une quelconque des revendications précédentes, où ledit animal est tout animal qui peut être manipulé de manière transgénique.

29. Animal transgénique non humain selon l'une quelconque des revendications 1 à 28, où ledit animal est une souris.

30. Animal transgénique non humain selon l'une quelconque des revendications 1 à 28, où ledit animal est un rat.

31. Animal transgénique non humain selon l'une quelconque des revendications 1 à 28, où ledit animal est un primate.

32. Animal transgénique non humain selon l'une quelconque des revendications 1 à 28, où ledit animal est un chimpanzé.

33. Animal transgénique non humain selon l'une quelconque des revendications 1 à 28, où ledit animal est une chèvre.

34. Animal transgénique non humain selon l'une quelconque des revendications 1 à 28, où ledit animal est un porc.

35. Animal transgénique non humain selon l'une quelconque des revendications 1 à 28, où ledit animal est un poisson zèbre.

36. Procédé de production d'un animal transgénique non humain selon la revendication 1 comprenant les étapes consistant à :
inactiver les loci des récepteurs des cellules T endogènes qui sont des loci des récepteurs de cellules T à chaînes α et β dans un embryon non humain ou une cellule souche embryonnaire non humaine ;
insérer des transgènes contenant des loci de récepteurs des cellules T humains actifs qui sont des loci de récepteurs à chaînes α et β dans ledit embryon non humain où ladite cellule souche embryonnaire non humaine ;
produire un animal transgénique à partir dudit embryon non humain ou de ladite cellule souche embryonnaire non humaine qui contient le transgène humain actif où l'animal est capable de produire des cellules T qui expriment des récepteurs de cellules T humains ; et
élever l'animal transgénique comme il est nécessaire pour produire l'animal transgénique selon la revendication 1.

37. Procédé de production d'un animal transgénique non humain selon la revendication 1 comprenant les étapes consistant à :
inactiver les loci des récepteurs de cellules T endogènes dans un embryon non humain ou une cellule souche embryonnaire non humaine, où lesdits loci sont des loci de récepteurs de cellules T α ou de récepteurs de cellules T β ;
produire un animal transgénique à partir dudit embryon non humain ou de ladite cellule souche embryonnaire non humaine qui contient des loci inactivés ou l'animal est incapable d'exprimer lesdits loci endogènes;
croiser un animal transgénique produit ayant des loci des récepteurs de cellules T α endogènes inactivés avec un animal transgénique produit ayant des loci de récepteurs de cellules T β endogènes inactivés ;
sélectionner la descendance ayant les loci à la fois des récepteurs de cellules T α et des récepteurs de cellules T β endogènes inactivés ;
insérer des transgènes contenant des loci de récepteurs de cellules T humains actifs dans un embryon non humain ou une cellule souche embryonnaire non humaine où lesdits loci des récepteurs de cellules T humains sont des loci des récepteurs de cellules T α ou des récepteurs de cellules T β humains ;
produire un animal transgénique à partir dudit embryon non humain ou de ladite cellule souche embryonnaire non humaine qui contient le transgène humain actif ;
croiser un animal transgénique produit ayant des transgènes de récepteurs de cellules T α humains actifs avec un animal transgénique produit ayant des transgènes de récepteurs de cellules T β humains actifs ;
sélectionner la descendance ayant des transgènes à la fois de récepteurs de cellules T α et de récepteurs de cellules T β humains actifs où l'animal est capable de produire des cellules T qui expriment des récepteurs de cellules T humains ;
croiser un animal transgénique produit ayant des loci à la fois de récepteurs de cellules T α et de récepteurs de cellules T β endogènes inactivés avec un animal transgénique produit ayant des transgènes à la fois de récepteurs de cellules T α et de récepteurs de cellules T β humains actifs ;
sélectionner la descendance ayant des loci de récepteurs de cellules T α et de récepteurs de cellules T β endogènes inactivés et contenant des transgènes de récepteurs de cellules T α et de récepteurs de cellules T β humains actifs ; et
élever l'animal transgénique comme il est nécessaire pour produire l'animal transgénique selon la revendication 1.

38. Procédé selon l'une quelconque des revendications 36 ou 37, où lesdits loci de récepteurs de cellules T endogènes sont inactivés par une imitation fonctionnelle des loci.

39. Procédé selon l'une quelconque des revendications 36 ou 37, où lesdits loci des récepteurs de cellules T endogènes sont inactivés par la délétion des gènes des segments J à partir desdits loci.

40. Procédé selon l'une quelconque des revendications 36 à 37, où lesdits loci de récepteurs de cellules T endogènes sont inactivés par la délétion des gènes des segments D à partir desdits loci.

41. Procédé selon l'une quelconque des revendications 36 à 37, où lesdits loci de récepteurs de cellules T endogènes sont inactivés par la délétion des gènes des segments C à partir desdits loci.

42. Procédé selon l'une quelconque des revendications 36 ou 37, où lesdits loci de récepteurs de cellules T humains sont non réarrangés.

43. Procédé selon l'une quelconque des revendications 36 ou 37, où lesdits transgènes contenant les loci de récepteurs de cellules T humains actifs comprennent, dans une liaison fonctionnelle, une pluralité de gènes V et de gènes D et/ou J et C de récepteurs de cellules T humains.

44. Procédé de production d'un animal transgénique non humain selon la revendication 1, capable en outre de produire des molécules hétérologues du CMH, comprenant les étapes consistant à :
croiser un animal transgénique exprimant des récepteurs de cellules T humains produit par le procédé selon la revendication 36 avec un animal transgénique contenant des loci du CMH humain et exprimant des molécules du CMH humain ;
sélectionner la descendance des animaux transgéniques qui expriment des récepteurs de cellules T humains et des molécules hétérologues du CMH ; et
élever l'animal transgénique comme il est nécessaire pour produire l'animal transgénique selon la revendication 1, capable en outre de produire des molécules hétérologues du CMH.

45. Procédé selon la revendication 44, dans lequel lesdits loci du CMH contiennent tous les gènes HLA humains.

46. Procédé selon la revendication 44, dans lequel lesdits loci du CMH contiennent une partie des gènes HLA humains.

47. Procédé selon l'une quelconque des revendications 44 à 46, dans lequel lesdits gènes HLA humains sont du CMH de classe I et du CMH de classe II.

48. Procédé selon l'une quelconque des revendications 44 à 47, dans lequel lesdits gènes HLA humains sont du CMH de classe I.

49. Procédé selon l'une quelconque des revendications 44 à 47, dans lequel lesdits gènes HLA humains sont du CMH de classe II.

50. Procédé de production d'un animal transgénique non humain selon la revendication 1, capable en outre de produire des molécules hétérologues du CMH et des molécules de corécepteurs hétérologues, comprenant les étapes consistant à :
croiser un animal transgénique exprimant des récepteurs de cellules T humains et des molécules hétérologues du CMH produit par le procédé selon la revendication 44 avec un animal transgénique contenant un gène de corécepteur hétérologue ;
sélectionner la progéniture des animaux transgéniques qui expriment des récepteurs de cellules T humains, des molécules hétérologues du CMH et des molécules de corécepteurs hétérologues ; et
élever l'animal transgénique comme il est nécessaire pour produire l'animal transgénique selon la revendication 1, capable en outre de produire des molécules hétérologues du CMH et des molécules de corécepteurs hétérologues.

51. Procédé selon la revendication 50, dans lequel ledit corécepteur hétérologue est un corécepteur CD8 et un corécepteur CD4.

52. Procédé selon la revendication 50, dans lequel ledit corécepteur hétérologue est un corécepteur CD8.

53. Procédé selon la revendication 50, dans lequel ledit corécepteur hétérologue est un corécepteur CD4.

54. Procédé de génération d'une lignée cellulaire immortelle capable de produire des récepteurs de cellules T humains, comprenant les étapes consistant à :
produire un animal transgénique capable de produire des récepteurs de cellules T humains par le procédé selon la revendication 37 ;
induire une réponse immunitaire chez ledit animal ;
isoler une cellule T exprimant des récepteurs de cellules T humains ; et
fusionner la cellule T isolée avec une lignée cellulaire immortalisante pour générer une lignée cellulaire immortelle capable de produire des récepteurs de cellules T humains.

55. Procédé selon la revendication 54, dans lequel ladite cellule T isolée exprime des RCT spécifiques d'un antigène d'intérêt particulier.

56. Procédé selon la revendication 54 ou la revendication 55, dans lequel ladite cellule T isolée exprime des RCT capables de réagir avec un complexe peptide/CMH choisi d'intérêt.

57. Procédé selon l'une quelconque des revendications 54 à 56, dans lequel ladite lignée cellulaire immortalisante est une lignée cellulaire de myélome.

58. Animal transgénique non humain selon la revendication 1, contenant en outre dans son génome des transgènes comprenant, dans une liaison fonctionnelle, une pluralité de gènes V et de gènes D et/ou J et C des récepteurs de cellules T humains.

59. Animal transgénique non humain selon la revendication 1, où les transgènes contenus au sein de son génome sont contenus au sein de la lignée germinale.
